# EUROPEAN PATENT SPECIFICATION

(11) **EP 3 541 973 B1**
(45) Date of publication and mention of the grant of the patent: **27.03.2024**
(21) Application number: 17872347.4
(22) Date of filing: 17.11.2017
(51) Int. Cl.: C12N 15/10, C12Q 1/6874

(54) **POLYNUCLEOTIDE LIBRARIES HAVING CONTROLLED STOICHIOMETRY AND SYNTHESIS THEREOF**
POLYNUKLEOTIDBIBLIOTHEKEN MIT GESTEUERTER STÖCHIOMETRIE UND SYNTHESE DAVON
BANQUES DE POLYNUCLÉOTIDES À STOECHIOMÉTRIE CONTRÔLÉE ET LEUR PROCÉDÉ DE SYNTHÈSE

(30) Priority: 18.11.2016 US 201662424302 P; 21.08.2017 US 201762548307 P; 14.09.2017 US 201762558666 P
(43) Date of publication of application: 25.09.2019
(73) Proprietor: Twist Bioscience Corporation, San Francisco, CA 94158 (US)
(72) Inventor: ZEITOUN, Ramsey Ibrahim, San Francisco, California 94107 (US); CHEN, Siyuan, San Mateo, California 94403 (US)
(74) Representative: Murray, Adrian D'Coligny
(86) International application number: PCT/US2017/062391
(87) International publication number: WO 2018/094263

(56) References cited:
- WO-A1-2014/151117
- WO-A1-2015/031689
- WO-A1-2015/066174
- WO-A2-2015/021080
- US-A1- 2015 056 609
- US-A1- 2016 032 396

## Description

### CROSS-REFERENCE

This application claims the benefit of U.S. provisional patent application number 62/424,302 filed on November 18, 2016, U.S. provisional patent application number 62/548,307 filed on August 21, 2017, and U.S. provisional patent application number 62/558,666 filed on September 14, 2017.

### SEQUENCE LISTING

The instant application contains a Sequence Listing which has been submitted electronically in ASCII format. Said ASCII copy, created on November 13, 2017, is named 44854-730_601_SL.txt and is 5,304 bytes in size.

### BACKGROUND

Highly efficient chemical gene synthesis with high fidelity and low cost has a central role in biotechnology and medicine, and in basic biomedical research. De novo gene synthesis is a powerful tool for basic biological research and biotechnology applications. While various methods are known for the synthesis of relatively short fragments in a small scale, these techniques often suffer from scalability, automation, speed, accuracy, and cost.

WO 2014/151117, US 2016/032396, WO 2015/066174 and US 2015/056609 provide methods for analysis of genetic sequences. WO 2015/021080 discloses de novo synthesized large libraries of nucleic acids and devices and methods for manufacturing building blocks such as oligonucleotides.

### BRIEF SUMMARY

Provided herein are polynucleotide libraries with controlled stoichiometry, the polynucleotide library comprising at least 5000 polynucleotides, wherein the controlled stoichiometry comprises at least 30 percent of the at least 5000 polynucleotides have a GC percentage from 10 percent to 30 percent or 70 percent to 90 percent, such that, following hybridization with genomic fragments and sequencing of the hybridized genomic fragments, the polynucleotide library provides for at least 30 fold read depth of at least 90 percent of the bases of the genomic fragments under conditions for up to a 55 fold theoretical read depth for the bases of the genomic fragments, wherein the theoretical read depth comprises a distribution wherein every base of the genomic fragments has the same read depth. Further provided herein are polynucleotide libraries wherein the polynucleotide library provides for at least 30 fold read depth of at least 95 percent of the bases of the genomic fragments under conditions for up to a 55 fold theoretical read depth for the bases of the genomic fragments. Further provided herein are polynucleotide libraries wherein the polynucleotide library provides for at least 30 fold read depth of at least 98 percent of the bases of the genomic fragments under conditions for up to a 55 fold theoretical read depth for the bases of the genomic fragments. Further provided herein are polynucleotide libraries wherein the polynucleotide library provides for at least 90 percent unique reads for the bases of the genomic fragments. Further provided herein are polynucleotide libraries wherein the polynucleotide library provides for at least 95 percent unique reads for the bases of the genomic fragments. Further provided herein are polynucleotide libraries wherein the polynucleotide library provides for at least 90 percent of the bases of the genomic fragments having a read depth within about 1.5 times the mean read depth. Further provided herein are polynucleotide libraries wherein the polynucleotide library provides for at least 95 percent of the bases of the genomic fragments having a read depth within about 1.5 times the mean read depth. Further provided herein are polynucleotide libraries wherein the polynucleotide library provides for at least 90 percent of the genomic fragments having a GC percentage from 10 percent to 30 percent or 70 percent to 90 percent having a read depth within about 1.5x of the mean read depth. Further provided herein are polynucleotide libraries wherein the polynucleotide library provides for at least about 80 percent of the genomic fragments having a repeating or secondary structure sequence percentage from 10 percent to 30 percent or 70 percent to 90 percent having a read depth within about 1.5x of the mean read depth. Further provided herein are polynucleotide libraries wherein each of the genomic fragments are about 100 bases to about 500 bases in length. Further provided herein are polynucleotide libraries wherein at least about 80 percent of the at least 5000 polynucleotides are represented in an amount within at least about 1.5 times the mean representation for the polynucleotide library. Further provided herein are polynucleotide libraries wherein at least about 15 percent of the at least 5000 polynucleotides comprise polynucleotides having a repeating or secondary structure sequence percentage from 10 percent to 30 percent or 70 percent to 90 percent. Further provided herein are polynucleotide libraries wherein the at least 5000 polynucleotides encode for at least 1000 genes. Further provided herein are polynucleotide libraries wherein the polynucleotide library comprises at least 100,000 polynucleotides. Further provided herein are polynucleotide libraries wherein the polynucleotide library comprises at least 700,000 polynucleotides. Further provided herein are polynucleotide libraries wherein the at least 5000 polynucleotides comprise at least one exon sequence. Further provided herein are polynucleotide libraries wherein the at least 700,000 polynucleotides comprise at least one set of polynucleotides collectively comprising a single exon sequence. Further provided herein are polynucleotide libraries wherein the at least 700,000 polynucleotides comprises at least 150,000 sets.

Further provided herein are polynucleotide libraries of the invention wherein the polynucleotide library provides for at least 30 fold read depth of at least 95 percent of the bases of the genomic fragments under conditions for up to a 55 fold theoretical read depth for the bases of the genomic fragments. Further provided herein are polynucleotide libraries wherein the polynucleotide library provides for at least 30 fold read depth of at least 98 percent of the bases of the genomic fragments under conditions for up to a 55 fold theoretical read depth for the bases of the genomic fragments. Further provided herein are polynucleotide libraries wherein the polynucleotide library provides for at least 90 percent unique reads for the bases of the genomic fragments. Further provided herein are polynucleotide libraries wherein the polynucleotide library provides for at least 95 percent unique reads for the bases of the genomic fragments. Further provided herein are polynucleotide libraries wherein the polynucleotide library provides for at least 90 percent of the bases of the genomic fragments having a read depth within about 1.5 times of the mean read depth. Further provided herein are polynucleotide libraries wherein the polynucleotide library provides for at least 95 percent of the bases of the genomic fragments having a read depth within about 1.5 times of the mean read depth. Further provided herein are polynucleotide libraries wherein the polynucleotide library provides for greater than 90 percent of the genomic fragments having a GC percentage from 10 percent to 30 percent or 70 percent to 90 percent having a read depth within about 1.5 times of the mean read depth. Further provided herein are polynucleotide libraries wherein the polynucleotide library provides for greater than about 80 percent of the genomic fragments having a repeating or secondary structure sequence percentage from 10 percent to 30 percent or 70 percent to 90 percent having a read depth within about 1.5 times of the mean read depth. Further provided herein are polynucleotide libraries wherein each of the genomic fragments are about 100 bases to about 500 bases in length. Further provided herein are polynucleotide libraries wherein greater than about 80 percent of the at least 5000 polynucleotides are represented in an amount within at least about 1.5 times the mean representation for the polynucleotide library. Further provided herein are polynucleotide libraries wherein greater than about 15 percent of the at least 5000 polynucleotides comprise polynucleotides having a repeating or secondary structure sequence percentage from 10 percent to 30 percent or 70 percent to 90 percent. Further provided herein are polynucleotide libraries wherein the polynucleotide library comprises at least 100,000 polynucleotides. Further provided herein are polynucleotide libraries wherein the polynucleotide library comprises at least 700,000 polynucleotides. Further provided herein are polynucleotide libraries wherein the at least 700,000 polynucleotides comprise at least one set of polynucleotides collectively comprising a single exon sequence. Further provided herein are polynucleotide libraries wherein the at least 700,000 polynucleotides comprises at least 150,000 sets.

### BRIEF DESCRIPTION OF THE DRAWINGS

**Figure 1A** depicts a schematic workflow, including application of a first polynucleotide library, measuring bias from the application output, designing and synthesizing a second controlled stoichiometry polynucleotide library, and application of the second polynucleotide library to produce a desired representation output.
**Figure 1B** depicts a schematic for enriching target polynucleotides with a target binding polynucleotide library.
**Figure 1C** depicts an exemplary workflow for enrichment and sequencing of a nucleic acid sample.
**Figure 2** depicts a schematic for generation of polynucleotide libraries from cluster amplification.
**Figure 3A** depicts a pair of polynucleotides for targeting and enrichment. The polynucleotides comprise complementary target binding (insert) sequences, as well as primer binding sites.
**Figure 3B** depicts a pair of polynucleotides for targeting and enrichment. The polynucleotides comprise complementary target sequence binding (insert) sequences, primer binding sites, and non-target sequences.
**Figure 4A** depicts a polynucleotide binding configuration to a target sequence of a larger polynucleotide. The target sequence is shorter than the polynucleotide binding region, and the polynucleotide binding region (or insert sequence) is offset relative to the target sequence, and also binds to a portion of adjacent sequence.
**Figure 4B** depicts a polynucleotide binding configuration to a target sequence of a larger polynucleotide. The target sequence length is less than or equal to the polynucleotide binding region, and the polynucleotide binding region is centered with the target sequence, and also binds to a portion of adjacent sequence.
**Figure 4C** depicts a polynucleotide binding configuration to a target sequence of a larger polynucleotide. The target sequence is slightly longer than the polynucleotide binding region, and the polynucleotide binding region is centered on the target sequence with a buffer region on each side.
**Figure 4D** depicts a polynucleotide binding configuration to a target sequence of a larger polynucleotide. The target sequence is longer than the polynucleotide binding region, and the binding regions of two polynucleotides are overlapped to span the target sequence.
**Figure 4E** depicts a polynucleotide binding configuration to a target sequence of a larger polynucleotide. The target sequence is longer than the polynucleotide binding region, and the binding regions of two polynucleotides are overlapped to span the target sequence.
**Figure 4F** depicts a polynucleotide binding configuration to a target sequence of a larger polynucleotide. The target sequence is longer than the polynucleotide binding region, and the binding regions of two polynucleotides are not overlapped to span the target sequence, leaving a gap **405.**
**Figure 4G** depicts a polynucleotide binding configuration to a target sequence of a larger polynucleotide. The target sequence is longer than the polynucleotide binding region, and the binding regions of three polynucleotides are overlapped to span the target sequence.
**Figure 5** presents a diagram of steps demonstrating an exemplary process workflow for gene synthesis as disclosed herein.
**Figure 6** illustrates a computer system.
**Figure 7** is a block diagram illustrating an architecture of a computer system.
**Figure 8** is a diagram demonstrating a network configured to incorporate a plurality of computer systems, a plurality of cell phones and personal data assistants, and Network Attached Storage (NAS).
**Figure 9** is a block diagram of a multiprocessor computer system using a shared virtual address memory space.
**Figure 10** is an image of a plate having 256 clusters, each cluster having 121 loci with polynucleotides extending therefrom.
**Figure 11A** is a plot of polynucleotide representation (polynucleotide frequency versus abundance, as measured absorbance) across a plate from synthesis of 29,040 unique polynucleotides from 240 clusters, each cluster having 121 polynucleotides.
**Figure 11B** is a plot of measurement of polynucleotide frequency versus abundance absorbance (as measured absorbance) across each individual cluster, with control clusters identified by a box.
**Figure 12** is a plot of measurements of polynucleotide frequency versus abundance (as measured absorbance) across four individual clusters.
**Figure 13A** is a plot of on frequency versus error rate across a plate from synthesis of 29,040 unique polynucleotides from 240 clusters, each cluster having 121 polynucleotides.
**Figure 13B** is a plot of measurement of polynucleotide error rate versus frequency across each individual cluster, with control clusters identified by a box.
**Figure 14** is a plot of measurements of polynucleotide frequency versus error rate across four clusters.
**Figure 15** is a plot of GC content as a measure of the number of polynucleotides versus percent per polynucleotide.
**Figure 16** provides plots with results from PCR with two different polymerases. Each chart depicts number of polynucleotides (0 to 2,000) versus observed frequency ("0 to 35" measured in counts per 100,000).

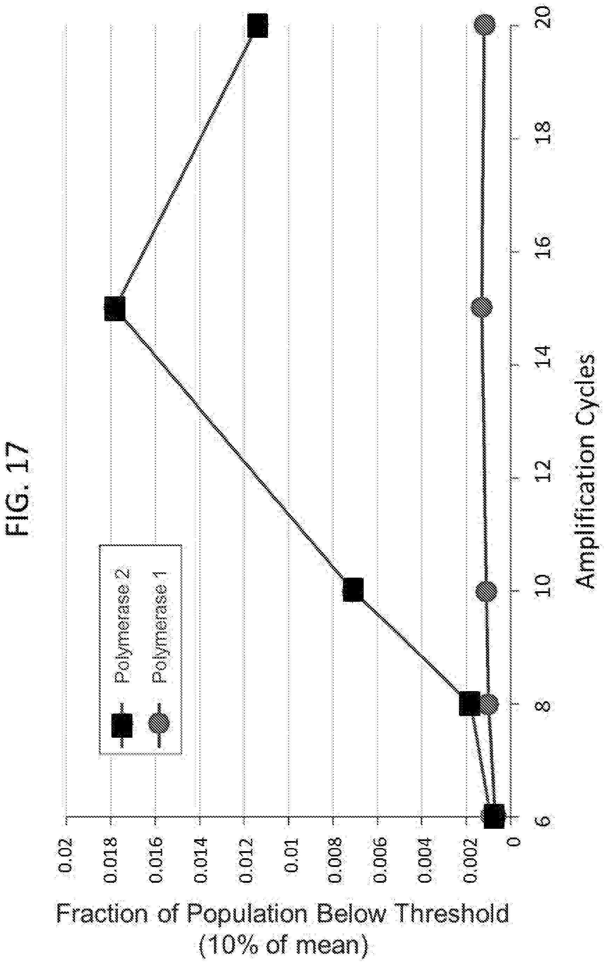

**Figure 17** provides a chart with quantification of polynucleotide population uniformity post amplification that was recorded.
**Figure 18** depicts a plot demonstrating the impact of over-amplification on sequence dropouts.
**Figure 19** depicts plots of percentage GC content per polynucleotide frequency (per 100,000 reads) in pooled unamplified and amplified populations of polynucleotides.
**Figure 20** is a plot of percentage GC content per polynucleotide frequency (per 100,000 reads) for two separate runs after amplification of clusters.
**Figure 21A** is a plot of percentage GC content per polynucleotide frequency for a GC-balanced library of polynucleotides.
**Figure 21B** is a plot of percentage GC content per polynucleotide frequency for a heavily high and low GC-biased library of polynucleotides.
**Figure 21C** is a plot of percentage GC content per polynucleotide frequency for a mildly high and low GC-biased library of polynucleotides.
**Figure 21D** is a plot of percentage GC content per polynucleotide frequency for a low GC biased library of polynucleotides.
**Figure 21E** is a plot of percentage GC content per polynucleotide frequency for a high GC biased library of polynucleotides.
**Figure 22** is a plot of percentage GC content per polynucleotide frequency for a theoretical 13,000 plex polynucleotide library with sequences containing 15% to 85% GC content.
**Figure 23** is a plot of number of polynucleotides verses polynucleotide frequency (per 100,000 reads) for a GC-balanced polynucleotide library.
**Figure 24A** is a plot showing the amount of sampling required to obtain 80% sequencing coverage for a GC-balanced polynucleotide library, compared to the theoretical maximum of a monodispersed library.
**Figure 24B** is a plot showing the amount of sampling required to obtain 90% sequencing coverage for a GC-balanced polynucleotide library compared to the theoretical maximum of a monodispersed library.

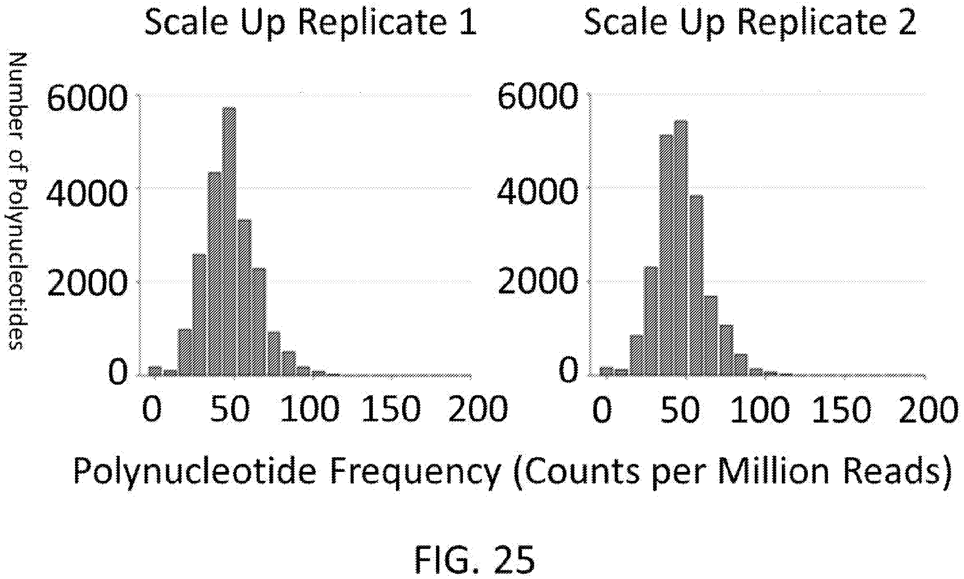

**Figure 25** is a plot of number of polynucleotides verses polynucleotide frequency (counts per 1,000,000 reads) for a library containing polynucleotides that are 80 nucleotides long.
**Figure 26** is a plot of number of polynucleotides verses polynucleotide frequency (counts per 1,000,000 reads) for a library containing polynucleotides that are 120 nucleotides long.
**Figure 27** depicts plots showing the mean frequency of polynucleotides (per 1,000,000 reads) for both 80- and 120- nucleotide long GC-balanced polynucleotide libraries.
**Figure 28** depicts plots showing the effect of PCR amplification cycle number, GC content, and choice of DNA polymerase on polynucleotide sequence representation.
**Figure 29** is a plot of the sequence dropouts as a function of amplification cycles for two different high-fidelity polymerases.
**Figure 30** depicts plots showing the effect of different DNA polymerases on sequence representation. The same polynucleotide library was amplified for 15 cycles with either DNA polymerase 1 or DNA polymerase 2.

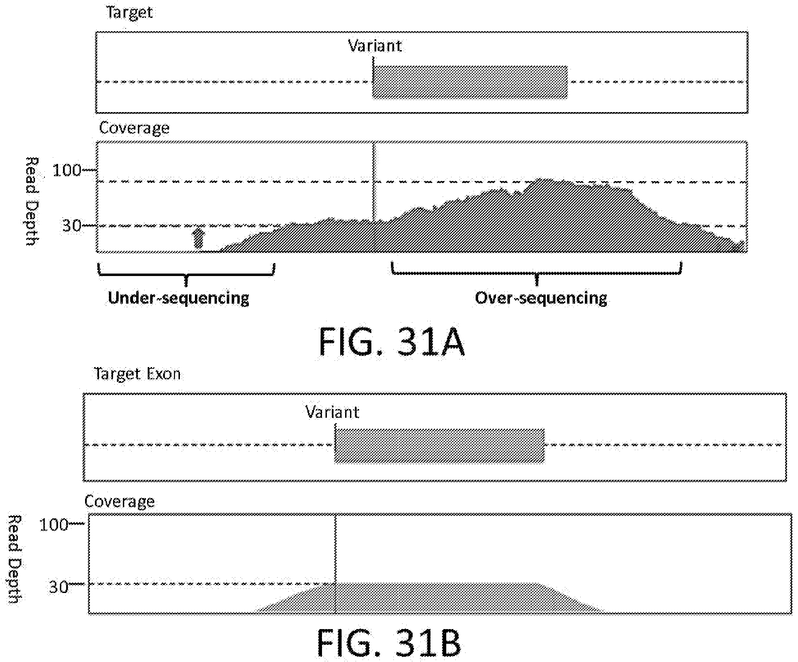

**Figure 31A** depicts the amount of over sequencing required to achieve a given read depth for a target sequence using an exome probe library without controlled stoichiometry.
**Figure 31B** depicts the reduction in over sequencing required to achieve a given read depth for a target sequence using an exome probe library with controlled stoichiometry, when compared to an exome probe library without controlled stoichiometry.
**Figure 32A** is a plot of percent bases possessing 1x, 20x, or 30x sequencing read depth (X coverage) for both a comparator exome probe kit A and controlled stoichiometry probe Library 1.
**Figure 32B** is a plot of percent bases possessing 1x or 10x sequencing read depth (X coverage) normalized at 4.5Gb of sequencing for a panel of comparator exome probe kits and the controlled stoichiometry probe library 1.
**Figure 33** depicts the synthesis of polynucleotide probe libraries of different scales as a function of the number of polynucleotides in the library.
**Figure 34** depicts a comparison between coverage (number of bases) as a function of read depth of a comparator array-based probe library vs. a controlled stoichiometry probe library 2.
**Figure 35A** depicts a comparison between coverage as a function of read depth of a comparator array-based probe library vs. a controlled stoichiometry probe library 2 for targets with GC content between 10-30%, and between 30-50%.
**Figure 35B** depicts a comparison between coverage as a function of read depth of a comparator array-based probe library vs. a controlled stoichiometry probe library 2 for targets with GC content between greater than 50-70%, and between greater than 70-90%.
**Figure 36A** depicts a comparison between the percent (%) on target rate of a comparator array-based probe library vs. 0.1×, 1x, and 3x concentrations of a controlled stoichiometry probe library 3.
**Figure 36B** depicts a comparison between the read depth of a comparator array-based probe library vs. 0.1×, 1×, and 3× concentrations of a controlled stoichiometry probe library 3.
**Figure 37** depicts a comparison between percentages of unique reads, and target bases at 1X, 20X, and 30X read depth of a comparator exome kit vs. controlled stoichiometry probe library 4.

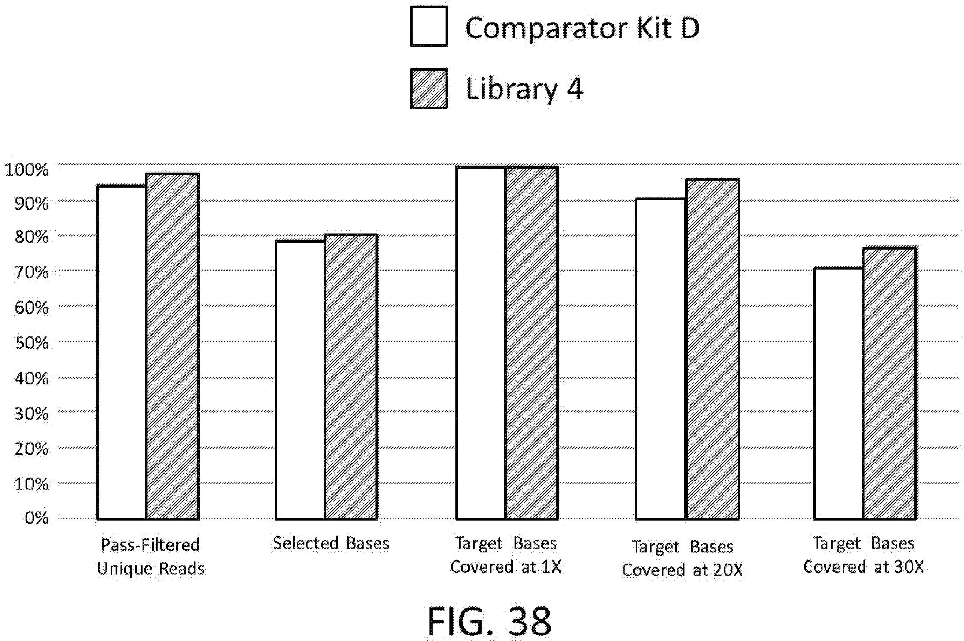

**Figure 38** depicts a comparison between percentages of bases covered, and target bases at 1X, 20X, and 30X read depth of a comparator exome kit vs. controlled stoichiometry probe library 4.

### DETAILED DESCRIPTION

The present disclosure employs, unless otherwise indicated, conventional molecular biology techniques, which are within the skill of the art. Unless defined otherwise, all technical and scientific terms used herein have the same meaning as is commonly understood by one of ordinary skill in the art.

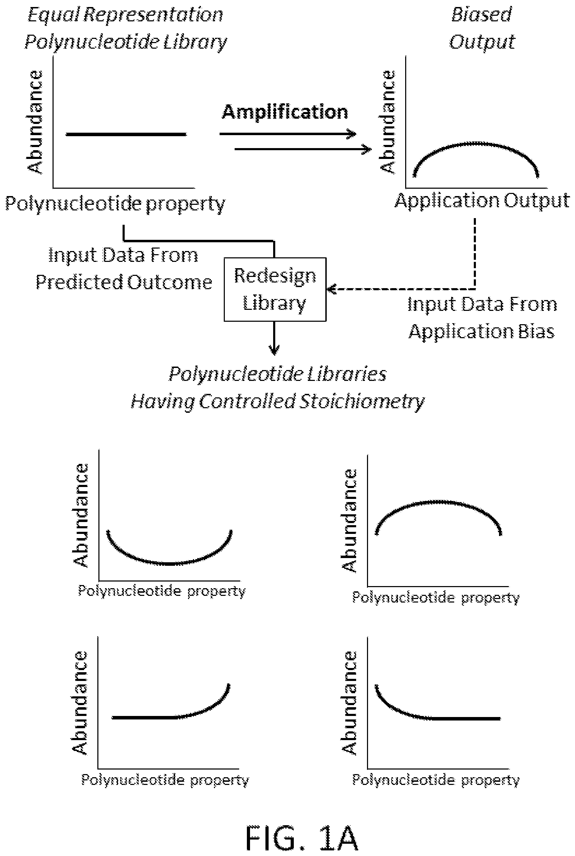

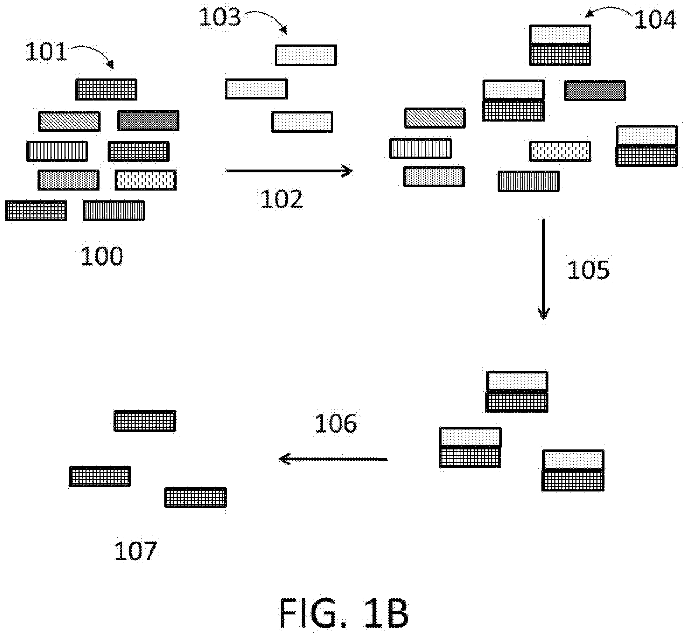

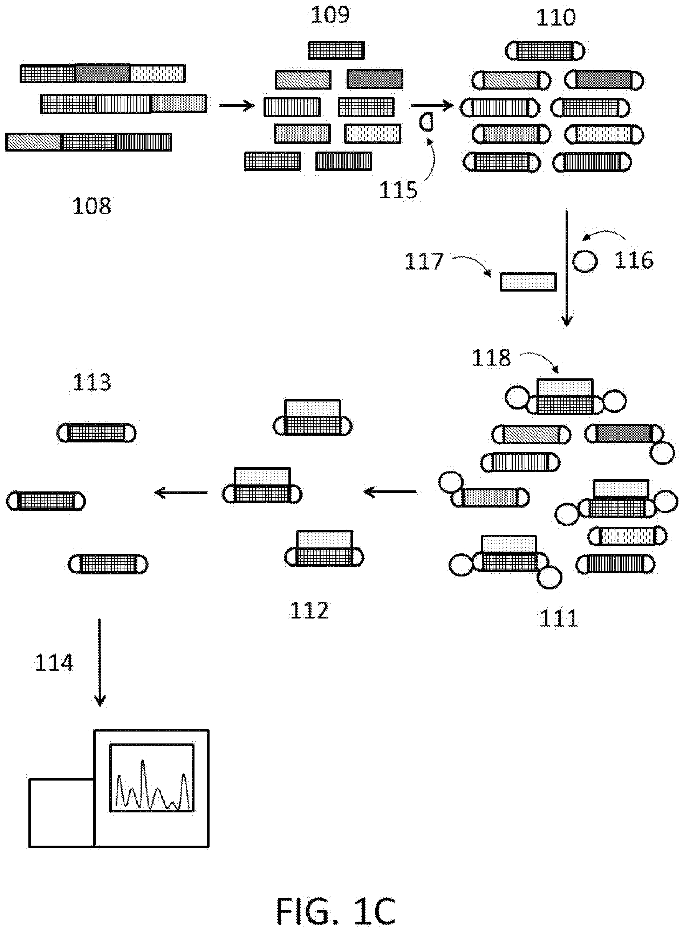

Disclosed herein but not encompassed by the appended claims are methods for designing, synthesizing and controlling the stoichiometry of large polynucleotide libraries. When a first population of polynucleotides is subjected to a preliminary application step, e.g., for amplification, as capture probes for an enrichment, and gene synthesis, subsequent amplification reactions of the population of polynucleotides can result in a biased representative output due to variance in polynucleotide sequence, resulting in certain polynucleotides being more abundantly represented than others. **FIG. 1A****.** The resulting bias observed from this preliminary application output is measured, and used to control the first population of polynucleotides with a preselected stoichiometry, e.g., relative frequency of polynucleotides in the population taking into account any number of sequence features, such as GC content, repeating sequences, trailing adenines, secondary structure, affinity for target sequence binding, or modified nucleotides. After modifying the stoichiometry of polynucleotides, a second population of polynucleotides is designed and synthesized with a preselected stoichiometry to correct for the undesirable bias effects associated with an application step. In some instances, subjecting the second controlled stoichiometry population of polynucleotides to the application step, such as PCR amplification, results in a balanced output, such as a population of amplified polynucleotides with highly uniform representation, or non-uniform representation with preselected shift in representation. See **FIG. 1A****,** lower charts. In some instances, methods described herein comprise controlling sequence representation of polynucleotide probes such that the polynucleotide population provides for highly uniform target sequence capture frequency **(****FIG. 1B****).** For example, a sample of polynucleotides **100** comprises target polynucleotides **101.** Contact of the sample **100** with target binding polynucleotides **103** under appropriate conditions **102** results in the formation of hybridization pairs **104,** which are separated from non-target polynucleotides in sample **100.** Denaturation and separation of the pairs **104** releases the enriched target polynucleotides **107** for downstream applications, such as sequencing. Also disclosed herein but not encompassed by the appended claims are de novo synthesized polynucleotides for use in hybridization to genomic DNA, for example in the context of a sequencing process. In a first step of an exemplary sequencing workflow **(****FIG. 1C****),** a nucleic acid sample **108** comprising target polynucleotides is fragmented by mechanical or enzymatic shearing to form a library of fragments **109.** Adapters **115** optionally comprising primer sequences and/or barcodes are ligated to form an adapter-tagged library **110.** This library is then optionally amplified, and hybridized with target binding polynucleotides **117** which hybridize to target polynucleotides, along with blocking polynucleotides **116** that prevent hybridization between target binding polynucleotides **117** and adapters **115.** Capture of target polynucleotide-target binding polynucleotide hybridization pairs **112,** and removal of target binding polynucleotides **117** allows isolation/enrichment of target polynucleotides **113,** which are then optionally amplified and sequenced **114.**

### Definitions

Throughout this disclosure, numerical features are presented in a range format. It should be understood that the description in range format is merely for convenience and brevity and should not be construed as an inflexible limitation on the scope of any embodiments. Accordingly, the description of a range should be considered to have specifically disclosed all the possible subranges as well as individual numerical values within that range to the tenth of the unit of the lower limit unless the context clearly dictates otherwise. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual values within that range, for example, 1.1,2, 2.3, 5, and 5.9. This applies regardless of the breadth of the range. The upper and lower limits of these intervening ranges may independently be included in the smaller ranges, and are also encompassed within the invention, subject to any specifically excluded limit in the stated range. Where the stated range includes one or both of the limits, ranges excluding either or both of those included limits are also included in the invention, unless the context clearly dictates otherwise.

The terminology used herein is for the purpose of describing particular embodiments only and is not intended to be limiting of any embodiment. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items.

Unless specifically stated or obvious from context, as used herein, the term "about" in reference to a number or range of numbers is understood to mean the stated number and numbers +/- 10% thereof, or 10% below the lower listed limit and 10% above the higher listed limit for the values listed for a range.

As used herein, the terms "preselected sequence", "predefined sequence" or "predetermined sequence" are used interchangeably. The terms mean that the sequence of the polymer is known and chosen before synthesis or assembly of the polymer. In particular, various aspects of the invention are described herein primarily with regard to the preparation of nucleic acids molecules, the sequence of the oligonucleotide or polynucleotide being known and chosen before the synthesis or assembly of the nucleic acid molecules.

The term nucleic acid encompasses double- or triple-stranded nucleic acids, as well as single-stranded molecules. In double- or triple-stranded nucleic acids, the nucleic acid strands need not be coextensive (i.e., a double-stranded nucleic acid need not be double-stranded along the entire length of both strands). Nucleic acid sequences, when provided, are listed in the 5' to 3' direction, unless stated otherwise. Methods described herein provide for the generation of isolated nucleic acids. Methods described herein additionally provide for the generation of isolated and purified nucleic acids. The length of polynucleotides, when provided, are described as the number of bases and abbreviated, such as nt (nucleotides), bp (bases), kb (kilobases), or Gb (gigabases).

Disclosed herein but not encompassed by the appended claims are methods and compositions for production of synthetic (i.e. de novo synthesized or chemically synthesizes) polynucleotides. The term oligonucleic acid, oligonucleotide, oligo, and polynucleotide are defined to be synonymous throughout. Libraries of synthesized polynucleotides described herein may comprise a plurality of polynucleotides collectively encoding for one or more genes or gene fragments. In some instances, the polynucleotide library comprises coding or non-coding sequences. In some instances, the polynucleotide library encodes for a plurality of cDNA sequences. Reference gene sequences from which the cDNA sequences are based may contain introns, whereas cDNA sequences exclude introns. Polynucleotides described herein may encode for genes or gene fragments from an organism. Exemplary organisms include, without limitation, prokaryotes (e.g., bacteria) and eukaryotes (e.g., mice, rabbits, humans, and non-human primates). In some instances, the polynucleotide library comprises one or more polynucleotides, each of the one or more polynucleotides encoding sequences for multiple exons. Each polynucleotide within a library described herein may encode a different sequence, i.e., non-identical sequence. In some instances, each polynucleotide within a library described herein comprises at least one portion that is complementary to sequence of another polynucleotide within the library. Polynucleotide sequences described herein may be, unless stated otherwise, comprise DNA or RNA. A polynucleotide library of the invention described herein may comprise at least 10,000, 20,000, 30,000, 50,000, 100,000, 200,000, 500,000, 1,000,000, or more than 1,000,000 polynucleotides. A polynucleotide library of the invention described herein may have no more than 5,000, 10,000, 20,000, 30,000, 50,000, 100,000, 200,000, 500,000, or no more than 1,000,000 polynucleotides. A polynucleotide library of the invention described herein may comprise10,000 to 50,000, 100,000 to 500,000, or to 50,000 to 1,000,000 polynucleotides. A polynucleotide library described herein may comprise about 370,000; 400,000; 500,000 or more different polynucleotides.

Disclosed herein but not encompassed by the appended claims are methods and compositions for production of synthetic (i.e. de novo synthesized) genes. Libraries comprising synthetic genes may be constructed by a variety of methods described in further detail elsewhere herein, such as PCA, non-PCA gene assembly methods or hierarchical gene assembly, combining ("stitching") two or more double-stranded polynucleotides to produce larger DNA units (i.e., a chassis). Libraries of large constructs may involve polynucleotides that are at least 1, 1.5, 2, 3, 4, 5, 6, 7, 8, 9, 10, 15, 20, 30, 40, 50, 60, 70, 80, 90, 100, 125, 150, 175, 200, 250, 300, 400, 500 kb long or longer. The large constructs can be bounded by an independently selected upper limit of about 5000, 10000, 20000 or 50000 base pairs. The synthesis of any number of polypeptide-segment encoding nucleotide sequences, including sequences encoding non-ribosomal peptides (NRPs), sequences encoding non-ribosomal peptide-synthetase (NRPS) modules and synthetic variants, polypeptide segments of other modular proteins, such as antibodies, polypeptide segments from other protein families, including non-coding DNA or RNA, such as regulatory sequences e.g. promoters, transcription factors, enhancers, siRNA, shRNA, RNAi, miRNA, small nucleolar RNA derived from microRNA, or any functional or structural DNA or RNA unit of interest. The following are non-limiting examples of polynucleotides: coding or non-coding regions of a gene or gene fragment, intergenic DNA, loci (locus) defined from linkage analysis, exons, introns, messenger RNA (mRNA), transfer RNA, ribosomal RNA, short interfering RNA (siRNA), shorthairpin RNA (shRNA), micro-RNA (miRNA), small nucleolar RNA, ribozymes, complementary DNA (cDNA), which is a DNA representation of mRNA, usually obtained by reverse transcription of messenger RNA (mRNA) or by amplification; DNA molecules produced synthetically or by amplification, genomic DNA, recombinant polynucleotides, branched polynucleotides, plasmids, vectors, isolated DNA of any sequence, isolated RNA of any sequence, nucleic acid probes, and primers. cDNA encoding for a gene or gene fragment referred to herein, may comprise at least one region encoding for exon sequence(s) without an intervening intron sequence found in the corresponding genomic sequence. Alternatively, the corresponding genomic sequence to a cDNA may lack an intron sequence in the first place.

### De Novo Synthesis of Small Polynucleotide Populations for Amplification Reactions

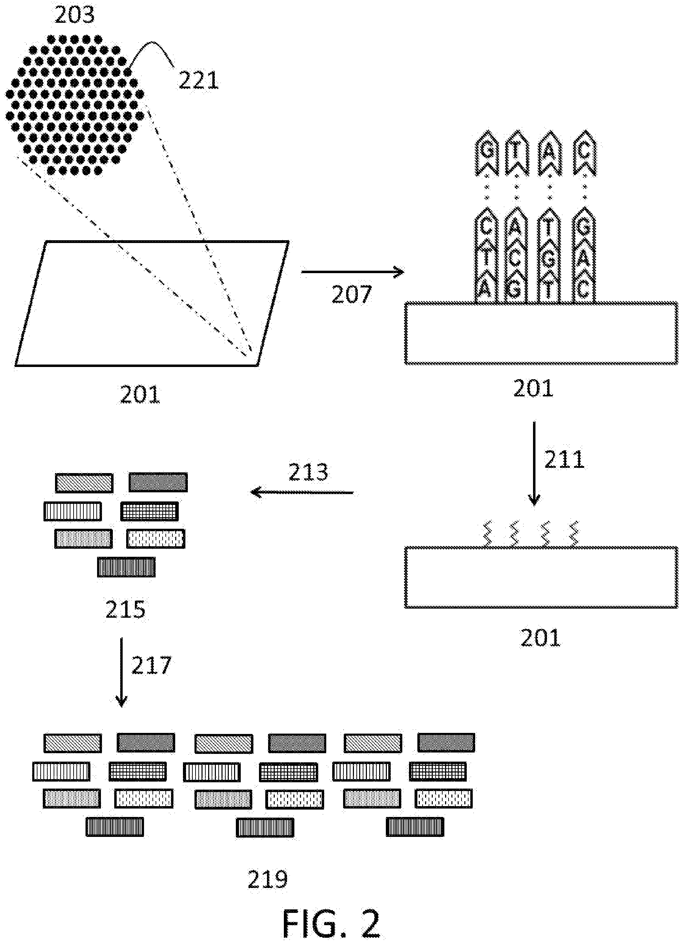

Described herein but not encompassed by the appended claims are methods of synthesis of polynucleotides from a surface, e.g., a plate. In some instances, the polynucleotides are synthesized on a cluster of loci for polynucleotide extension, released and then subsequently subjected to an amplification reaction, e.g., PCR. An exemplary workflow of synthesis of polynucleotides from a cluster is depicted in **FIG. 2****.** A silicon plate **201** includes multiple clusters **203.** Within each cluster are multiple loci **221.** Polynucleotides are synthesized **207** de novo on a plate **201** from the cluster **203.** Polynucleotides are cleaved **211** and removed **213** from the plate to form a population of released polynucleotides **215.** The population of released polynucleotides **215** are then amplified **217** to form a library of amplified polynucleotides **219.**

Disclosed herein but not encompassed by the appended claims are methods where amplification of polynucleotides synthesized on a cluster provide for enhanced control over polynucleotide representation compared to amplification of polynucleotides across an entire surface of a structure without such a clustered arrangement. In some instances, amplification of polynucleotides synthesized from a surface having a clustered arrangement of loci for polynucleotides extension provides for overcoming the negative effects on representation due to repeated synthesis of large polynucleotide populations. Exemplary negative effects on representation due to repeated synthesis of large polynucleotide populations include, without limitation, amplification bias resulting from high/low GC content, repeating sequences, trailing adenines, secondary structure, affinity for target sequence binding, or modified nucleotides in the polynucleotide sequence.

Cluster amplification as opposed to amplification of polynucleotides across an entire plate without a clustered arrangement can result in a tighter distribution around the mean. For example, if 100,000 reads are randomly sampled, an average of 8 reads per sequence would yield a library with a distribution of about 1.5X from the mean. In some cases, single cluster amplification results in at most about 1.5X, 1.6X, 1.7X, 1.8X, 1.9X, or 2.0X from the mean. In some cases, single cluster amplification results in at least about 1.0X, 1.2X, 1.3X, 1.5X 1.6X, 1.7X, 1.8X, 1.9X, or 2.0X from the mean.

Cluster amplification methods described herein when compared to amplification across a plate can result in a polynucleotide library that requires less sequencing for equivalent sequence representation. In some instances at least 10%, at least 20%, at least 30%, at least 40%, at least 50%, at least 60%, at least 70%, at least 80%, at least 90%, or at least 95% less sequencing is required. In some instances up to 10%, up to 20%, up to 30%, up to 40%, up to 50%, up to 60%, up to 70%, up to 80%, up to 90%, or up to 95% less sequencing is required. Sometimes 30% less sequencing is required following cluster amplification compared to amplification across a plate. Sequencing of polynucleotides in some instances are verified by high-throughput sequencing such as by next generation sequencing. Sequencing of the sequencing library can be performed with any appropriate sequencing technology, including but not limited to single-molecule real-time (SMRT) sequencing, Polony sequencing, sequencing by ligation, reversible terminator sequencing, proton detection sequencing, ion semiconductor sequencing, nanopore sequencing, electronic sequencing, pyrosequencing, Maxam-Gilbert sequencing, chain termination (e.g., Sanger) sequencing, +S sequencing, or sequencing by synthesis. The number of times a single nucleotide or polynucleotide is identified or "read" is defined as the sequencing depth or read depth. In some cases, the read depth is referred to as a fold coverage, for example, 55 fold (or 55X) coverage, optionally describing a percentage of bases.

In some instances, amplification from a clustered arrangement compared to amplification across a plate results in less dropouts, or sequences which are not detected after sequencing of amplification product. Dropouts can be of AT and/or GC. In some instances, a number of dropouts is at most about 1%, 2%, 3%, 4%, or 5% of a polynucleotide population. In some cases, the number of dropouts is zero.

A cluster as described herein but not encompassed by the appended claims comprises a collection of discrete, non-overlapping loci for polynucleotide synthesis. A cluster can comprise about 50-1000, 75-900, 100-800, 125-700, 150-600, 200-500, or 300-400 loci. In some instances, each cluster includes 121 loci. In some instances, each cluster includes about 50-500, 50-200, 100-150 loci. In some instances, each cluster includes at least about 50, 100, 150, 200, 500, 1000 or more loci. In some instances, a single plate includes 100, 500, 10000, 20000, 30000, 50000, 100000, 500000, 700000, 1000000 or more loci. A locus can be a spot, well, microwell, channel, or post. In some instances, each cluster has at least 1X, 2X, 3X, 4X, 5X, 6X, 7X, 8X, 9X, 10X, or more redundancy of separate features supporting extension of polynucleotides having identical sequence.

### Design of Polynucleotide Libraries having Controlled Stoichiometry

Disclosed herein but not encompassed by the appended claims are methods for design and synthesis of a polynucleotide library wherein the amount (or stoichiometry) of each polynucleotide species (i.e., having a different sequence than another polynucleotide in the library) is adjusted to a predetermined amount such that a desirable outcome is controlled for in a downstream application. As such, disclosed herein but not encompassed by the appended claims are methods for controlled and predetermined modification of polynucleotide species stoichiometry. For example, polynucleotide species distribution subsequent to an amplification reaction may be controlled for using methods described herein. Polynucleotide species distribution is preselected for in order to provide for highly uniform capture of target sequences, e.g., using a panel of polynucleotides for hybridization based assays such as for sequencing analysis. Moreover, methods described herein provide for designing a polynucleotide library of sequences with one or more sequence features that would typically result in non-uniform amplification products or capture products due to structural features of the certain "problematic" polynucleotide sequences, wherein the "problematic" polynucleotide sequences comprise one or more properties associated with creating bias in application of the polynucleotide library. Exemplary "problematic" polynucleotide sequence properties for controlling stoichiometry using methods described herein include, without limitation, high or low GC or AT content, repeating sequences, trailing adenines, secondary structure, affinity for target sequence binding (for amplification, enrichment, or detection), stability, melting temperature, biological activity, ability to assemble into larger fragments, sequences containing modified nucleotides or nucleotide analogues, or any other property of polynucleotides to generate a second polynucleotide library of sequences based on predicted or empirical data. In some instances, a library of sequences is obtained for controlling stoichiometry, and organized or clustered (binning) into two or more pre-defined groups (bins) based on the one or more sequence features. In some instances the two or more bins represent individual unique sequences. In some instances, the bins represent ranges of values based on the defined one or more sequence features that each contain at least 10%, 20%, 30%, 40%, 50%, 60%, 70%, 80%, 90%, 95%, or at least 99% of the total sequences. In some instances, the bins represent ranges of values that each contain at most 10%, 20%, 30%, 40%, 50%, 60%, 70%, 80%, 90%, 95%, or less than 100% of the total sequences. In one example, bins may be defined by % GC content, with multiple bins representing a range of 25-75% in 5% increments (e.g., 25-29%, 30-34%, 35-39%, etc.), one bin representing less than 25%, and one bin representing greater than 75% GC content. An abundance value for each bin, representing the stoichiometry of molecules for all sequences in each bin is assigned. In some instances, the abundance value is initially set to 100, leading to an equal representation of sequences per bin. In some instances, control of stoichiometry is accomplished by using obtained application bias data to increase, decrease, or maintain the abundance value for each bin. Other methods of adjusting sequence abundance consistent with the specification are also employed. In some instances, a previously acquired distribution is used to determine the initial abundance values.

In some instances, the application bias data is obtained by predictive algorithms. The application bias data may be obtained empirically or obtained from an uncontrolled or previously controlled stoichiometry library. For example, the application bias data is obtained from amplification of a polynucleotide library; the frequency of polynucleotides per bin after amplification is plotted against %GC bins to establish amplification bias as a function of %GC content. In another example, the application bias data is obtained from next generation sequencing (NGS) data after enrichment of target sequences with a polynucleotide probe library; the reads per target gene are used to sort probe sequences into bins; reads per target gene are plotted against number of NGS reads bins to establish NGS sequencing bias as a function of polynucleotide probe sequence. In another example, the application bias data could be obtained from a cellular assay output, such as fluorescence, after treatment of cells with a polynucleotide library-containing vector; the reads per sequence identified in fluorescent cells are used to sort probe sequences into bins; reads per sequence are plotted against number of reads bins to establish bias as a function of polynucleotide probe sequence.

After controlling stoichiometry, the modified sequence library is synthesized to generate a controlled stoichiometry library of polynucleotides. In some instances, the controlled stoichiometry library is used for a downstream application. In some instances, data from the downstream application with the controlled stoichiometry polynucleotide library is used to conduct additional rounds of stoichiometric modification of the library.

### Generation of Polynucleotide Libraries with Controlled Stoichiometry of GC Content

Disclosed herein but not encompassed by the appended claims are methods for synthesizing polynucleotide libraries with a defined property, such as such as GC content, repeating sequences, trailing adenines, secondary structure, affinity for target sequence binding, or modified nucleotides to generate a second population of polynucleotides based on predicted or empirical data. For example, where a polynucleotide library is selected for synthesis to result in a defined GC content post-amplification, adjustment of the species representation for polynucleotides in the library at synthesis stage dependent on GC content results in improved polynucleotide representation post-amplification. GC content in a polynucleotide library can be at least 10%, 20%, 30%, 40%, 50%, 60%, 70%, 80%, 90%, 95%, or more than 95%. In some instances, GC content in a polynucleotide library is at most 10%, 20%, 30%, 40%, 50%, 60%, 70%, 80%, 90%, 95%, or less than 100%. In some cases, GC content is in a range of about 5-95%, 10-90%, 30-80%, 40-75%, or 50-70%.

Polynucleotide libraries described herein may be adjusted for their GC content. In some instances, polynucleotide libraries favor high GC content. For example, a library is designed where increased polynucleotide frequency has a GC content in a range of about 40% to about 90%. In some instances, polynucleotide libraries contain low GC content. For example, a library is designed where increased polynucleotide frequency has a GC content is in a range of about 10% to about 60%. A library can be designed to favor high and low GC content. For example, a library can be designed where increased polynucleotide frequency has a GC content primarily in a range of about 10% and about 30% and in a range of about 70% to about 90%. In some instances, a library favors uniform GC content. For example, polynucleotide frequency is uniform with a GC content in a range of about 10% to about 90%. In some instances, a library comprises polynucleotides with a GC percentage of about 10% to about 95%. The library described herein comprises polynucleotides having greater than 30% different polynucleotides having a GC percentage from 10% to 30% or 70 to 90%. In some instances, a library described herein comprises polynucleotides having less than about 15% of the polynucleotides have a GC percentage from 10% to 30% or 60 to 90%.

Generation of polynucleotide libraries with a specified GC content in some cases occurs by combining at least 2 polynucleotide libraries with different GC content. In some instances, at least 2, 3, 4, 5, 6, 7, 10, or more than 10 polynucleotide libraries are combined to generate a population of polynucleotides with a specified GC content. In some cases, no more than 2, 3, 4, 5, 6, 7, or 10 polynucleotide libraries are combined to generate a population of non-identical polynucleotides with a specified GC content.

In some instances, GC content is adjusted by synthesizing fewer or more polynucleotides per cluster. For example, at least 25, 50, 100, 200, 300, 400, 500, 600, 700, 800, 900, 1000, or more than 1000 non-identical polynucleotides are synthesized on a single cluster. In some cases, no more than about 50, 100, 200, 300, 400, 500, 600, 700, 800, 900, 1000 non-identical polynucleotides are synthesized on a single cluster. In some instances, 50 to 500 non-identical polynucleotides are synthesized on a single cluster. In some instances, 100 to 200 non-identical polynucleotides are synthesized on a single cluster. In some instances, about 100, about 120, about 125, about 130, about 150, about 175, or about 200 non-identical polynucleotides are synthesized on a single cluster.

In some cases, GC content is adjusted by synthesizing non-identical polynucleotides of varying length. For example, the length of each of the non-identical polynucleotides synthesized may be at least or about at least 10, 15, 20, 25, 30, 35, 40, 45, 50, 100, 150, 200, 300, 400, 500, 2000 nucleotides, or more. The length of the non-identical polynucleotides synthesized may be at most or about at most 2000, 500, 400, 300, 200, 150, 100, 50, 45, 35, 30, 25, 20, 19, 18, 17, 16, 15, 14, 13, 12, 11, 10 nucleotides, or less. The length of each of the non-identical polynucleotides synthesized may fall from 10-2000, 10-500, 9-400, 11-300, 12-200, 13-150, 14-100, 15-50, 16-45, 17-40, 18-35, and 19-25.

### Generation of Polynucleotide Libraries with Controlled Stoichiometry of Repeating Sequence Content

A polynucleotide library described herein may be synthesized with a specified repeating sequence distribution . In some instances, adjusting polynucleotide libraries for repeating sequence content results in improved polynucleotide representation.

A repeating sequence can be the repetition of a single nucleotide or the repetition of a block of two or more nucleotides. In some instances, a repeating sequence is at least 2, 3, 4, 5, 6, 7, 8, 9, or at least 10 nucleotides. In some instances, a repeating sequence is at most 2, 3, 4, 5, 6, 7, 8, 9, or at most 10 nucleotides. In some instances, a block of nucleotides comprises at least 2, 3, 4, 5, 10, 15, 25, 50, 100, 200, 500, or at least 1000 nucleotides. In some instances, a block of nucleotides comprises at most 2, 3, 4, 5, 10, 15, 25, 50, 100, 200, 500, or at most 1000 nucleotides. The repeating sequence can be located at an internal or a terminal location of a larger synthesized polynucleotide. The terminal location may be near the 5', 3', or both 5' and 3' termini of the polynucleotide. In some instances, the repeating sequence is located within at least 1, 2, 3, 4, 5, 6, 7, 8, 9, or at least 10 nucleotides of the terminus. In some instances, the repeating sequence is located within at most 1, 2, 3, 4, 5, 6, 7, 8, 9, or at most 10 nucleotides of the terminus. In some instances, the repeating nucleotide is an adenine. In some instances, the repeating sequence is located at the polynucleotide terminus, for example, a polyadenine tail.

Repeating sequence content in a polynucleotide library can be at least 10%, 20%, 30%, 40%, 50%, 60%, 70%, 80%, 90%, 95%, or more than 95%. In some instances, repeating sequence content in a polynucleotide library is at most 10%, 20%, 30%, 40%, 50%, 60%, 70%, 80%, 90%, 95%, or no more than 100%. In some cases, repeating sequence content is in a range of about 5-95%, 10-90%, 30-80%, 40-75%, or 50-70%.

Polynucleotide libraries can be adjusted for their repeating sequence content. In some instances, polynucleotide libraries favor high repeating sequence content. For example, a library is designed where increased polynucleotide frequency has a repeating sequence content in a range of about 40% to about 90%. In some instances, polynucleotide libraries contain low repeating sequence content. For example, a library is designed where increased polynucleotide frequency has a repeating sequence content is in a range of about 10% to about 60%. A library can be designed to favor high and low repeating sequence content. For example, a library can be designed where increased polynucleotide frequency has a repeating sequence content primarily in a range of about 10% and about 30% and in a range of about 70% to about 90%. In some instances, a library favors uniform repeating sequence content. For example, polynucleotide frequency is uniform with a repeating sequence content in a range of about 10% to about 90%. In some instances, a library comprises polynucleotides with a repeating sequence percentage of about 10% to about 95%. In some instances, a library described herein comprises polynucleotides having greater than 30% different polynucleotides having a repeating sequence percentage from 10% to 30% or 70 to 90%. In some instances, a library described herein comprises polynucleotides having less than about 15% of the polynucleotides have a repeating sequence percentage from 10% to 30% or 60 to 90%.

Generation of polynucleotide libraries with a specified repeating sequence content in some cases occurs by combining at least 2 polynucleotide libraries with different repeating sequence content. In some instances, at least 2, 3, 4, 5, 6, 7, 10, or more than 10 polynucleotide libraries are combined to generate a population of polynucleotides with a specified repeating sequence content. In some cases, no more than 2, 3, 4, 5, 6, 7, or 10 polynucleotide libraries are combined to generate a population of non-identical polynucleotides with a specified repeating sequence content.

In some instances, repeating sequence content is adjusted by synthesizing fewer or more polynucleotides per cluster. For example, at least 25, 50, 100, 200, 300, 400, 500, 600, 700, 800, 900, 1000, or more than 1000 non-identical polynucleotides are synthesized on a single cluster. In some cases, no more than about 50, 100, 200, 300, 400, 500, 600, 700, 800, 900, 1000 non-identical polynucleotides are synthesized on a single cluster. In some instances, 50 to 500 non-identical polynucleotides are synthesized on a single cluster. In some instances, 100 to 200 non-identical polynucleotides are synthesized on a single cluster. In some instances, about 100, about 120, about 125, about 130, about 150, about 175, or about 200 non-identical polynucleotides are synthesized on a single cluster.

In some cases, repeating sequence content is adjusted by synthesizing non-identical polynucleotides of varying length. For example, the length of each of the non-identical polynucleotides synthesized may be at least or about at least 10, 15, 20, 25, 30, 35, 40, 45, 50, 100, 150, 200, 300, 400, 500, 2000 nucleotides, or more. The length of the non-identical polynucleotides synthesized may be at most or about at most 2000, 500, 400, 300, 200, 150, 100, 50, 45, 35, 30, 25, 20, 19, 18, 17, 16, 15, 14, 13, 12, 11, 10 nucleotides, or less. The length of each of the non-identical polynucleotides synthesized may fall from 10-2000, 10-500, 9-400, 11-300, 12-200, 13-150, 14-100, 15-50, 16-45, 17-40, 18-35, and 19-25.

### Generation of Polynucleotide Libraries with Controlled Stoichiometry of Secondary Structure Content

A polynucleotide library described herein may be synthesized with a specified secondary structure content. In some instances, adjusting polynucleotide libraries for secondary structure content results in improved polynucleotide representation.

A secondary structure can comprise three or more nucleotides in one or more polynucleotide strands that form a structure, such as a helix (e.g., alpha helix), a beta sheet, a stem-loop, pseudoknot, homodimer, or heterodimer. A stem-loop can be a hairpin loop, interior loop, bulge, or multiloop. Secondary structure type and their potential for formation can be predicted from sequence data. Folding or hybridization of linear sequences into secondary structures may occur while polynucleotides are attached to a solid support, or after cleavage into solution.

Secondary structure content in a polynucleotide library can be at least 10%, 20%, 30%, 40%, 50%, 60%, 70%, 80%, 90%, 95%, or more than 95%. In some instances, secondary structure content in a polynucleotide library is at most 10%, 20%, 30%, 40%, 50%, 60%, 70%, 80%, 90%, 95%, or no more than 100%. In some cases, secondary structure content is in a range of about 5-95%, 10-90%, 30-80%, 40-75%, or 50-70%.

Polynucleotide libraries can be adjusted for their secondary structure content. In some instances, polynucleotide libraries favor high secondary structure content. For example, a library is designed where increased polynucleotide frequency has a secondary structure content in a range of about 40% to about 90%. In some instances, polynucleotide libraries contain low secondary structure content. For example, a library is designed where increased polynucleotide frequency has a secondary structure content that is in a range of about 10% to about 60%. A library can be designed to favor high and low secondary structure content. For example, a library can be designed where increased polynucleotide frequency has a secondary structure content primarily in a range of about 10% and about 30% and in a range of about 70% to about 90%. In some instances, a library favors uniform secondary structure content. For example, polynucleotide frequency is uniform with a secondary structure content in a range of about 10% to about 90%. In some instances, a library comprises polynucleotides with a secondary structure percentage of about 10% to about 95%. In some instances, a library described herein comprises polynucleotides having greater than 30% different polynucleotides having a secondary structure percentage from 10% to 30% or 70 to 90%. In some instances, a library described herein comprises polynucleotides having less than about 15% of the polynucleotides have a secondary structure percentage from 10% to 30% or 60 to 90%.

Generation of polynucleotide libraries with a specified secondary structure content in some cases occurs by combining at least 2 polynucleotide libraries with different repeating sequence content. In some instances, at least 2, 3, 4, 5, 6, 7, 10, or more than 10 polynucleotide libraries are combined to generate a population of polynucleotides with a specified secondary structure content. In some cases, no more than 2, 3, 4, 5, 6, 7, or 10 polynucleotide libraries are combined to generate a population of non-identical polynucleotides with a specified secondary structure content.

In some instances, secondary structure content is adjusted by synthesizing fewer or more polynucleotides per cluster. For example, at least 25, 50, 100, 200, 300, 400, 500, 600, 700, 800, 900, 1000, or more than 1000 non-identical polynucleotides are synthesized on a single cluster. In some cases, no more than about 50, 100, 200, 300, 400, 500, 600, 700, 800, 900, 1000 non-identical polynucleotides are synthesized on a single cluster. In some instances, 50 to 500 non-identical polynucleotides are synthesized on a single cluster. In some instances, 100 to 200 non-identical polynucleotides are synthesized on a single cluster. In some instances, about 100, about 120, about 125, about 130, about 150, about 175, or about 200 non-identical polynucleotides are synthesized on a single cluster.

In some cases, secondary structure content is adjusted by synthesizing non-identical polynucleotides of varying length. For example, the length of each of the non-identical polynucleotides synthesized may be at least or about at least 10, 15, 20, 25, 30, 35, 40, 45, 50, 100, 150, 200, 300, 400, 500, 2000 nucleotides, or more. The length of the non-identical polynucleotides synthesized may be at most or about at most 2000, 500, 400, 300, 200, 150, 100, 50, 45, 35, 30, 25, 20, 19, 18, 17, 16, 15, 14, 13, 12, 11, 10 nucleotides, or less. The length of each of the non-identical polynucleotides synthesized may fall from 10-2000, 10-500, 9-400, 11-300, 12-200, 13-150, 14-100, 15-50, 16-45, 17-40, 18-35, and 19-25.

### Generation of Polynucleotide Libraries with Controlled Stoichiometry of Sequence Content

In some instances, the polynucleotide library is synthesized with a specified distribution of desired polynucleotide sequences. In some instances, adjusting polynucleotide libraries for enrichment of specific desired sequences results in improved downstream application outcomes.

One or more specific sequences can be selected based on their evaluation in a downstream application. In some instances, the evaluation is binding affinity to target sequences for amplification, enrichment, or detection, stability, melting temperature, biological activity, ability to assemble into larger fragments, or other property of polynucleotides. In some instances, the evaluation is empirical or predicted from prior experiments and/or computer algorithms.

Selected sequences in a polynucleotide library can be at least 10%, 20%, 30%, 40%, 50%, 60%, 70%, 80%, 90%, 95%, or more than 95% of the sequences. In some instances, selected sequences in a polynucleotide library are at most 10%, 20%, 30%, 40%, 50%, 60%, 70%, 80%, 90%, 95%, or at most 100% of the sequences. In some cases, selected sequences are in a range of about 5-95%, 10-90%, 30-80%, 40-75%, or 50-70% of the sequences.

Polynucleotide libraries can be adjusted for the frequency of each selected sequence. In some instances, polynucleotide libraries favor a higher number of selected sequences. For example, a library is designed where increased polynucleotide frequency of selected sequences is in a range of about 40% to about 90%. In some instances, polynucleotide libraries contain a low number of selected sequences. For example, a library is designed where increased polynucleotide frequency of the selected sequences is in a range of about 10% to about 60%. A library can be designed to favor a higher and lower frequency of selected sequences. In some instances, a library favors uniform sequence representation. For example, polynucleotide frequency is uniform with regard to selected sequence frequency, in a range of about 10% to about 90%. In some instances, a library comprises polynucleotides with a selected sequence frequency of about 10% to about 95% of the sequences.

Generation of polynucleotide libraries with a specified selected sequence frequency in some cases occurs by combining at least 2 polynucleotide libraries with different selected sequence frequency content. In some instances, at least 2, 3, 4, 5, 6, 7, 10, or more than 10 polynucleotide libraries are combined to generate a population of polynucleotides with a specified selected sequence frequency. In some cases, no more than 2, 3, 4, 5, 6, 7, or 10 polynucleotide libraries are combined to generate a population of non-identical polynucleotides with a specified selected sequence frequency.

In some instances, selected sequence frequency is adjusted by synthesizing fewer or more polynucleotides per cluster. For example, at least 25, 50, 100, 200, 300, 400, 500, 600, 700, 800, 900, 1000, or more than 1000 non-identical polynucleotides are synthesized on a single cluster. In some cases, no more than about 50, 100, 200, 300, 400, 500, 600, 700, 800, 900, 1000 non-identical polynucleotides are synthesized on a single cluster. In some instances, 50 to 500 non-identical polynucleotides are synthesized on a single cluster. In some instances, 100 to 200 non-identical polynucleotides are synthesized on a single cluster. In some instances, about 100, about 120, about 125, about 130, about 150, about 175, or about 200 non-identical polynucleotides are synthesized on a single cluster.

In some cases, selected sequence frequency is adjusted by synthesizing non-identical polynucleotides of varying length. For example, the length of each of the non-identical polynucleotides synthesized may be at least or about at least 10, 15, 20, 25, 30, 35, 40, 45, 50, 100, 150, 200, 300, 400, 500, 2000 nucleotides, or more. The length of the non-identical polynucleotides synthesized may be at most or about at most 2000, 500, 400, 300, 200, 150, 100, 50, 45, 35, 30, 25, 20, 19, 18, 17, 16, 15, 14, 13, 12, 11, 10 nucleotides, or less. The length of each of the non-identical polynucleotides synthesized may fall from 10-2000, 10-500, 9-400, 11-300, 12-200, 13-150, 14-100, 15-50, 16-45, 17-40, 18-35, and 19-25.

### Polynucleotide Probe Structures

Libraries of polynucleotide probes can be used to enrich particular target sequences in a larger population of sample polynucleotides. In some instances, polynucleotide probes each comprise an target binding sequence complementary to one or more target sequences, one or more non-target binding sequences, and one or more primer binding sites, such as universal primer binding sites. Target binding sequences that are complementary or at least partially complementary in some instances bind (hybridize) to target sequences. Primer binding sites, such as universal primer binding sites facilitate simultaneous amplification of all members of the probe library, or a subpopulation of members. In some instances, the probes further comprise a barcode or index sequence. Barcodes are nucleic acid sequences that allow some feature of a polynucleotide with which the barcode is associated to be identified. After sequencing, the barcode region provides an indicator for identifying a characteristic associated with the coding region or sample source. Barcodes can be designed at suitable lengths to allow sufficient degree of identification, e.g., at least about 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 ,36 ,37, 38, 39, 40, 41, 42, 43, 44, 45, 46, 47, 48, 49, 50, 51, 52, 53, 54, 55, or more bases in length. Multiple barcodes, such as about 2, 3, 4, 5, 6, 7, 8, 9, 10, or more barcodes, may be used on the same molecule, optionally separated by non-barcode sequences. In some embodiments, each barcode in a plurality of barcodes differ from every other barcode in the plurality at least three base positions, such as at least about 3, 4, 5, 6, 7, 8, 9, 10, or more positions. In some instances, the polynucleotides are ligated to one or more molecular (or affinity) tags such as a small molecule, peptide, antigen, metal, or protein to form a probe for subsequent capture of the target sequences of interest. In some instances, two probes that possess complementary target binding sequences which are capable of hybridization form a double stranded probe pair.

Probes described here may be complementary to target sequences which are sequences in a genome. Probes described here may be complementary to target sequences which are exome sequences in a genome. Probes described here may be complementary to target sequences which are intron sequences in a genome. In some instances, probes comprise an target binding sequence complementary to a target sequence, and at least one non-target binding sequence that is not complementary to the target. In some instances, the target binding sequence of the probe is about 120 nucleotides in length, or at least 10, 15, 20, 25, 50, 75, 100, 110, 120, 125, 140, 150, 160, 175, 200, 300, 400, 500, or more than 500 nucleotides in length. The target binding sequence is in some instances no more than 10, 15, 20, 25, 50, 75, 100, 125, 150, 175, 200, or no more than 500 nucleotides in length. The target binding sequence of the probe is in some instances about 120 nucleotides in length, or about 10, 15, 20, 25, 40, 50, 60, 70, 80, 85, 87, 90, 95, 97, 100, 105, 110, 115, 117, 118, 119, 120, 121, 122, 123, 124, 125, 126, 127, 128, 129, 130, 135, 140, 145, 150, 155, 157, 158, 159, 160, 161, 162, 163, 164, 165, 166, 167, 168, 169, 170, 175, 180, 190, 200, 210, 220, 230, 240, 250, 300, 400, or about 500 nucleotides in length. The target binding sequence is in some instances about 20 to about 400 nucleotides in length, or about 30 to about 175, about 40 to about 160, about 50 to about 150, about 75 to about 130, about 90 to about 120, or about 100 to about 140 nucleotides in length. The non-target binding sequence(s) of the probe is in some instances at least about 20 nucleotides in length, or at least about 1, 5, 10, 15, 17, 20, 23, 25, 50, 75, 100, 110, 120, 125, 140, 150, 160, 175, or more than about 175 nucleotides in length. The non-target binding sequence often is no more than about 5, 10, 15, 20, 25, 50, 75, 100, 125, 150, 175, or no more than about 200 nucleotides in length. The non-target binding sequence of the probe often is about 20 nucleotides in length, or about 1, 2, 3, 4, 5, 6, 7, 8, 9, 10, 11, 12, 13, 14, 15, 16, 17, 18, 19, 20, 21, 22, 23, 25, 40, 50, 60, 70, 80, 90, 100, 110, 120, 130, 140, 150, or about 200 nucleotides in length. The non-target binding sequence in some instances is about 1 to about 250 nucleotides in length, or about 20 to about 200, about 10 to about 100, about 10 to about 50, about 30 to about 100, about 5 to about 40, or about 15 to about 35 nucleotides in length. The non-target binding sequence often comprises sequences that are not complementary to the target sequence, and/or comprise sequences that are not used to bind primers. In some instances, the non-target binding sequence comprises a repeat of a single nucleotide, for example polyadenine or polythymidine. A probe often comprises none or at least one non-target binding sequence. In some instances, a probe comprises one or two non-target binding sequences. The non-target binding sequence may be adjacent to one or more target binding sequences in a probe. For example, an non-target binding sequence is located on the 5' or 3' end of the probe. In some instances, the non-target binding sequence is attached to a molecular tag or spacer.

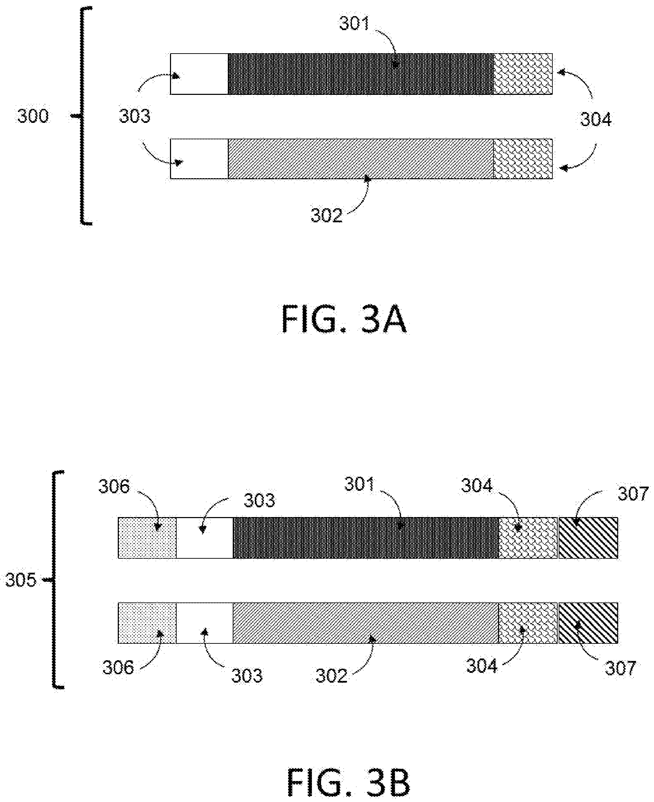

In some instances, the non-target binding sequence(s) may be a primer binding site. The primer binding sites often are each at least about 20 nucleotides in length, or at least about 10, 12, 14, 16, 18, 20, 22, 24, 26, 28, 30, 32, 34, 36, 38, or at least about 40 nucleotides in length. Each primer binding site in some instances is no more than about 10, 12, 14, 16, 18, 20, 22, 24, 26, 28, 30, 32, 34, 36, 38, or no more than about 40 nucleotides in length. Each primer binding site in some instances is about 10 to about 50 nucleotides in length, or about 15 to about 40, about 20 to about 30, about 10 to about 40, about 10 to about 30, about 30 to about 50, or about 20 to about 60 nucleotides in length. In some instances the polynucleotide probes comprise at least two primer binding sites. In some instances, primer binding sites may be universal primer binding sites, wherein all probes comprise identical primer binding sequences at these sites. In some instances, a pair of polynucleotide probes targeting a particular sequence and its reverse complement (e.g., a region of genomic DNA) are represented by **300** in **FIG. 3A****,** comprising a first target binding sequence **301,** a second target binding sequence **302,** a first non-target binding sequence **303,** and a second non-target binding sequence **304.** For example, a pair of polynucleotide probes complementary to a particular sequence (e.g., a region of genomic DNA).

In some instances, the first target binding sequence **301** is the reverse complement of the second target binding sequence **302.** In some instances, both target binding sequences are chemically synthesized prior to amplification. In an alternative arrangement, a pair of polynucleotide probes targeting a particular sequence and its reverse complement (e.g., a region of genomic DNA) are represented by **305** in **FIG. 3B****,** comprising a first target binding sequence **301,** a second target binding sequence **302,** a first non-target binding sequence **303,** a second non-target binding sequence **304,** a third non-target binding sequence **306,** and a fourth non-target binding sequence **307.** In some instances, the first target binding sequence **301** is the reverse complement of the second target binding sequence **302.** In some instances, one or more non-target binding sequences comprise polyadenine or polythymidine.

In some instances, both probes in the pair are labeled with at least one molecular tag. In some instances, PCR is used to introduce molecular tags (via primers comprising the molecular tag) onto the probes during amplification. In some instances, the molecular tag comprises one or more biotin, folate, a polyhistidine, a FLAG tag, glutathione, or other molecular tag consistent with the specification. In some instances probes are labeled at the 5' terminus. In some instances, the probes are labeled at the 3' terminus. In some instances, both the 5' and 3' termini are labeled with a molecular tag. In some instances, the 5' terminus of a first probe in a pair is labeled with at least one molecular tag, and the 3' terminus of a second probe in the pair is labeled with at least one molecular tag. In some instances, a spacer is present between one or more molecular tags and the nucleic acids of the probe. In some instances, the spacer may comprise an alkyl, polyol, or polyamino chain, a peptide, or a polynucleotide. The solid support used to capture probe-target nucleic acid complexes in some instances, is a bead or a surface. The solid support in some instances comprises glass, plastic, or other material capable of comprising a capture moiety that will bind the molecular tag. In some instances, a bead is a magnetic bead. For example, probes labeled with biotin are captured with a magnetic bead comprising streptavidin. The probes are contacted with a library of nucleic acids to allow binding of the probes to target sequences. In some instances, blocking polynucleic acids are added to prevent binding of the probes to one or more adapter sequences attached to the target nucleic acids. In some instances, blocking polynucleic acids comprise one or more nucleic acid analogues. In some instances, blocking polynucleic acids have a uracil substituted for thymine at one or more positions.

Probes described herein may comprise complementary target binding sequences which bind to one or more target nucleic acid sequences. In some instances, the target sequences are any DNA or RNA nucleic acid sequence. In some instances, target sequences may be longer than the probe insert. In some instance, target sequences may be shorter than the probe insert. In some instance, target sequences may be the same length as the probe insert. For example, the length of the target sequence may be at least or about at least 2, 10, 15, 20, 25, 30, 35, 40, 45, 50, 100, 150, 200, 300, 400, 500, 1000, 2000, 5,000, 12,000, 20,000 nucleotides, or more. The length of the target sequence may be at most or about at most 20,000, 12,000, 5,000, 2,000, 1,000, 500, 400, 300, 200, 150, 100, 50, 45, 35, 30, 25, 20, 19, 18, 17, 16, 15, 14, 13, 12, 11, 10, 2 nucleotides, or less. The length of the target sequence may fall from 2-20,000, 3-12,000, 5-5, 5000, 10-2,000, 10-1,000, 10-500, 9-400, 11-300, 12-200, 13-150, 14-100, 15-50, 16-45, 17-40, 18-35, and 19-25. The probe sequences may target sequences associated with specific genes, diseases, regulatory pathways, or other biological functions consistent with the specification.

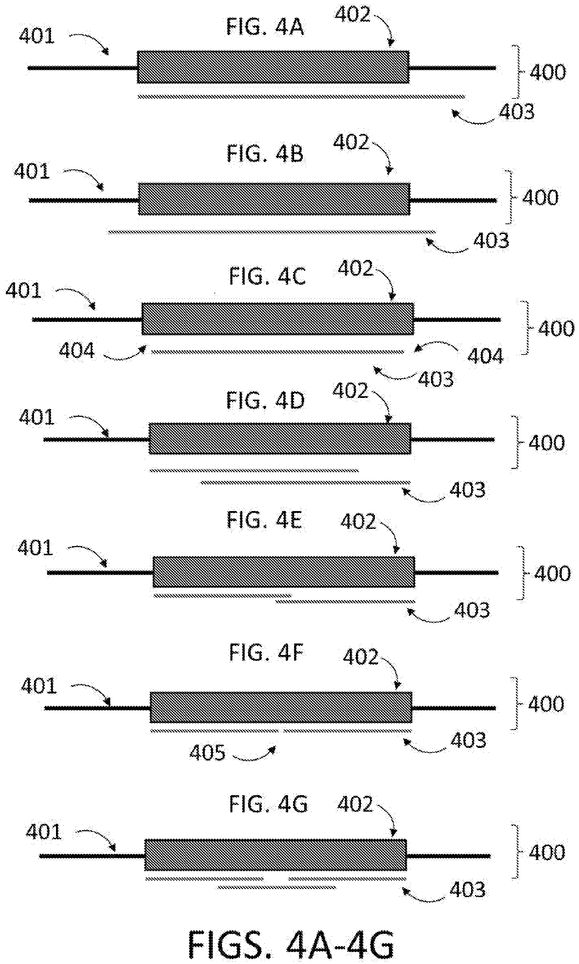

In some instances, a single probe insert **403** is complementary to one or more target sequences **402 (****FIGS. 4A-4G****)** in a larger polynucleic acid **400.** An exemplary target sequence is an exon. In some instances, one or more probes target a single target sequence **(****FIGS. 4A-4G****).** In some instances, a single probe may target more than one target sequence. In some instances, the target binding sequence of the probe targets both a target sequence **402** and an adjacent sequence **401 (****FIG. 4A and 4B****).** In some instances, a first probe targets a first region and a second region of a target sequence, and a second probe targets the second region and a third region of the target sequence **(****FIG. 4D** and **FIG. 4E****).** In some instances, a plurality of probes targets a single target sequence, wherein the target binding sequences of the plurality of probes contain one or more sequences which overlap with regard to complementarity to a region of the target sequence **(****FIG. 4G****).** In some instances, probe inserts do not overlap with regard to complementarity to a region of the target sequence. In some instances, at least at least 2, 10, 15, 20, 25, 30, 35, 40, 45, 50, 100, 150, 200, 300, 400, 500, 1000, 2000, 5,000, 12,000, 20,000, or more than 20,000 probes target a single target sequence. In some instances no more than 4 probes directed to a single target sequence overlap, or no more than 3, 2, 1, or no probes targeting a single target sequence overlap. In some instances, one or more probes do not target all bases in an target sequence, leaving one or more gaps **(****FIG. 4C** and **FIG. 4F****).** In some instances, the gaps are near the middle of the target sequence **405 (****FIG. 4F****).** In some instances, the gaps **404** are at the 5' or 3' ends of the target sequence **(****FIG. 4C****).** In some instances, the gaps are 6 nucleotides in length. In some instances, the gaps are no more than 1, 2, 3, 4, 5, 6, 7, 8, 9, 10, 20, 30, 40, or no more than 50 nucleotides in length. In some instances, the gaps are at least 1, 2, 3, 4, 5, 6, 7, 8, 9, 10, 20, 30, 40, or at least 50 nucleotides in length. In some instances, the gaps length falls within 1-50, 1-40, 1-30, 1-20, 1-10, 2-30, 2-20, 2-10, 3-50, 3-25, 3-10, or 3-8 nucleotides in length. In some instances, a set of probes targeting a sequence do not comprise overlapping regions amongst probes in the set when hybridized to complementary sequence. In some instances, a set of probes targeting a sequence do not have any gaps amongst probes in the set when hybridized to complementary sequence. Probes may be designed to maximize uniform binding to target sequences. In some instances, probes are designed to minimize target binding sequences of high or low GC content, secondary structure, repetitive/palindromic sequences, or other sequence feature that may interfere with probe binding to a target. In some instances, a single probe may target a plurality of target sequences.

A probe library described herein may comprise at least 10, 20, 50, 100, 200, 500, 1,000, 2,000, 5,000, 10,000, 20,000, 50,000, 100,000, 200,000, 500,000, 1,000,000 or more than 1,000,000 probes. A probe library may have no more than 10, 20, 50, 100, 200, 500, 1,000, 2,000, 5,000, 10,000, 20,000, 50,000, 100,000, 200,000, 500,000, or no more than 1,000,000 probes. A probe library may comprise 10 to 500, 20 to 1000, 50 to 2000, 100 to 5000, 500 to 10,000, 1,000 to 5,000, 10,000 to 50,000, 100,000 to 500,000, or to 50,000 to 1,000,000 probes. A probe library may comprise about 370,000; 400,000; 500,000 or more different probes.

### Next Generation Sequencing Applications

Downstream applications of polynucleotide libraries may include next generation sequencing. For example, enrichment of target sequences with a controlled stoichiometry polynucleotide probe library results in more efficient sequencing. The performance of a polynucleotide library for capturing or hybridizing to targets may be defined by a number of different metrics describing efficiency, accuracy, and precision. For example, Picard metrics comprise variables such as HS library size (the number of unique molecules in the library that correspond to target regions, calculated from read pairs), mean target coverage (the percentage of bases reaching a specific coverage level), depth of coverage (number of reads including a given nucleotide) fold enrichment (sequence reads mapping uniquely to the target/reads mapping to the total sample, multiplied by the total sample length/target length), percent off-bait bases (percent of bases not corresponding to bases of the probes/baits), usable bases on target, AT or GC dropout rate, fold 80 base penalty (fold over-coverage needed to raise 80 percent of non-zero targets to the mean coverage level), percent zero coverage targets, PF reads (the number of reads passing a quality filter), percent selected bases (the sum of on-bait bases and near-bait bases divided by the total aligned bases), percent duplication, or other variable consistent with the specification.

Read depth (sequencing depth, or sampling) represents the total number of times a sequenced nucleic acid fragment (a "read") is obtained for a sequence. Theoretical read depth is defined as the expected number of times the same nucleotide is read, assuming reads are perfectly distributed throughout an idealized genome. Read depth is expressed as function of % coverage (or coverage breadth). For example, 10 million reads of a 1 million base genome, perfectly distributed, theoretically results in 10X read depth of 100% of the sequences. Experimentally, a greater number of reads (higher theoretical read depth, or oversampling) may be needed to obtain the desired read depth for a percentage of the target sequences. Enrichment of target sequences with a controlled stoichiometry probe library increases the efficiency of downstream sequencing, as fewer total reads will be required to obtain an experimental outcome with an acceptable number of reads over a desired % of target sequences. For example, in some instances 55x theoretical read depth of target sequences results in at least 30x coverage of at least 90% of the sequences. In some instances no more than 55x theoretical read depth of target sequences results in at least 30x read depth of at least 80% of the sequences. In some instances no more than 55x theoretical read depth of target sequences results in at least 30x read depth of at least 95% of the sequences. In some instances no more than 55x theoretical read depth of target sequences results in at least 10x read depth of at least 98% of the sequences. In some instances, 55x theoretical read depth of target sequences results in at least 20x read depth of at least 98% of the sequences. In some instances no more than 55x theoretical read depth of target sequences results in at least 5x read depth of at least 98% of the sequences. Increasing the concentration of probes during hybridization with targets can lead to an increase in read depth. In some instances, the concentration of probes is increased by at least 1.5x, 2.0x, 2.5x, 3x, 3.5x, 4x, 5x, or more than 5x. In some instances, increasing the probe concentration results in at least a 1000% increase, or a 20%, 30%, 40%, 50%, 60%, 70%, 80%, 90%, 100%, 200%, 300%, 500%, 750%, 1000%, or more than a 1000% increase in read depth. In some instances, increasing the probe concentration by 3x results in a 1000% increase in read depth.

On-target rate represents the percentage of sequencing reads that correspond with the desired target sequences. In some instances, a controlled stoichiometry polynucleotide probe library results in an on-target rate of at least 30%, or at least 35%, 40%, 45%, 50%, 55%, 60%, 65%, 70%, 75%, 80%, 85%, or at least 90%. Increasing the concentration of polynucleotide probes during contact with target nucleic acids leads to an increase in the on-target rate. In some instances, the concentration of probes is increased by at least 1.5x, 2.0x, 2.5x, 3x, 3.5x, 4x, 5x, or more than 5x. In some instances, increasing the probe concentration results in at least a 20% increase, or a 10%, 20%, 30%, 40%, 50%, 60%, 70%, 80%, 90%, 100%, 200%, 300%, or at least a 500% increase in on-target binding. In some instances, increasing the probe concentration by 3x results in a 20% increase in on-target rate.

Coverage uniformity is in some cases calculated as the read depth as a function of the target sequence identity. Higher coverage uniformity results in a lower number of sequencing reads needed to obtain the desired read depth. For example, a property of the target sequence may affect the read depth, for example, high or low GC or AT content, repeating sequences, trailing adenines, secondary structure, affinity for target sequence binding (for amplification, enrichment, or detection), stability, melting temperature, biological activity, ability to assemble into larger fragments, sequences containing modified nucleotides or nucleotide analogues, or any other property of polynucleotides. Enrichment of target sequences with controlled stoichiometry polynucleotide probe libraries results in higher coverage uniformity after sequencing. In some instances, 95% of the sequences have a read depth that is within 1x of the mean library read depth, or about 0.05, 0.1, 0.2, 0.5, 0.7, 1, 1.2, 1.5, 1.7 or about within 2x the mean library read depth. In some instances, 80%, 85%, 90%, 95%, 97%, or 99% of the sequences have a read depth that is within 1x of the mean.

### Enrichment of Target Nucleic Acids with a Polynucleotide Probe Library

A probe library described herein may be used to enrich target polynucleotides present in a population of sample polynucleotides, for a variety of downstream applications. In some instances, a sample is obtained from one or more sources, and the population of sample polynucleotides is isolated using conventional techniques known in the art. Samples are obtained (by way of non-limiting example) from biological sources such as saliva, blood, tissue, skin, or completely synthetic sources. The plurality of polynucleotides obtained from the sample are fragmented, end-repaired, and adenylated to form a double stranded sample nucleic acid fragment. In some instances, end repair is accomplished by treatment with one or more enzymes, such as T4 DNA polymerase, klenow enzyme, and T4 polynucleotide kinase in an appropriate buffer. A nucleotide overhang to facilitate ligation to adapters is added, in some instances with 3' to 5' exo minus klenow fragment and dATP.

Adapters may be ligated to both ends of the sample polynucleotide fragments with a ligase, such as T4 ligase, to produce a library of adapter-tagged polynucleotide strands, and the adapter-tagged polynucleotide library is amplified with primers, such as universal primers. In some instances, the adapters are Y-shaped adapters comprising one or more primer binding sites, one or more grafting regions, and one or more index regions. In some instances, the one or more index region is present on each strand of the adapter. In some instances, grafting regions are complementary to a flowcell surface, and facilitate next generation sequencing of sample libraries. In some instances, Y-shaped adapters comprise partially complementary sequences. In some instances, Y-shaped adapters comprise a single thymidine overhang which hybridizes to the overhanging adenine of the double stranded adapter-tagged polynucleotide strands. Y-shaped adapters may comprise modified nucleic acids, that are resistant to cleavage. For example, a phosphorothioate backbone is used to attach an overhanging thymidine to the 3' end of the adapters. The library of double stranded sample nucleic acid fragments is then denatured in the presence of adapter blockers. Adapter blockers minimize off-target hybridization of probes to the adapter sequences (instead of target sequences) present on the adapter-tagged polynucleotide strands. Denaturation is carried out in some instances at 96°C, or at about 85, 87, 90, 92, 95, 97, 98 or about 99°C. A polynucleotide targeting library (probe library) is denatured in a hybridization solution, in some instances at 96°C, at about 85, 87, 90, 92, 95, 97, 98 or 99°C. The denatured adapter-tagged polynucleotide library and the hybridization solution are incubated for a suitable amount of time and at a suitable temperature to allow the probes to hybridize with their complementary target sequences. In some instances, a suitable hybridization temperature is about 45 to 80°C, or at least 45, 50, 55, 60, 65, 70, 75, 80, 85, or 90°C. In some instances, the hybridization temperature is 70°C. In some instances, a suitable hybridization time is 16 hours, or at least 4, 6, 8, 10, 12, 14, 16, 18, 20, 22, or more than 22 hours, or about 12 to 20 hours. Binding buffer is then added to the hybridized adapter-tagged-polynucleotide probes, and a solid support comprising a capture moiety are used to selectively bind the hybridized adapter-tagged polynucleotide-probes. The solid support is washed with buffer to remove unbound polynucleotides before an elution buffer is added to release the enriched, tagged polynucleotide fragments from the solid support. In some instances, the solid support is washed 2 times, or 1, 2, 3, 4, 5, or 6 times. The enriched library of adapter-tagged polynucleotide fragments is amplified and the enriched library is sequenced.

A plurality of nucleic acids (i.e. genomic sequence) may be obtained from a sample, and fragmented, optionally end-repaired, and adenylated. Adapters are ligated to both ends of the polynucleotide fragments to produce a library of adapter-tagged polynucleotide strands, and the adapter-tagged polynucleotide library is amplified. The adapter-tagged polynucleotide library is then denatured at high temperature, preferably 96°C, in the presence of adapter blockers. A polynucleotide targeting library (probe library) is denatured in a hybridization solution at high temperature, preferably about 90 to 99°C, and combined with the denatured, tagged polynucleotide library in hybridization solution for about 10 to 24 hours at about 45 to 80°C. Binding buffer is then added to the hybridized tagged polynucleotide probes, and a solid support comprising a capture moiety are used to selectively bind the hybridized adapter-tagged polynucleotide-probes. The solid support is washed one or more times with buffer, preferably about 2 and 5 times to remove unbound polynucleotides before an elution buffer is added to release the enriched, adapter-tagged polynucleotide fragments from the solid support. The enriched library of adapter-tagged polynucleotide fragments is amplified and then the library is sequenced. Alternative experimental variables such as incubation times, temperatures, reaction volumes/concentrations, number of washes, or other variables consistent with the specification are also employed in the method.

A population of polynucleotides may be enriched prior to adapter ligation. In one example, a plurality of polynucleotides is obtained from a sample, fragmented, optionally end-repaired, and denatured at high temperature, preferably 90-99°C. A polynucleotide targeting library (probe library) is denatured in a hybridization solution at high temperature, preferably about 90 to 99°C, and combined with the denatured, tagged polynucleotide library in hybridization solution for about 10 to 24 hours at about 45 to 80°C. Binding buffer is then added to the hybridized tagged polynucleotide probes, and a solid support comprising a capture moiety are used to selectively bind the hybridized adapter-tagged polynucleotide-probes. The solid support is washed one or more times with buffer, preferably about 2 and 5 times to remove unbound polynucleotides before an elution buffer is added to release the enriched, adapter-tagged polynucleotide fragments from the solid support. The enriched polynucleotide fragments are then polyadenylated, adapters are ligated to both ends of the polynucleotide fragments to produce a library of adapter-tagged polynucleotide strands, and the adapter-tagged polynucleotide library is amplified. The adapter-tagged polynucleotide library is then sequenced.

A polynucleotide targeting library may also be used to filter undesired sequences from a plurality of polynucleotides, by hybridizing to undesired fragments. For example, a plurality of polynucleotides is obtained from a sample, and fragmented, optionally end-repaired, and adenylated. Adapters are ligated to both ends of the polynucleotide fragments to produce a library of adapter-tagged polynucleotide strands, and the adapter-tagged polynucleotide library is amplified. Alternatively, adenylation and adapter ligation steps are instead performed after enrichment of the sample polynucleotides. The adapter-tagged polynucleotide library is then denatured at high temperature, preferably 90-99°C, in the presence of adapter blockers. A polynucleotide filtering library (probe library) designed to remove undesired, non-target sequences is denatured in a hybridization solution at high temperature, preferably about 90 to 99°C, and combined with the denatured, tagged polynucleotide library in hybridization solution for about 10 to 24 hours at about 45 to 80°C. Binding buffer is then added to the hybridized tagged polynucleotide probes, and a solid support comprising a capture moiety are used to selectively bind the hybridized adapter-tagged polynucleotide-probes. The solid support is washed one or more times with buffer, preferably about 1 and 5 times to elute unbound adapter-tagged polynucleotide fragments . The enriched library of unbound adapter-tagged polynucleotide fragments is amplified and then the amplified library is sequenced.

### Highly Parallel De Novo Nucleic Acid Synthesis

Described herein but not encompassed by the appended claims is a platform approach utilizing miniaturization, parallelization, and vertical integration of the end-to-end process from polynucleotide synthesis to gene assembly within Nano wells on silicon to create a revolutionary synthesis platform. Devices described herein provide, with the same footprint as a 96-well plate, a silicon synthesis platform is capable of increasing throughput by a factor of 100 to 1,000 compared to traditional synthesis methods, with production of up to approximately 1,000,000 polynucleotides in a single highly-parallelized run. In some instances, a single silicon plate described herein provides for synthesis of about 6,100 non-identical polynucleotides. In some instances, each of the non-identical polynucleotides is located within a cluster. A cluster may comprise 50 to 500 non-identical polynucleotides.

Methods described herein but not encompassed by the appended claims provide for synthesis of a library of polynucleotides each encoding for a predetermined variant of at least one predetermined reference nucleic acid sequence. In some cases, the predetermined reference sequence is nucleic acid sequence encoding for a protein, and the variant library comprises sequences encoding for variation of at least a single codon such that a plurality of different variants of a single residue in the subsequent protein encoded by the synthesized nucleic acid are generated by standard translation processes. The synthesized specific alterations in the nucleic acid sequence can be introduced by incorporating nucleotide changes into overlapping or blunt ended polynucleotide primers. Alternatively, a population of polynucleotides may collectively encode for a long nucleic acid (e.g., a gene) and variants thereof. In this arrangement, the population of polynucleotides can be hybridized and subject to standard molecular biology techniques to form the long nucleic acid (e.g., a gene) and variants thereof. When the long nucleic acid (e.g., a gene) and variants thereof are expressed in cells, a variant protein library is generated. Similarly, disclosed here are methods for synthesis of variant libraries encoding for RNA sequences (e.g., miRNA, shRNA, and mRNA) or DNA sequences (e.g., enhancer, promoter, UTR, and terminator regions). Also disclosed here are downstream applications for variants selected out of the libraries synthesized using methods described here. Downstream applications include identification of variant nucleic acid or protein sequences with enhanced biologically relevant functions, e.g., biochemical affinity, enzymatic activity, changes in cellular activity, and for the treatment or prevention of a disease state.

### Substrates

Disclosed herein but not encompassed by the appended claims are substrates comprising a plurality of clusters, wherein each cluster comprises a plurality of loci that support the attachment and synthesis of polynucleotides. The term "locus" as used herein refers to a discrete region on a structure which provides support for polynucleotides encoding for a single predetermined sequence to extend from the surface. In some instances, a locus is on a two dimensional surface, *e.g.,* a substantially planar surface. In some instances, a locus refers to a discrete raised or lowered site on a surface *e.g.,* a well, micro well, channel, or post. In some instances, a surface of a locus comprises a material that is actively functionalized to attach to at least one nucleotide for polynucleotide synthesis, or preferably, a population of identical nucleotides for synthesis of a population of polynucleotides. In some instances, polynucleotide refers to a population of polynucleotides encoding for the same nucleic acid sequence. In some instances, a surface of a device is inclusive of one or a plurality of surfaces of a substrate.

Disclosed herein but not encompassed by the appended claims are structures that may comprise a surface that supports the synthesis of a plurality of polynucleotides having different predetermined sequences at addressable locations on a common support. In some instances, a device provides support for the synthesis of more than 2,000; 5,000; 10,000; 20,000; 30,000; 50,000; 75,000; 100,000; 200,000; 300,000; 400,000; 500,000; 600,000; 700,000; 800,000; 900,000; 1,000,000; 1,200,000; 1,400,000; 1,600,000; 1,800,000; 2,000,000; 2,500,000; 3,000,000; 3,500,000; 4,000,000; 4,500,000; 5,000,000; 10,000,000 or more non-identical polynucleotides. In some instances, the device provides support for the synthesis of more than 2,000; 5,000; 10,000; 20,000; 30,000; 50,000; 75,000; 100,000; 200,000; 300,000; 400,000; 500,000; 600,000; 700,000; 800,000; 900,000; 1,000,000; 1,200,000; 1,400,000; 1,600,000; 1,800,000; 2,000,000; 2,500,000; 3,000,000; 3,500,000; 4,000,000; 4,500,000; 5,000,000; 10,000,000 or more polynucleotides encoding for distinct sequences. In some instances, at least a portion of the polynucleotides have an identical sequence or are configured to be synthesized with an identical sequence.

Disclosed herein but not encompassed by the appended claims are methods and devices for manufacture and growth of polynucleotides about 5, 10, 20, 30, 40, 50, 60, 70, 80, 90, 100, 125, 150, 175, 200, 225, 250, 275, 300, 325, 350, 375, 400, 425, 450, 475, 500, 600, 700, 800, 900, 1000, 1100, 1200, 1300, 1400, 1500, 1600, 1700, 1800, 1900, or 2000 bases in length. In some instances, the length of the polynucleotide formed is about 5, 10, 20, 30, 40, 50, 60, 70, 80, 90, 100, 125, 150, 175, 200, or 225 bases in length. A polynucleotide may be at least 5, 10, 20, 30, 40, 50, 60, 70, 80, 90, or 100 bases in length. A polynucleotide may be from 10 to 225 bases in length, from 12 to 100 bases in length, from 20 to 150 bases in length, from 20 to 130 bases in length, or from 30 to 100 bases in length.

In some instances, polynucleotides are synthesized on distinct loci of a substrate, wherein each locus supports the synthesis of a population of polynucleotides. In some instances, each locus supports the synthesis of a population of polynucleotides having a different sequence than a population of polynucleotides grown on another locus. In some instances, the loci of a device are located within a plurality of clusters. In some instances, a device comprises at least 10, 500, 1000, 2000, 3000, 4000, 5000, 6000, 7000, 8000, 9000, 10000, 11000, 12000, 13000, 14000, 15000, 20000, 30000, 40000, 50000 or more clusters. In some instances, a device comprises more than 2,000; 5,000; 10,000; 100,000; 200,000; 300,000; 400,000; 500,000; 600,000; 700,000; 800,000; 900,000; 1,000,000; 1,100,000; 1,200,000; 1,300,000; 1,400,000; 1,500,000; 1,600,000; 1,700,000; 1,800,000; 1,900,000; 2,000,000; 300,000; 400,000; 500,000; 600,000; 700,000; 800,000; 900,000; 1,000,000; 1,200,000; 1,400,000; 1,600,000; 1,800,000; 2,000,000; 2,500,000; 3,000,000; 3,500,000; 4,000,000; 4,500,000; 5,000,000; or 10,000,000 or more distinct loci. In some instances, a device comprises about 10,000 distinct loci. The amount of loci within a single cluster is varied in different instances. In some instances, each cluster includes 1, 2, 3, 4, 5, 6, 7, 8, 9, 10, 20, 30, 40, 50, 60, 70, 80, 90, 100, 120, 130, 150, 200, 300, 400, 500, 1000 or more loci. In some instances, each cluster includes about 50-500 loci. In some instances, each cluster includes about 100-200 loci. In some instances, each cluster includes about 100-150 loci. In some instances, each cluster includes about 109, 121, 130 or 137 loci. In some instances, each cluster includes about 19, 20, 61, 64 or more loci.

The number of distinct polynucleotides synthesized on a device may be dependent on the number of distinct loci available in the substrate. In some instances, the density of loci within a cluster of a device is at least or about 1 locus per mm², 10 loci per mm², 25 loci per mm², 50 loci per mm², 65 loci per mm², 75 loci per mm², 100 loci per mm², 130 loci per mm², 150 loci per mm², 175 loci per mm², 200 loci per mm², 300 loci per mm², 400 loci per mm², 500 loci per mm², 1,000 loci per mm² or more. In some instances, a device comprises from about 10 loci per mm² to about 500 mm², from about 25 loci per mm² to about 400 mm², from about 50 loci per mm² to about 500 mm², from about 100 loci per mm² to about 500 mm², from about 150 loci per mm² to about 500 mm², from about 10 loci per mm² to about 250 mm², from about 50 loci per mm² to about 250 mm², from about 10 loci per mm² to about 200 mm², or from about 50 loci per mm² to about 200 mm². In some instances, the distance from the centers of two adjacent loci within a cluster is from about 10 um to about 500 um, from about 10 um to about 200 um, or from about 10 um to about 100 um. In some instances, the distance from two centers of adjacent loci is greater than about 10 um, 20 um, 30 um, 40 um, 50 um, 60 um, 70 um, 80 um, 90 um or 100 um. In some instances, the distance from the centers of two adjacent loci is less than about 200 um, 150 um, 100 um, 80 um, 70 um, 60 um, 50 um, 40 um, 30 um, 20 um or 10 um. In some instances, each locus has a width of about 0.5 um, 1 um, 2 um, 3 um, 4 um, 5 um, 6 um, 7 um, 8 um, 9 um, 10 um, 20 um, 30 um, 40 um, 50 um, 60 um, 70 um, 80 um, 90 um or 100 um. In some instances, the each locus is has a width of about 0.5 um to 100um, about 0.5 um to 50 um, about 10 um to 75 um, or about 0.5 um to 50 um.

In some instances, the density of clusters within a device is at least or about 1 cluster per 100 mm², 1 cluster per 10 mm², 1 cluster per 5 mm², 1 cluster per 4 mm², 1 cluster per 3 mm², 1 cluster per 2 mm², 1 cluster per 1 mm², 2 clusters per 1 mm², 3 clusters per 1 mm², 4 clusters per 1 mm², 5 clusters per 1 mm², 10 clusters per 1 mm², 50 clusters per 1 mm² or more. In some instances, a device comprises from about 1 cluster per 10 mm² to about 10 clusters per 1 mm². In some instances, the distance from the centers of two adjacent clusters is less than about 50 um, 100 um, 200 um, 500 um, 1000 um, or 2000 um or 5000 um. In some instances, the distance from the centers of two adjacent clusters is from about 50 um and about 100 um, from about 50 um and about 200 um, from about 50 um and about 300 um, from about 50 um and about 500 um, and from about 100 um to about 2000 um. In some instances, the distance from the centers of two adjacent clusters is from about 0.05 mm to about 50 mm, from about 0.05 mm to about 10 mm, from about 0.05 mm and about 5 mm, from about 0.05 mm and about 4 mm, from about 0.05 mm and about 3 mm, from about 0.05 mm and about 2 mm, from about 0.1 mm and 10 mm, from about 0.2 mm and 10 mm, from about 0.3 mm and about 10 mm, from about 0.4 mm and about 10 mm, from about 0.5 mm and 10 mm, from about 0.5 mm and about 5 mm, or from about 0.5 mm and about 2 mm. In some instances, each cluster has a diameter or width along one dimension of about 0.5 to 2 mm, about 0.5 to 1 mm, or about 1 to 2 mm. In some instances, each cluster has a diameter or width along one dimension of about 0.5, 0.6, 0.7, 0.8, 0.9, 1, 1.1, 1.2, 1.3, 1.4, 1.5, 1.6, 1.7, 1.8, 1.9 or 2 mm. In some instances, each cluster has an interior diameter or width along one dimension of about 0.5, 0.6, 0.7, 0.8, 0.9, 1, 1.1, 1.15, 1.2, 1.3, 1.4, 1.5, 1.6, 1.7, 1.8, 1.9 or 2 mm.

A device may be about the size of a standard 96 well plate, for example from about 100 and 200 mm by from about 50 and 150 mm. In some instances, a device has a diameter less than or equal to about 1000 mm, 500 mm, 450 mm, 400 mm, 300 mm, 250 nm, 200 mm, 150 mm, 100 mm or 50 mm. In some instances, the diameter of a device is from about 25 mm and 1000 mm, from about 25 mm and about 800 mm, from about 25 mm and about 600 mm, from about 25 mm and about 500 mm, from about 25 mm and about 400 mm, from about 25 mm and about 300 mm, or from about 25 mm and about 200. Non-limiting examples of device size include about 300 mm, 200 mm, 150 mm, 130 mm, 100 mm, 76 mm, 51 mm and 25 mm. In some instances, a device has a planar surface area of at least about 100 mm²; 200 mm²; 500 mm²; 1,000 mm²; 2,000 mm²; 5,000 mm²; 10,000 mm²; 12,000 mm²; 15,000 mm²; 20,000 mm²; 30,000 mm²; 40,000 mm²; 50,000 mm² or more. In some instances, the thickness of a device is from about 50 mm and about 2000 mm, from about 50 mm and about 1000 mm, from about 100 mm and about 1000 mm, from about 200 mm and about 1000 mm, or from about 250 mm and about 1000 mm. Non-limiting examples of device thickness include 275 mm, 375 mm, 525 mm, 625 mm, 675 mm, 725 mm, 775 mm and 925 mm. In some instances, the thickness of a device varies with diameter and depends on the composition of the substrate. For example, a device comprising materials other than silicon has a different thickness than a silicon device of the same diameter. Device thickness may be determined by the mechanical strength of the material used and the device must be thick enough to support its own weight without cracking during handling. In some instances, a structure comprises a plurality of devices described herein.

### Surface Materials

Disclosed herein but not encompassed by the appended claims is a device comprising a surface, wherein the surface is modified to support polynucleotide synthesis at predetermined locations and with a resulting low error rate, a low dropout rate, a high yield, and a high oligo representation. In some instances, surfaces of a device for polynucleotide synthesis provided herein are fabricated from a variety of materials capable of modification to support a de novo polynucleotide synthesis reaction. In some cases, the devices are sufficiently conductive, e.g., are able to form uniform electric fields across all or a portion of the device. A device described herein may comprise a flexible material. Exemplary flexible materials include, without limitation, modified nylon, unmodified nylon, nitrocellulose, and polypropylene. A device described herein may comprise a rigid material. Exemplary rigid materials include, without limitation, glass, fuse silica, silicon, silicon dioxide, silicon nitride, plastics (for example, polytetrafluoroethylene, polypropylene, polystyrene, polycarbonate, and blends thereof, and metals (for example, gold, platinum). Device disclosed herein may be fabricated from a material comprising silicon, polystyrene, agarose, dextran, cellulosic polymers, polyacrylamides, polydimethylsiloxane (PDMS), glass, or any combination thereof. In some cases, a device disclosed herein is manufactured with a combination of materials listed herein or any other suitable material known in the art.

A listing of tensile strengths for exemplary materials described herein is provides as follows: nylon (70 MPa), nitrocellulose (1.5 MPa), polypropylene (40 MPa), silicon (268 MPa), polystyrene (40 MPa), agarose (1-10 MPa), polyacrylamide (1-10 MPa), polydimethylsiloxane (PDMS) (3.9-10.8 MPa). Solid supports described herein can have a tensile strength from 1 to 300, 1 to 40, 1 to 10, 1 to 5, or 3 to 11 MPa. Solid supports described herein can have a tensile strength of about 1, 1.5, 2, 3, 4, 5, 6, 7, 8, 9, 10, 11, 20, 25, 40, 50, 60, 70, 80, 90, 100, 150, 200, 250, 270, or more MPa. In some instances, a device described herein comprises a solid support for polynucleotide synthesis that is in the form of a flexible material capable of being stored in a continuous loop or reel, such as a tape or flexible sheet.

Young's modulus measures the resistance of a material to elastic (recoverable) deformation under load. A listing of Young's modulus for stiffness of exemplary materials described herein is provides as follows: nylon (3 GPa), nitrocellulose (1.5 GPa), polypropylene (2 GPa), silicon (150 GPa), polystyrene (3 GPa), agarose (1-10 GPa), polyacrylamide (1-10 GPa), polydimethylsiloxane (PDMS) (1-10 GPa). Solid supports described herein can have a Young's moduli from 1 to 500, 1 to 40, 1 to 10, 1 to 5, or 3 to 11 GPa. Solid supports described herein can have a Young's moduli of about 1, 1.5, 2, 3, 4, 5, 6, 7, 8, 9, 10, 11, 20, 25, 40, 50, 60, 70, 80, 90, 100, 150, 200, 250, 400, 500 GPa, or more. As the relationship between flexibility and stiffness are inverse to each other, a flexible material has a low Young's modulus and changes its shape considerably under load.

In some cases, a device disclosed herein comprises a silicon dioxide base and a surface layer of silicon oxide. Alternatively, the device may have a base of silicon oxide. Surface of the device provided here may be textured, resulting in an increase overall surface area for polynucleotide synthesis. Device disclosed herein may comprise at least 5 %, 10%, 25%, 50%, 80%, 90%, 95%, or 99% silicon. A device disclosed herein may be fabricated from a silicon on insulator (SOI) wafer.

### Surface Architecture

Described herein but not encompassed by the appended claims are devices comprising raised and/or lowered features. One benefit of having such features is an increase in surface area to support polynucleotide synthesis. In some instances, a device having raised and/or lowered features is referred to as a three-dimensional substrate. In some instances, a three-dimensional device comprises one or more channels. In some instances, one or more loci comprise a channel. In some instances, the channels are accessible to reagent deposition via a deposition device such as a polynucleotide synthesizer. In some instances, reagents and/or fluids collect in a larger well in fluid communication one or more channels. For example, a device comprises a plurality of channels corresponding to a plurality of loci with a cluster, and the plurality of channels are in fluid communication with one well of the cluster. In some methods, a library of polynucleotides is synthesized in a plurality of loci of a cluster.

In some instances, the structure is configured to allow for controlled flow and mass transfer paths for polynucleotide synthesis on a surface. In some instances, the configuration of a device allows for the controlled and even distribution of mass transfer paths, chemical exposure times, and/or wash efficacy during polynucleotide synthesis. In some instances, the configuration of a device allows for increased sweep efficiency, for example by providing sufficient volume for a growing a polynucleotide such that the excluded volume by the growing polynucleotide does not take up more than 50, 45, 40, 35, 30, 25, 20, 15, 14, 13, 12, 11, 10, 9, 8, 7, 6, 5, 4, 3, 2, 1%, or less of the initially available volume that is available or suitable for growing the polynucleotide. In some instances, a three-dimensional structure allows for managed flow of fluid to allow for the rapid exchange of chemical exposure.

Disclosed herein but not encompassed by the appended claims are methods to synthesize an amount of DNA of 1 fM, 5 fM, 10 fM, 25 fM, 50 fM, 75 fM, 100 fM, 200 fM, 300 fid, 400 fM, 500 fM, 600 fid, 700 fid, 800 fid, 900 fM, 1 pM, 5 pM, 10 pM, 25 pM, 50 pM, 75 pM, 100 pM, 200 pM, 300 pM, 400 pM, 500 pM, 600 pM, 700 pM, 800 pM, 900 pM, or more. In some instances, a polynucleotide library may span the length of about 1 %, 2 %, 3 %, 4 %, 5 %, 10 %, 15 %, 20 %, 30 %, 40 %, 50 %, 60 %, 70 %, 80 %, 90 %, 95 %, or 100 % of a gene. A gene may be varied up to about 1 %, 2 %, 3 %, 4 %, 5 %, 10 %, 15 %, 20 %, 30 %, 40 %, 50 %, 60 %, 70 %, 80 %, 85%, 90 %, 95 %, or 100 %.

Non-identical polynucleotides may collectively encode a sequence for at least 1 %, 2 %, 3 %, 4 %, 5 %, 10 %, 15 %, 20 %, 30 %, 40 %, 50 %, 60 %, 70 %, 80 %, 85%, 90 %, 95 %, or 100 % of a gene. In some instances, a polynucleotide may encode a sequence of 50 %, 60 %, 70 %, 80 %, 85%, 90 %, 95 %, or more of a gene. In some instances, a polynucleotide may encode a sequence of 80 %, 85%, 90 %, 95 %, or more of a gene.

In some instances, segregation is achieved by physical structure. In some instances, segregation is achieved by differential functionalization of the surface generating active and passive regions for polynucleotide synthesis. Differential functionalization is also be achieved by alternating the hydrophobicity across the device surface, thereby creating water contact angle effects that cause beading or wetting of the deposited reagents. Employing larger structures can decrease splashing and cross-contamination of distinct polynucleotide synthesis locations with reagents of the neighboring spots. In some instances, a device, such as a polynucleotide synthesizer, is used to deposit reagents to distinct polynucleotide synthesis locations. Substrates having three-dimensional features are configured in a manner that allows for the synthesis of a large number of polynucleotides (*e.g*., more than about 10,000) with a low error rate (*e.g.,* less than about 1:500, 1:1000, 1:1500, 1:2,000; 1:3,000; 1:5,000; or 1:10,000). In some instances, a device comprises features with a density of about or greater than about 1, 5, 10, 20, 30, 40, 50, 60, 70, 80, 100, 110, 120, 130, 140, 150, 160, 170, 180, 190, 200, 300, 400 or 500 features per mm².

A well of a device may have the same or different width, height, and/or volume as another well of the substrate. A channel of a device may have the same or different width, height, and/or volume as another channel of the substrate. In some instances, the width of a cluster is from about 0.05 mm to about 50 mm, from about 0.05 mm to about 10 mm, from about 0.05 mm and about 5 mm, from about 0.05 mm and about 4 mm, from about 0.05 mm and about 3 mm, from about 0.05 mm and about 2 mm, from about 0.05 mm and about 1 mm, from about 0.05 mm and about 0.5 mm, from about 0.05 mm and about 0.1 mm, from about 0.1 mm and 10 mm, from about 0.2 mm and 10 mm, from about 0.3 mm and about 10 mm, from about 0.4 mm and about 10 mm, from about 0.5 mm and 10 mm, from about 0.5 mm and about 5 mm, or from about 0.5 mm and about 2 mm. In some instances, the width of a well comprising a cluster is from about 0.05 mm to about 50 mm, from about 0.05 mm to about 10 mm, from about 0.05 mm and about 5 mm, from about 0.05 mm and about 4 mm, from about 0.05 mm and about 3 mm, from about 0.05 mm and about 2 mm, from about 0.05 mm and about 1 mm, from about 0.05 mm and about 0.5 mm, from about 0.05 mm and about 0.1 mm, from about 0.1 mm and 10 mm, from about 0.2 mm and 10 mm, from about 0.3 mm and about 10 mm, from about 0.4 mm and about 10 mm, from about 0.5 mm and 10 mm, from about 0.5 mm and about 5 mm, or from about 0.5 mm and about 2 mm. In some instances, the width of a cluster is less than or about 5 mm, 4 mm, 3 mm, 2 mm, 1 mm, 0.5 mm, 0.1 mm, 0.09 mm, 0.08 mm, 0.07 mm, 0.06 mm or 0.05 mm. In some instances, the width of a cluster is from about 1.0 and 1.3 mm. In some instances, the width of a cluster is about 1.150 mm. In some instances, the width of a well is less than or about 5 mm, 4 mm, 3 mm, 2 mm, 1 mm, 0.5 mm, 0.1 mm, 0.09 mm, 0.08 mm, 0.07 mm, 0.06 mm or 0.05 mm. In some instances, the width of a well is from about 1.0 and 1.3 mm. In some instances, the width of a well is about 1.150 mm. In some instances, the width of a cluster is about 0.08 mm. In some instances, the width of a well is about 0.08 mm. The width of a cluster may refer to clusters within a two-dimensional or three-dimensional substrate.

In some instances, the height of a well is from about 20 um to about 1000 um, from about 50 um to about 1000 um, from about 100 um to about 1000 um, from about 200 um to about 1000 um, from about 300 um to about 1000 um, from about 400 um to about 1000 um, or from about 500 um to about 1000 um. In some instances, the height of a well is less than about 1000 um, less than about 900 um, less than about 800 um, less than about 700 um, or less than about 600 um.

In some instances, a device comprises a plurality of channels corresponding to a plurality of loci within a cluster, wherein the height or depth of a channel is from about 5 um to about 500 um, from about 5 um to about 400 um, from about 5 um to about 300 um, from about 5 um to about 200 um, from about 5 um to about 100 um, from about 5 um to about 50 um, or from about 10 um to about 50 um. In some instances, the height of a channel is less than 100 um, less than 80 um, less than 60 um, less than 40 um or less than 20 um.

In some instances, the diameter of a channel, locus (*e.g*., in a substantially planar substrate) or both channel and locus (*e.g*., in a three-dimensional device wherein a locus corresponds to a channel) is from about 1 um to about 1000 um, from about 1 um to about 500 um, from about 1 um to about 200 um, from about 1 um to about 100 um, from about 5 um to about 100 um, or from about 10 um to about 100 um, for example, about 90 um, 80 um, 70 um, 60 um, 50 um, 40 um, 30 um, 20 um or 10 um. In some instances, the diameter of a channel, locus, or both channel and locus is less than about 100 um, 90 um, 80 um, 70 um, 60 um, 50 um, 40 um, 30 um, 20 um or 10 um. In some instances, the distance from the center of two adjacent channels, loci, or channels and loci is from about 1 um to about 500 um, from about 1 um to about 200 um, from about 1 um to about 100 um, from about 5 um to about 200 um, from about 5 um to about 100 um, from about 5 um to about 50 um, or from about 5 um to about 30 um, for example, about 20 um.

### Surface Modifications

In various instances, surface modifications are employed for the chemical and/or physical alteration of a surface by an additive or subtractive process to change one or more chemical and/or physical properties of a device surface or a selected site or region of a device surface. For example, surface modifications include, without limitation, (1) changing the wetting properties of a surface, (2) functionalizing a surface, i.e., providing, modifying or substituting surface functional groups, (3) defunctionalizing a surface, i.e., removing surface functional groups, (4) otherwise altering the chemical composition of a surface, *e.g*., through etching, (5) increasing or decreasing surface roughness, (6) providing a coating on a surface, *e.g*., a coating that exhibits wetting properties that are different from the wetting properties of the surface, and/or (7) depositing particulates on a surface.

In some instances, the addition of a chemical layer on top of a surface (referred to as adhesion promoter) facilitates structured patterning of loci on a surface of a substrate. Exemplary surfaces for application of adhesion promotion include, without limitation, glass, silicon, silicon dioxide and silicon nitride. In some instances, the adhesion promoter is a chemical with a high surface energy. In some instances, a second chemical layer is deposited on a surface of a substrate. In some instances, the second chemical layer has a low surface energy. In some instances, surface energy of a chemical layer coated on a surface supports localization of droplets on the surface. Depending on the patterning arrangement selected, the proximity of loci and/or area of fluid contact at the loci are alterable.

In some instances, a device surface, or resolved loci, onto which nucleic acids or other moieties are deposited, *e.g.,* for polynucleotide synthesis, are smooth or substantially planar (*e.g.,* two-dimensional) or have irregularities, such as raised or lowered features (*e.g*., three-dimensional features). In some instances, a device surface is modified with one or more different layers of compounds. Such modification layers of interest include, without limitation, inorganic and organic layers such as metals, metal oxides, polymers, small organic molecules and the like. Non-limiting polymeric layers include peptides, proteins, nucleic acids or mimetics thereof (*e.g*., peptide nucleic acids and the like), polysaccharides, phospholipids, polyurethanes, polyesters, polycarbonates, polyureas, polyamides, polyethyleneamines, polyarylene sulfides, polysiloxanes, polyimides, polyacetates, and any other suitable compounds described herein or otherwise known in the art. In some instances, polymers are heteropolymeric. In some instances, polymers are homopolymeric. In some instances, polymers comprise functional moieties or are conjugated.

In some instances, resolved loci of a device are functionalized with one or more moieties that increase and/or decrease surface energy. In some instances, a moiety is chemically inert. In some instances, a moiety is configured to support a desired chemical reaction, for example, one or more processes in a polynucleotide synthesis reaction. The surface energy, or hydrophobicity, of a surface is a factor for determining the affinity of a nucleotide to attach onto the surface. In some instances, a method for device functionalization may comprise: (a) providing a device having a surface that comprises silicon dioxide; and (b) silanizing the surface using, a suitable silanizing agent described herein or otherwise known in the art, for example, an organofunctional alkoxysilane molecule.

In some instances, the organofunctional alkoxysilane molecule comprises dimethylchloro-octodecyl-silane, methyldichloro-octodecyl-silane, trichloro-octodecyl-silane, trimethyl-octodecyl-silane, triethyl-octodecyl-silane, or any combination thereof. In some instances, a device surface comprises functionalized with polyethylene/polypropylene (functionalized by gamma irradiation or chromic acid oxidation, and reduction to hydroxyalkyl surface), highly crosslinked polystyrene-divinylbenzene (derivatized by chloromethylation, and aminated to benzylamine functional surface), nylon (the terminal aminohexyl groups are directly reactive), or etched with reduced polytetrafluoroethylene. Other methods and functionalizing agents are described in U.S. Patent No. 5474796.

In some instances, a device surface is functionalized by contact with a derivatizing composition that contains a mixture of silanes, under reaction conditions effective to couple the silanes to the device surface, typically via reactive hydrophilic moieties present on the device surface. Silanization generally covers a surface through self-assembly with organofunctional alkoxysilane molecules.

A variety of siloxane functionalizing reagents can further be used as currently known in the art, *e.g*., for lowering or increasing surface energy. The organofunctional alkoxysilanes can be classified according to their organic functions.

Described herein but not encompassed by the appended claims are devices that may contain patterning of agents capable of coupling to a nucleoside. In some instances, a device may be coated with an active agent. In some instances, a device may be coated with a passive agent. Exemplary active agents for inclusion in coating materials described herein includes, without limitation, N-(3-triethoxysilylpropyl)-4-hydroxybutyramide (HAPS), 11-acetoxyundecyltriethoxysilane, n-decyltriethoxysilane, (3-aminopropyl)trimethoxysilane, (3-aminopropyl)triethoxysilane, 3-glycidoxypropyltrimethoxysilane (GOPS), 3-iodo-propyltrimethoxysilane, butyl-aldehydr-trimethoxysilane, dimeric secondary aminoalkyl siloxanes, (3-aminopropyl)-diethoxy-methylsilane, (3-aminopropyl)-dimethyl-ethoxysilane, and (3-aminopropyl)-trimethoxysilane, (3-glycidoxypropyl)-dimethyl-ethoxysilane, glycidoxytrimethoxysilane, (3-mercaptopropyl)-trimethoxysilane, 3-4 epoxycyclohexylethyltrimethoxysilane, and (3-mercaptopropyl)-methyl-dimethoxysilane, allyl trichlorochlorosilane, 7-oct-1-enyl trichlorochlorosilane, or bis (3-trimethoxysilylpropyl) amine.

Exemplary passive agents for inclusion in a coating material described herein includes, without limitation, perfluorooctyltrichlorosilane; tridecafluoro-1,1,2,2-tetrahydrooctyl)trichlorosilane; 1H, 1H, 2H, 2H-fluorooctyltriethoxysilane (FOS); trichloro(1H, 1H, 2H, 2H - perfluorooctyl)silane; tert-butyl-[5-fluoro-4-(4,4,5,5-tetramethyl-1,3,2-dioxaborolan-2-yl)indol-1-yl]-dimethyl-silane; CYTOP^{™}; Fluorinert^{™}; perfluoroctyltrichlorosilane (PFOTCS); perfluorooctyldimethylchlorosilane (PFODCS); perfluorodecyltriethoxysilane (PFDTES); pentafluorophenyl-dimethylpropylchloro-silane (PFPTES); perfluorooctyltriethoxysilane; perfluorooctyltrimethoxysilane; octylchlorosilane; dimethylchloro-octodecyl-silane; methyldichloro-octodecyl-silane; trichloro-octodecyl-silane; trimethyl-octodecyl-silane; triethyl-octodecyl-silane; or octadecyltrichlorosilane.

In some instances, a functionalization agent comprises a hydrocarbon silane such as octadecyltrichlorosilane. In some instances, the functionalizing agent comprises 11-acetoxyundecyltriethoxysilane, n-decyltriethoxysilane, (3-aminopropyl)trimethoxysilane, (3-aminopropyl)triethoxysilane, glycidyloxypropyl/trimethoxysilane and N-(3-triethoxy silylpropyl)-4-hydroxybutyramide.

### Polynucleotide Synthesis

Methods of the current disclosure for polynucleotide synthesis may include processes involving phosphoramidite chemistry. In some instances, polynucleotide synthesis comprises coupling a base with phosphoramidite. Polynucleotide synthesis may comprise coupling a base by deposition of phosphoramidite under coupling conditions, wherein the same base is optionally deposited with phosphoramidite more than once, i.e., double coupling. Polynucleotide synthesis may comprise capping of unreacted sites. In some instances, capping is optional. Polynucleotide synthesis may also comprise oxidation or an oxidation step or oxidation steps. Polynucleotide synthesis may comprise deblocking, detritylation, and sulfurization. In some instances, polynucleotide synthesis comprises either oxidation or sulfurization. In some instances, between one or each step during a polynucleotide synthesis reaction, the device is washed, for example, using tetrazole or acetonitrile. Time frames for any one step in a phosphoramidite synthesis method may be less than about 2 min, 1 min, 50 sec, 40 sec, 30 sec, 20 sec and 10 sec.

Polynucleotide synthesis using a phosphoramidite method may comprise a subsequent addition of a phosphoramidite building block (*e.g*., nucleoside phosphoramidite) to a growing polynucleotide chain for the formation of a phosphite triester linkage. Phosphoramidite polynucleotide synthesis proceeds in the 3' to 5' direction. Phosphoramidite polynucleotide synthesis allows for the controlled addition of one nucleotide to a growing nucleic acid chain per synthesis cycle. In some instances, each synthesis cycle comprises a coupling step. Phosphoramidite coupling involves the formation of a phosphite triester linkage between an activated nucleoside phosphoramidite and a nucleoside bound to the substrate, for example, via a linker. In some instances, the nucleoside phosphoramidite is provided to the device activated. In some instances, the nucleoside phosphoramidite is provided to the device with an activator. In some instances, nucleoside phosphoramidites are provided to the device in a 1.5, 2, 3, 4, 5, 6, 7, 8, 9, 10, 11, 12, 13, 14, 15, 16, 17, 18, 19, 20, 25, 30, 35, 40, 50, 60, 70, 80, 90, 100-fold excess or more over the substrate-bound nucleosides. In some instances, the addition of nucleoside phosphoramidite is performed in an anhydrous environment, for example, in anhydrous acetonitrile. Following addition of a nucleoside phosphoramidite, the device is optionally washed. In some instances, the coupling step is repeated one or more additional times, optionally with a wash step between nucleoside phosphoramidite additions to the substrate. In some instances, a polynucleotide synthesis method used herein comprises 1, 2, 3 or more sequential coupling steps. Prior to coupling, in many cases, the nucleoside bound to the device is de-protected by removal of a protecting group, where the protecting group functions to prevent polymerization. A common protecting group is 4,4'-dimethoxytrityl (DMT).

Following coupling, phosphoramidite polynucleotide synthesis methods optionally comprise a capping step. In a capping step, the growing polynucleotide is treated with a capping agent. A capping step is useful to block unreacted substrate-bound 5'-OH groups after coupling from further chain elongation, preventing the formation of polynucleotides with internal base deletions. Further, phosphoramidites activated with 1H-tetrazole may react, to a small extent, with the O6 position of guanosine. Without being bound by theory, upon oxidation with I₂ /water, this side product, possibly via O6-N7 migration, may undergo depurination. The apurinic sites may end up being cleaved in the course of the final deprotection of the polynucleotide thus reducing the yield of the full-length product. The O6 modifications may be removed by treatment with the capping reagent prior to oxidation with I₂/water. In some instances, inclusion of a capping step during polynucleotide synthesis decreases the error rate as compared to synthesis without capping. As an example, the capping step comprises treating the substrate-bound polynucleotide with a mixture of acetic anhydride and 1-methylimidazole. Following a capping step, the device is optionally washed.

In some instances, following addition of a nucleoside phosphoramidite, and optionally after capping and one or more wash steps, the device bound growing nucleic acid is oxidized. The oxidation step comprises the phosphite triester is oxidized into a tetracoordinated phosphate triester, a protected precursor of the naturally occurring phosphate diester internucleoside linkage. In some instances, oxidation of the growing polynucleotide is achieved by treatment with iodine and water, optionally in the presence of a weak base (*e.g*., pyridine, lutidine, collidine). Oxidation may be carried out under anhydrous conditions using, *e.g*. tert-Butyl hydroperoxide or (1S)-(+)-(10-camphorsulfonyl)-oxaziridine (CSO). In some methods, a capping step is performed following oxidation. A second capping step allows for device drying, as residual water from oxidation that may persist can inhibit subsequent coupling. Following oxidation, the device and growing polynucleotide is optionally washed. In some instances, the step of oxidation is substituted with a sulfurization step to obtain polynucleotide phosphorothioates, wherein any capping steps can be performed after the sulfurization. Many reagents are capable of the efficient sulfur transfer, including but not limited to 3-(Dimethylaminomethylidene)amino)-3H-1,2,4-dithiazole-3-thione, DDTT, 3H-1,2-benzodithiol-3-one 1,1-dioxide, also known as Beaucage reagent, and N,N,N'N'-Tetraethylthiuram disulfide (TETD).

In order for a subsequent cycle of nucleoside incorporation to occur through coupling, the protected 5' end of the device bound growing polynucleotide is removed so that the primary hydroxyl group is reactive with a next nucleoside phosphoramidite. In some instances, the protecting group is DMT and deblocking occurs with trichloroacetic acid in dichloromethane. Conducting detritylation for an extended time or with stronger than recommended solutions of acids may lead to increased depurination of solid support-bound polynucleotide and thus reduces the yield of the desired full-length product. Methods and compositions described herein provide for controlled deblocking conditions limiting undesired depurination reactions. In some instances, the device bound polynucleotide is washed after deblocking. In some instances, efficient washing after deblocking contributes to synthesized polynucleotides having a low error rate.

Methods for the synthesis of polynucleotides typically involve an iterating sequence of the following steps: application of a protected monomer to an actively functionalized surface (*e.g.*, locus) to link with either the activated surface, a linker or with a previously deprotected monomer; deprotection of the applied monomer so that it is reactive with a subsequently applied protected monomer; and application of another protected monomer for linking. One or more intermediate steps include oxidation or sulfurization. In some instances, one or more wash steps precede or follow one or all of the steps.

Methods for phosphoramidite-based polynucleotide synthesis comprise a series of chemical steps. In some instances, one or more steps of a synthesis method involve reagent cycling, where one or more steps of the method comprise application to the device of a reagent useful for the step. For example, reagents are cycled by a series of liquid deposition and vacuum drying steps. For substrates comprising three-dimensional features such as wells, microwells, channels and the like, reagents are optionally passed through one or more regions of the device via the wells and/or channels.

Methods and systems described herein but not encompassed by the appended claims relate to polynucleotide synthesis devices for the synthesis of polynucleotides. The synthesis may be in parallel. For example at least or about at least 2, 3, 4, 5, 6, 7, 8, 9, 10, 11, 12, 13, 14, 15, 16, 17, 18, 19, 20, 21, 22, 23, 24, 25, 30, 35, 40, 45, 50, 100, 150, 200, 250, 300, 350, 400, 450, 500, 550, 600, 650, 700, 750, 800, 850, 900, 1000, 10000, 50000, 75000, 100000 or more polynucleotides can be synthesized in parallel. The total number polynucleotides that may be synthesized in parallel may be from 2-100000, 3-50000, 4-10000, 5-1000, 6-900, 7-850, 8-800, 9-750, 10-700, 11-650, 12-600, 13-550, 14-500, 15-450, 16-400, 17-350, 18-300, 19-250, 20-200, 21-150,22-100, 23-50, 24-45, 25-40, 30-35. Those of skill in the art appreciate that the total number of polynucleotides synthesized in parallel may fall within any range bound by any of these values, for example 25-100. The total number of polynucleotides synthesized in parallel may fall within any range defined by any of the values serving as endpoints of the range. Total molar mass of polynucleotides synthesized within the device or the molar mass of each of the polynucleotides may be at least or at least about 10, 20, 30, 40, 50, 100, 250, 500, 750, 1000, 2000, 3000, 4000, 5000, 6000, 7000, 8000, 9000, 10000, 25000, 50000, 75000, 100000 picomoles, or more. The length of each of the polynucleotides or average length of the polynucleotides within the device may be at least or about at least 10, 15, 20, 25, 30, 35, 40, 45, 50, 100, 150, 200, 300, 400, 500 nucleotides, or more. The length of each of the polynucleotides or average length of the polynucleotides within the device may be at most or about at most 500, 400, 300, 200, 150, 100, 50, 45, 35, 30, 25, 20, 19, 18, 17, 16, 15, 14, 13, 12, 11, 10 nucleotides, or less. The length of each of the polynucleotides or average length of the polynucleotides within the device may fall from 10-500, 9-400, 11-300, 12-200, 13-150, 14-100, 15-50, 16-45, 17-40, 18-35, 19-25. Those of skill in the art appreciate that the length of each of the polynucleotides or average length of the polynucleotides within the device may fall within any range bound by any of these values, for example 100-300. The length of each of the polynucleotides or average length of the polynucleotides within the device may fall within any range defined by any of the values serving as endpoints of the range.

Methods for polynucleotide synthesis on a surface disclosed herein allow for synthesis at a fast rate. As an example, at least 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 70, 80, 90, 100, 125, 150, 175, 200 nucleotides per hour, or more are synthesized. Nucleotides include adenine, guanine, thymine, cytosine, uridine building blocks, or analogs/modified versions thereof. In some instances, libraries of polynucleotides are synthesized in parallel on substrate. For example, a device comprising about or at least about 100; 1,000; 10,000; 30,000; 75,000; 100,000; 1,000,000; 2,000,000; 3,000,000; 4,000,000; or 5,000,000 resolved loci is able to support the synthesis of at least the same number of distinct polynucleotides, wherein polynucleotide encoding a distinct sequence is synthesized on a resolved locus. In some instances, a library of polynucleotides are synthesized on a device with low error rates described herein in less than about three months, two months, one month, three weeks, 15, 14, 13, 12, 11, 10, 9, 8, 7, 6, 5, 4, 3, 2 days, 24 hours or less. In some instances, larger nucleic acids assembled from a polynucleotide library synthesized with low error rate using the substrates and methods described herein are prepared in less than about three months, two months, one month, three weeks, 15, 14, 13, 12, 11, 10, 9, 8, 7, 6, 5, 4, 3, 2 days, 24 hours or less.

In some instances, methods described herein but not encompassed by the appended claims provide for generation of a library of polynucleotides comprising variant polynucleotides differing at a plurality of codon sites. In some instances, a polynucleotide may have 1 site, 2 sites, 3 sites, 4 sites, 5 sites, 6 sites, 7 sites, 8 sites, 9 sites, 10 sites, 11 sites, 12 sites, 13 sites, 14 sites, 15 sites, 16 sites, 17 sites 18 sites, 19 sites, 20 sites, 30 sites, 40 sites, 50 sites, or more of variant codon sites.

In some instances, the one or more sites of variant codon sites may be adjacent. In some instances, the one or more sites of variant codon sites may be not be adjacent and separated by 1, 2, 3, 4, 5, 6, 7, 8, 9, 10, or more codons.

In some instances, a polynucleotide may comprise multiple sites of variant codon sites, wherein all the variant codon sites are adjacent to one another, forming a stretch of variant codon sites. In some instances, a polynucleotide may comprise multiple sites of variant codon sites, wherein none the variant codon sites are adjacent to one another. In some instances, a polynucleotide may comprise multiple sites of variant codon sites, wherein some the variant codon sites are adjacent to one another, forming a stretch of variant codon sites, and some of the variant codon sites are not adjacent to one another.

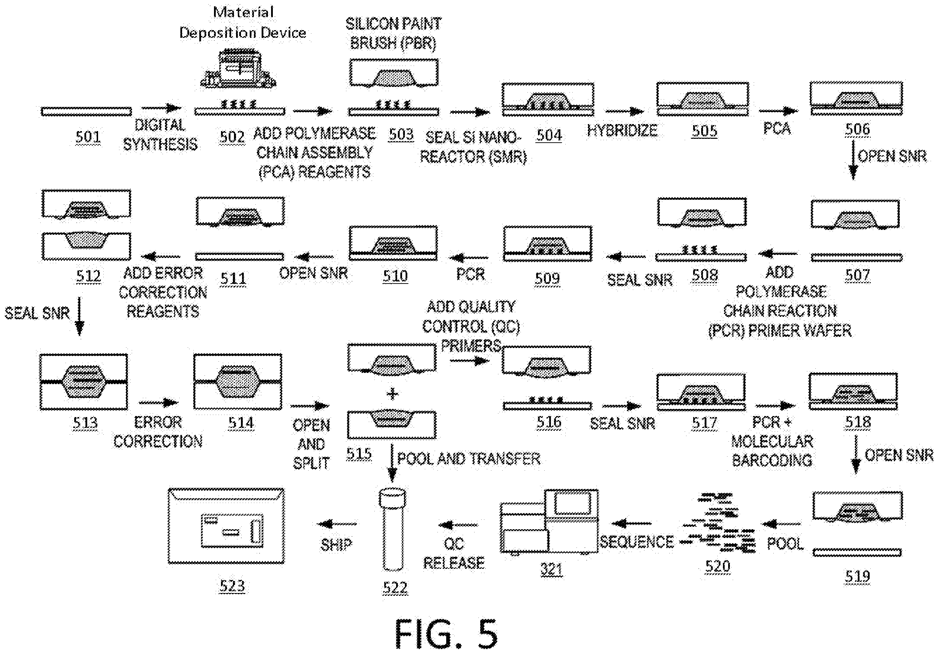

Referring to the Figures, **FIG. 5** illustrates an exemplary process workflow for synthesis of nucleic acids (e.g., genes) from shorter polynucleotides. The workflow is divided generally into phases: (1) de novo synthesis of a single stranded polynucleotide library, (2) joining polynucleotides to form larger fragments, (3) error correction, (4) quality control, and (5) shipment. Prior to de novo synthesis, an intended nucleic acid sequence or group of nucleic acid sequences is preselected. For example, a group of genes is preselected for generation.

Once large polynucleotides for generation are selected, a predetermined library of polynucleotides is designed for de novo synthesis. Various suitable methods are known for generating high density polynucleotide arrays. In the workflow example, a device surface layer **501** is provided. In the example, chemistry of the surface is altered in order to improve the polynucleotide synthesis process. Areas of low surface energy are generated to repel liquid while areas of high surface energy are generated to attract liquids. The surface itself may be in the form of a planar surface or contain variations in shape, such as protrusions or microwells which increase surface area. In the workflow example, high surface energy molecules selected serve a dual function of supporting DNA chemistry, as disclosed in International Patent Application Publication WO2015/021080.

In situ preparation of polynucleotide arrays is generated on a solid support and utilizes single nucleotide extension process to extend multiple oligomers in parallel. A material deposition device, such as a polynucleotide synthesizer, is designed to release reagents in a step wise fashion such that multiple polynucleotides extend, in parallel, one residue at a time to generate oligomers with a predetermined nucleic acid sequence **502.** In some instances, polynucleotides are cleaved from the surface at this stage. Cleavage includes gas cleavage, *e.g*., with ammonia or methylamine.

The generated polynucleotide libraries are placed in a reaction chamber. In this exemplary workflow, the reaction chamber (also referred to as "nanoreactor") is a silicon coated well, containing PCR reagents and lowered onto the polynucleotide library **503.** Prior to or after the sealing **504** of the polynucleotides, a reagent is added to release the polynucleotides from the substrate. In the exemplary workflow, the polynucleotides are released subsequent to sealing of the nanoreactor **505.** Once released, fragments of single stranded polynucleotides hybridize in order to span an entire long range sequence of DNA. Partial hybridization **505** is possible because each synthesized polynucleotide is designed to have a small portion overlapping with at least one other polynucleotide in the population.

After hybridization, a PCR reaction is commenced. During the polymerase cycles, the polynucleotides anneal to complementary fragments and gaps are filled in by a polymerase. Each cycle increases the length of various fragments randomly depending on which polynucleotides find each other. Complementarity amongst the fragments allows for forming a complete large span of double stranded DNA **506.**

After PCR is complete, the nanoreactor is separated from the device **507** and positioned for interaction with a device having primers for PCR **508.** After sealing, the nanoreactor is subject to PCR **309** and the larger nucleic acids are amplified. After PCR **510,** the nanochamber is opened **511,** error correction reagents are added **512,** the chamber is sealed **513** and an error correction reaction occurs to remove mismatched base pairs and/or strands with poor complementarity from the double stranded PCR amplification products **514.** The nanoreactor is opened and separated **515.** Error corrected product is next subject to additional processing steps, such as PCR and molecular bar coding, and then packaged **522** for shipment **523.**

In some instances, quality control measures are taken. After error correction, quality control steps include for example interaction with a wafer having sequencing primers for amplification of the error corrected product **516,** sealing the wafer to a chamber containing error corrected amplification product **517,** and performing an additional round of amplification **518.** The nanoreactor is opened **519** and the products are pooled **520** and sequenced **521.** After an acceptable quality control determination is made, the packaged product **522** is approved for shipment **523.**

In some instances, a nucleic acid generate by a workflow such as that in **FIG. 5** is subject to mutagenesis using overlapping primers disclosed herein. In some instances, a library of primers are generated by in situ preparation on a solid support and utilize single nucleotide extension process to extend multiple oligomers in parallel. A deposition device, such as a polynucleotide synthesizer, is designed to release reagents in a step wise fashion such that multiple polynucleotides extend, in parallel, one residue at a time to generate oligomers with a predetermined nucleic acid sequence **502.**

### Large Polynucleotide Libraries Having Low Error Rates

Average error rates for polynucleotides synthesized within a library using the systems and methods described herein may be less than 1 in 1000, less than 1 in 1250, less than 1 in 1500, less than 1 in 2000, less than 1 in 3000 or less often. In some instances, average error rates for polynucleotides synthesized within a library using the systems and methods provided are less than 1/500, 1/600, 1/700, 1/800, 1/900, 1/1000, 1/1100, 1/1200, 1/1250, 1/1300, 1/1400, 1/1500, 1/1600, 1/1700, 1/1800, 1/1900, 1/2000, 1/3000, or less. In some instances, average error rates for polynucleotides synthesized within a library using the systems and methods provided are less than 1/1000.

In some instances, aggregate error rates for polynucleotides synthesized within a library using the systems and methods provided are less than 1/500, 1/600, 1/700, 1/800, 1/900, 1/1000, 1/1100, 1/1200, 1/1250, 1/1300, 1/1400, 1/1500, 1/1600, 1/1700, 1/1800, 1/1900, 1/2000, 1/3000, or less compared to the predetermined sequences. In some instances, aggregate error rates for polynucleotides synthesized within a library using the systems and methods provided are less than 1/500, 1/600, 1/700, 1/800, 1/900, or 1/1000. In some instances, aggregate error rates for polynucleotides synthesized within a library using the systems and methods provided are less than 1/1000.

In some instances, an error correction enzyme may be used for polynucleotides synthesized within a library using the systems and methods provided can use. In some instances, aggregate error rates for polynucleotides with error correction can be less than 1/500, 1/600, 1/700, 1/800, 1/900, 1/1000, 1/1100, 1/1200, 1/1300, 1/1400, 1/1500, 1/1600, 1/1700, 1/1800, 1/1900, 1/2000, 1/3000, or less compared to the predetermined sequences. In some instances, aggregate error rates with error correction for polynucleotides synthesized within a library using the systems and methods provided can be less than 1/500, 1/600, 1/700, 1/800, 1/900, or 1/1000. In some instances, aggregate error rates with error correction for polynucleotides synthesized within a library using the systems and methods provided can be less than 1/1000.

Error rate may limit the value of gene synthesis for the production of libraries of gene variants. With an error rate of 1/300, about 0.7% of the clones in a 1500 base pair gene will be correct. As most of the errors from polynucleotide synthesis result in frame-shift mutations, over 99% of the clones in such a library will not produce a full-length protein. Reducing the error rate by 75% would increase the fraction of clones that are correct by a factor of 40. The methods and compositions of the disclosure allow for fast de novo synthesis of large polynucleotide and gene libraries with error rates that are lower than commonly observed gene synthesis methods both due to the improved quality of synthesis and the applicability of error correction methods that are enabled in a massively parallel and time-efficient manner. Accordingly, libraries may be synthesized with base insertion, deletion, substitution, or total error rates that are under 1/300, 1/400, 1/500, 1/600, 1/700, 1/800, 1/900, 1/1000, 1/1250, 1/1500, 1/2000, 1/2500, 1/3000, 1/4000, 1/5000, 1/6000, 1/7000, 1/8000, 1/9000, 1/10000, 1/12000, 1/15000, 1/20000, 1/25000, 1/30000, 1/40000, 1/50000, 1/60000, 1/70000, 1/80000, 1/90000, 1/100000, 1/125000, 1/150000, 1/200000, 1/300000, 1/400000, 1/500000, 1/600000, 1/700000, 1/800000, 1/900000, 1/1000000, or less, across the library, or across more than 80%, 85%, 90%, 93%, 95%, 96%, 97%, 98%, 99%, 99.5%, 99.8%, 99.9%, 99.95%, 99.98%, 99.99%, or more of the library. The methods and compositions of the disclosure further relate to large synthetic polynucleotide and gene libraries with low error rates associated with at least 30%, 40%, 50%, 60%, 70%, 75%, 80%, 85%, 90%, 93%, 95%, 96%, 97%, 98%, 99%, 99.5%, 99.8%, 99.9%, 99.95%, 99.98%, 99.99%, or more of the polynucleotides or genes in at least a subset of the library to relate to error free sequences in comparison to a predetermined/preselected sequence. In some instances, at least 30%, 40%, 50%, 60%, 70%, 75%, 80%, 85%, 90%, 93%, 95%, 96%, 97%, 98%, 99%, 99.5%, 99.8%, 99.9%, 99.95%, 99.98%, 99.99%, or more of the polynucleotides or genes in an isolated volume within the library have the same sequence. In some instances, at least 30%, 40%, 50%, 60%, 70%, 75%, 80%, 85%, 90%, 93%, 95%, 96%, 97%, 98%, 99%, 99.5%, 99.8%, 99.9%, 99.95%, 99.98%, 99.99%, or more of any polynucleotides or genes related with more than 95%, 96%, 97%, 98%, 99%, 99.5%, 99.6%, 99.7%, 99.8%, 99.9% or more similarity or identity have the same sequence. In some instances, the error rate related to a specified locus on a polynucleotide or gene is optimized. Thus, a given locus or a plurality of selected loci of one or more polynucleotides or genes as part of a large library may each have an error rate that is less than 1/300, 1/400, 1/500, 1/600, 1/700, 1/800, 1/900, 1/1000, 1/1250, 1/1500, 1/2000, 1/2500, 1/3000, 1/4000, 1/5000, 1/6000, 1/7000, 1/8000, 1/9000, 1/10000, 1/12000, 1/15000, 1/20000, 1/25000, 1/30000, 1/40000, 1/50000, 1/60000, 1/70000, 1/80000, 1/90000, 1/100000, 1/125000, 1/150000, 1/200000, 1/300000, 1/400000, 1/500000, 1/600000, 1/700000, 1/800000, 1/900000, 1/1000000, or less. In various instances, such error optimized loci may comprise at least 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 200, 300, 400, 500, 600, 700, 800, 900, 1000, 1500, 2000, 2500, 3000, 4000, 5000, 6000, 7000, 8000, 9000, 10000, 30000, 50000, 75000, 100000, 500000, 1000000, 2000000, 3000000 or more loci. The error optimized loci may be distributed to at least 1, 2, 3, 4, 5, 6, 7, 8, 9, 10, 11, 12, 13, 14, 15, 16, 17, 18, 19, 20, 25, 30, 35, 40, 45, 50, 60, 70, 80, 90, 100, 200, 300, 400, 500, 600, 700, 800, 900, 1000, 1500, 2000, 2500, 3000, 4000, 5000, 6000, 7000, 8000, 9000, 10000, 30000, 75000, 100000, 500000, 1000000, 2000000, 3000000 or more polynucleotides or genes.

The error rates can be achieved with or without error correction. The error rates can be achieved across the library, or across more than 80%, 85%, 90%, 93%, 95%, 96%, 97%, 98%, 99%, 99.5%, 99.8%, 99.9%, 99.95%, 99.98%, 99.99%, or more of the library.

### Computer systems

Any of the systems described herein but not encompassed by the appended claims may be operably linked to a computer and may be automated through a computer either locally or remotely. In various instances, the methods and systems of the disclosure may further comprise software programs on computer systems and use thereof. Accordingly, computerized control for the synchronization of the dispense/vacuum/refill functions such as orchestrating and synchronizing the material deposition device movement, dispense action and vacuum actuation are within the bounds of the disclosure. The computer systems may be programmed to interface between the user specified base sequence and the position of a material deposition device to deliver the correct reagents to specified regions of the substrate.

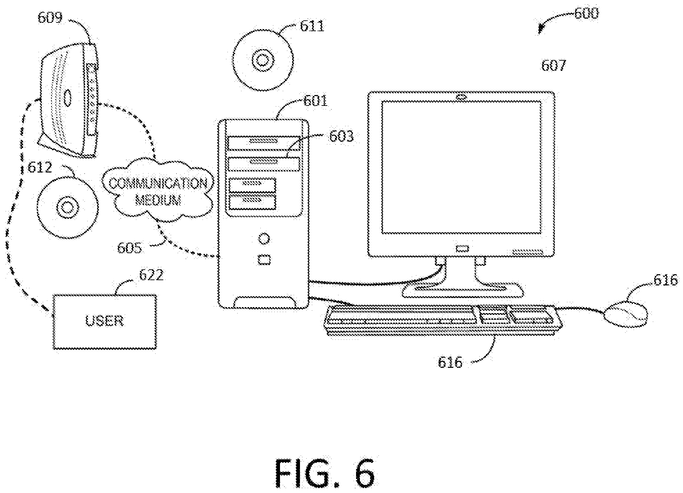

The computer system **600** illustrated in **FIG. 6** may be understood as a logical apparatus that can read instructions from media **611** and/or a network port **605,** which can optionally be connected to server **609** having fixed media **612.** The system, such as shown in **FIG. 6** can include a CPU **601,** disk drives **603,** optional input devices such as keyboard **615** and/or mouse **616** and optional monitor **607.** Data communication can be achieved through the indicated communication medium to a server at a local or a remote location. The communication medium can include any means of transmitting and/or receiving data. For example, the communication medium can be a network connection, a wireless connection or an internet connection. Such a connection can provide for communication over the World Wide Web. It is envisioned that data relating to the present disclosure can be transmitted over such networks or connections for reception and/or review by a party **622** as illustrated in **FIG. 6****.**

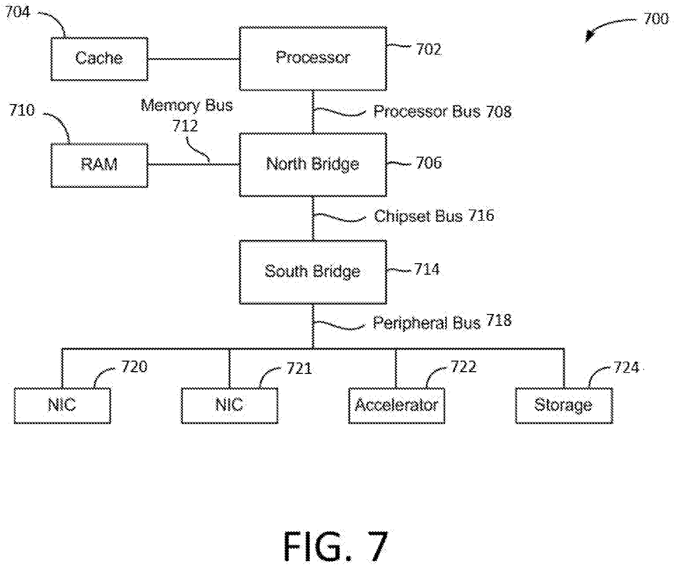

**FIG. 7** is a block diagram illustrating a first example architecture of a computer system **700** that can be used in connection with example instances of the present disclosure. As depicted in **FIG. 7****,** the example computer system can include a processor **702** for processing instructions. Non-limiting examples of processors include: Intel XeonTM processor, AMD OpteronTM processor, Samsung 32-bit RISC ARM 1176JZ(F)-S v1.0TM processor, ARM Cortex-A8 Samsung S5PC100TM processor, ARM Cortex-A8 Apple A4TM processor, Marvell PXA 930TM processor, or a functionally-equivalent processor. Multiple threads of execution can be used for parallel processing. In some instances, multiple processors or processors with multiple cores can also be used, whether in a single computer system, in a cluster, or distributed across systems over a network comprising a plurality of computers, cell phones, and/or personal data assistant devices.

As illustrated in **FIG. 7****,** a high speed cache **704** can be connected to, or incorporated in, the processor **702** to provide a high speed memory for instructions or data that have been recently, or are frequently, used by processor **702.** The processor **702** is connected to a north bridge **706** by a processor bus **708.** The north bridge **706** is connected to random access memory (RAM) **710** by a memory bus **712** and manages access to the RAM **710** by the processor **702.** The north bridge **706** is also connected to a south bridge **714** by a chipset bus **716.** The south bridge **714** is, in turn, connected to a peripheral bus **718.** The peripheral bus can be, for example, PCI, PCI-X, PCI Express, or other peripheral bus. The north bridge and south bridge are often referred to as a processor chipset and manage data transfer between the processor, RAM, and peripheral components on the peripheral bus **718.** In some alternative architectures, the functionality of the north bridge can be incorporated into the processor instead of using a separate north bridge chip. In some instances, system **700** can include an accelerator card **722** attached to the peripheral bus **718.** The accelerator can include field programmable gate arrays (FPGAs) or other hardware for accelerating certain processing. For example, an accelerator can be used for adaptive data restructuring or to evaluate algebraic expressions used in extended set processing.

Software and data are stored in external storage **724** and can be loaded into RAM **710** and/or cache **704** for use by the processor. The system **700** includes an operating system for managing system resources; non-limiting examples of operating systems include: Linux, WindowsTM, MACOSTM, BlackBerry OSTM, iOSTM, and other functionally-equivalent operating systems, as well as application software running on top of the operating system for managing data storage and optimization in accordance with example instances of the present disclosure. In this example, system **700** also includes network interface cards (NICs) **720** and **721** connected to the peripheral bus for providing network interfaces to external storage, such as Network Attached Storage (NAS) and other computer systems that can be used for distributed parallel processing.

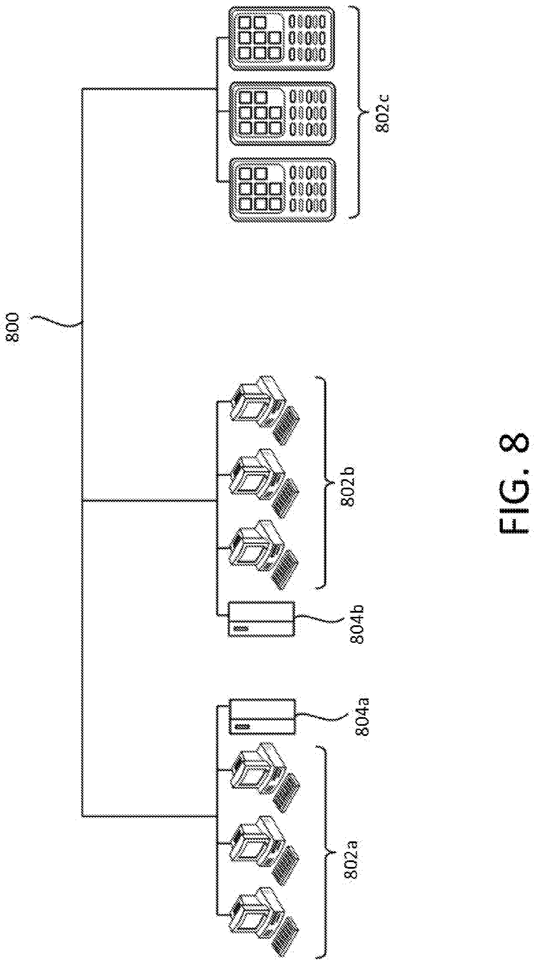

**FIG.8** is a diagram showing a network **800** with a plurality of computer systems **802a,** and **802b,** a plurality of cell phones and personal data assistants **802c,** and Network Attached Storage (NAS) **804a,** and **804b.** In example instances, systems **802a, 802b,** and **802c** can manage data storage and optimize data access for data stored in Network Attached Storage (NAS) **804a** and **804b.** A mathematical model can be used for the data and be evaluated using distributed parallel processing across computer systems **802a,** and **802b,** and cell phone and personal data assistant systems **802c.** Computer systems **802a,** and **802b,** and cell phone and personal data assistant systems **802c** can also provide parallel processing for adaptive data restructuring of the data stored in Network Attached Storage (NAS) **804a** and **804b.** **FIG. 8** illustrates an example only, and a wide variety of other computer architectures and systems can be used in conjunction with the various instances of the present disclosure. For example, a blade server can be used to provide parallel processing. Processor blades can be connected through a back plane to provide parallel processing. Storage can also be connected to the back plane or as Network Attached Storage (NAS) through a separate network interface. In some example instances, processors can maintain separate memory spaces and transmit data through network interfaces, back plane or other connectors for parallel processing by other processors. In other instances, some or all of the processors can use a shared virtual address memory space.

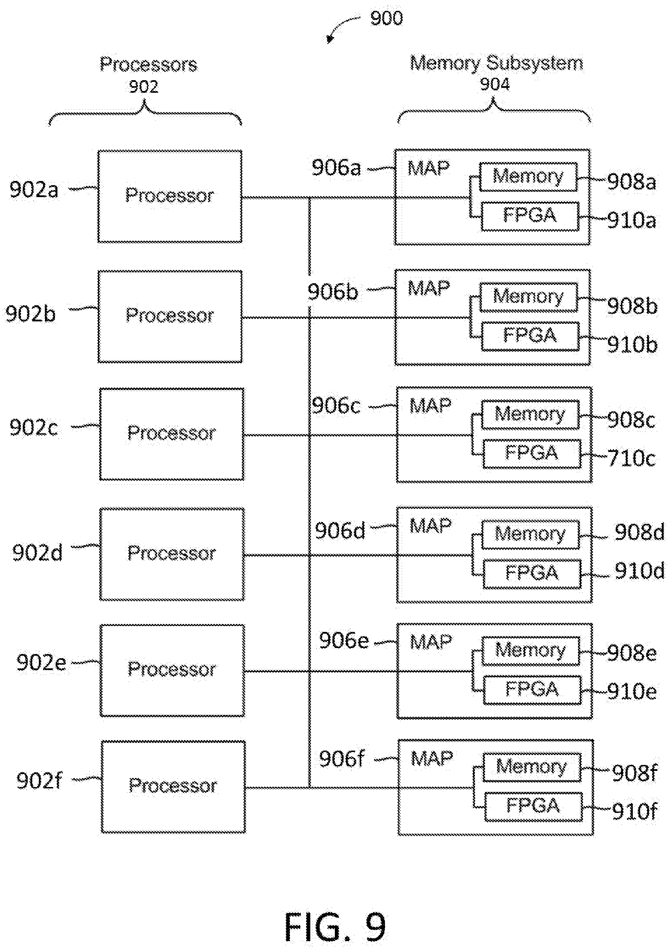

**FIG. 9** is a block diagram of a multiprocessor computer system **900** using a shared virtual address memory space in accordance with an example instance. The system includes a plurality of processors **902a-f** that can access a shared memory subsystem **904.** The system incorporates a plurality of programmable hardware memory algorithm processors (MAPs) **906a-f** in the memory subsystem **904.** Each MAP **906a-f** can comprise a memory **908a-f** and one or more field programmable gate arrays (FPGAs) **910a-f.** The MAP provides a configurable functional unit and particular algorithms or portions of algorithms can be provided to the FPGAs **910a-f** for processing in close coordination with a respective processor. For example, the MAPs can be used to evaluate algebraic expressions regarding the data model and to perform adaptive data restructuring in example instances. In this example, each MAP is globally accessible by all of the processors for these purposes. In one configuration, each MAP can use Direct Memory Access (DMA) to access an associated memory **908a-f,** allowing it to execute tasks independently of, and asynchronously from the respective microprocessor **902a-f.** In this configuration, a MAP can feed results directly to another MAP for pipelining and parallel execution of algorithms.

The above computer architectures and systems are examples only, and a wide variety of other computer, cell phone, and personal data assistant architectures and systems can be used in connection with example instances, including systems using any combination of general processors, co-processors, FPGAs and other programmable logic devices, system on chips (SOCs), application specific integrated circuits (ASICs), and other processing and logic elements. In some instances, all or part of the computer system can be implemented in software or hardware. Any variety of data storage media can be used in connection with example instances, including random access memory, hard drives, flash memory, tape drives, disk arrays, Network Attached Storage (NAS) and other local or distributed data storage devices and systems.

In example instances, the computer system can be implemented using software modules executing on any of the above or other computer architectures and systems. In other instances, the functions of the system can be implemented partially or completely in firmware, programmable logic devices such as field programmable gate arrays (FPGAs) as referenced in **FIG. 9****,** system on chips (SOCs), application specific integrated circuits (ASICs), or other processing and logic elements. For example, the Set Processor and Optimizer can be implemented with hardware acceleration through the use of a hardware accelerator card, such as accelerator card **722** illustrated in **FIG. 7****.**

### Additional Methods and Compositions

Provided herein are nucleic acid libraries comprising at least 5000 non-identical polynucleotides, wherein the at least 5000 non-identical polynucleotides are amplification products of synthesized polynucleotides, and wherein greater than about 80% of the at least 5000 non-identical polynucleotides are represented in an amount within at least about 2 times the mean representation for the nucleic acid libraries. Further provided herein are nucleic acid libraries wherein greater than about 80% of the at least 5000 non-identical polynucleotides are represented in an amount within at least about 1.5 times the mean representation for the nucleic acid libraries. Further provided herein are nucleic acid libraries wherein greater than about 90% of the at least 5000 non-identical polynucleotides are represented in an amount within at least about 2 times the mean representation for the nucleic acid libraries. Further provided herein are nucleic acid libraries wherein greater than about 90% of the at least 5000 non-identical polynucleotides are represented in an amount within at least about 1.5 times the mean representation for the nucleic acid libraries. Further provided herein are nucleic acid libraries wherein the nucleic acid libraries comprises less dropouts compared to an amplification product from a method using a structure having a surface of unclustered loci. Further provided herein are nucleic acid libraries wherein the at least about 5000 non-identical polynucleotides comprise polynucleotides having a GC percentage of at least about 10%. Further provided herein are nucleic acid libraries wherein the at least about 5000 non-identical polynucleotides comprise polynucleotides having a GC percentage of at most 95%.Further provided herein are nucleic acid libraries wherein the at least about 5000 non-identical polynucleotides comprise polynucleotides having a GC percentage of about 10% to about 95%. Further provided herein are nucleic acid libraries wherein greater than 30% at least about 5000 non-identical polynucleotides comprise polynucleotides having a GC percentage from 10% to 30% or 70 to 90%. Further provided herein are nucleic acid libraries wherein less than about 15% at least about 5000 non-identical polynucleotides comprise polynucleotides having a GC percentage from 10% to 30% or 60 to 90%. Further provided herein are nucleic acid libraries wherein the at least about 5000 non-identical polynucleotides comprise polynucleotides having a repeating sequence percentage of at least about 10%. Further provided herein are nucleic acid libraries wherein the at least about 5000 non-identical polynucleotides comprise polynucleotides having a repeating sequence percentage of at most 95%. Further provided herein are nucleic acid libraries wherein the at least about 5000 non-identical polynucleotides comprise polynucleotides having a repeating sequence percentage of about 10% to about 95%. Further provided herein are nucleic acid libraries wherein greater than 30% at least about 5000 non-identical polynucleotides comprise polynucleotides having a repeating sequence percentage from 10% to 30% or 70 to 90%. Further provided herein are nucleic acid libraries wherein less than about 15% at least about 5000 non-identical polynucleotides comprise polynucleotides having a repeating sequence percentage from 10% to 30% or 60 to 90%. Further provided herein are nucleic acid libraries wherein the at least about 5000 non-identical polynucleotides comprise polynucleotides having a secondary structure percentage of at least about 10%. Further provided herein are nucleic acid libraries wherein the at least about 5000 non-identical polynucleotides comprise polynucleotides having a secondary structure percentage of at most 95%. Further provided herein are nucleic acid libraries wherein the at least about 5000 non-identical polynucleotides comprise polynucleotides having a secondary structure percentage of about 10% to about 95%. Further provided herein are nucleic acid libraries wherein greater than 30% at least about 5000 non-identical polynucleotides comprise polynucleotides having a secondary structure percentage from 10% to 30% or 70 to 90%. Further provided herein are nucleic acid libraries wherein in less than about 15% at least about 5000 non-identical polynucleotides comprise polynucleotides having a secondary structure percentage from 10% to 30% or 60 to 90%. Further provided herein are nucleic acid libraries wherein the polynucleotide library encodes for a variant library. Further provided herein are nucleic acid libraries wherein the at least 5000 non-identical polynucleotides encode for at least one gene. Further provided herein are nucleic acid libraries wherein the at least 5000 non-identical polynucleotides encode for at least 50 genes. Further provided herein are nucleic acid libraries wherein the polynucleotide library encodes for at least a portion of an antibody, enzyme, or peptide. Further provided herein are nucleic acid libraries wherein the predetermined sequences encode for at least 700,000 non-identical polynucleotides.

Provided herein are nucleic acid libraries comprising at least 5000 non-identical polynucleotides, wherein a GC content is controlled, and wherein the libraries provide for a correct sequence rate of greater than 75% following an amplification reaction. Further provided herein are nucleic acid libraries having a correct sequence rate of greater than 85% following an amplification reaction. Further provided herein are nucleic acid libraries wherein the at least about 5000 non-identical polynucleotides comprise polynucleotides having a GC percentage of at least about 10%. Further provided herein are nucleic acid libraries wherein the at least about 5000 non-identical polynucleotides comprise polynucleotides having a GC percentage of at most 95%. Further provided herein are nucleic acid libraries wherein the at least about 5000 non-identical polynucleotides comprise polynucleotides having a GC percentage of about 10% to about 95%. Further provided herein are nucleic acid libraries wherein greater than 30% at least about 5000 non-identical polynucleotides comprise polynucleotides having a GC percentage from 10% to 30% or 70 to 90%. Further provided herein are nucleic acid libraries wherein less than about 15% at least about 5000 non-identical polynucleotides comprise polynucleotides having a GC percentage from 10% to 30% or 60 to 90%.

Provided herein are nucleic acid libraries comprising at least 5000 non-identical polynucleotides, wherein a repeating sequence content is controlled, and wherein the libraries provides for a correct sequence rate of greater than 75% following an amplification reaction. Further provided herein are nucleic acid libraries wherein the polynucleotide library has a correct sequence rate of greater than 85% following an amplification reaction. Further provided herein are nucleic acid libraries wherein the at least about 5000 non-identical polynucleotides comprise polynucleotides having a repeating sequence percentage of at least about 10%. Further provided herein are nucleic acid libraries wherein the at least about 5000 non-identical polynucleotides comprise polynucleotides having a repeating sequence percentage of at most 95%. Further provided herein are nucleic acid libraries wherein the at least about 5000 non-identical polynucleotides comprise polynucleotides having a repeating sequence percentage of about 10% to about 95%. Further provided herein are nucleic acid libraries wherein greater than 30% at least about 5000 non-identical polynucleotides comprise polynucleotides having a repeating sequence percentage from 10% to 30% or 70 to 90%. Further provided herein are nucleic acid libraries wherein less than about 15% at least about 5000 non-identical polynucleotides comprise polynucleotides having a repeating sequence percentage from 10% to 30% or 60 to 90%.

Provided herein are nucleic acid libraries comprising at least 5000 non-identical polynucleotides, wherein a secondary structure content encoded by the at least 5000 non-identical polynucleotides is preselected, and wherein the libraries provide for a correct sequence rate of greater than 75% following an amplification reaction. Further provided herein are nucleic acid libraries wherein the nucleic acid libraries have a correct sequence rate of greater than 85% following an amplification reaction. Further provided herein are nucleic acid libraries wherein the at least about 5000 non-identical polynucleotides comprise polynucleotides having a secondary structure percentage of at least about 10%. Further provided herein are nucleic acid libraries wherein the at least about 5000 non-identical polynucleotides comprise polynucleotides having a secondary structure percentage of at most 95%. Further provided herein are nucleic acid libraries wherein the at least about 5000 non-identical polynucleotides comprise polynucleotides having a secondary structure percentage of about 10% to about 95%. Further provided herein are nucleic acid libraries wherein greater than 30% at least about 5000 non-identical polynucleotides comprise polynucleotides having a secondary structure percentage from 10% to 30% or 70 to 90%. Further provided herein are nucleic acid libraries, wherein less than about 15% at least about 5000 non-identical polynucleotides comprise polynucleotides having a repeating sequence percentage from 10% to 30% or 60 to 90%. Further provided herein are nucleic acid libraries encoding for a variant library. Further provided herein are nucleic acid libraries encoding for at least one gene. Further provided herein are nucleic acid libraries encoding for at least a portion of an antibody, enzyme, or peptide. Further provided herein are nucleic acid libraries having an aggregate error rate of less than 1 in 500 bases compared to the predetermined sequences without correcting errors. Further provided herein are nucleic acid libraries having an aggregate error rate of less than 1 in 1000 bases compared to the predetermined sequences without correcting errors. Further provided herein are nucleic acid libraries wherein the predetermined sequences encode for at least 700,000 non-identical polynucleotides. Further provided herein are nucleic acid libraries wherein the at least 5000 non-identical polynucleotides encode for at least one gene. Further provided herein are nucleic acid libraries wherein the at least 5000 non-identical polynucleotides encode for at least 50 genes.

Provided herein are nucleic acid libraries comprising at least 100,000 non-identical polynucleotides, wherein each non-identical polynucleotide encodes for at least one different exome sequence, and wherein at least about 80% of the at least 100,000 non-identical polynucleotides are each present in the polynucleotide library in an amount within 2x of a mean frequency for each of the non-identical polynucleotides in the library. Further provided herein are nucleic acid libraries wherein the nucleic acid libraries are amplicon libraries, and wherein at least about 80% of the plurality of non-identical polynucleotides are each present in the amplicon libraries in an amount within 2x of a mean frequency for each of the non-identical polynucleotides in the libraries. Further provided herein are nucleic acid libraries wherein sequencing the libraries at up to 55 fold theoretical read depth results in at least 90% of the bases having at least 30 fold read depth. Further provided herein are nucleic acid libraries wherein sequencing the libraries at up to 55 fold theoretical read depth results in at least 98% of the bases having at least 10 fold read depth.

### EXAMPLES

### Example 1: Functionalization of a substrate surface

A substrate was functionalized to support the attachment and synthesis of a library of polynucleotides. The substrate surface was first wet cleaned using a piranha solution comprising 90% H₂SO₄ and 10% H₂O₂ for 20 minutes. The substrate was rinsed in several beakers with DI water, held under a DI water gooseneck faucet for 5 min, and dried with N₂. The substrate was subsequently soaked in NH₄OH (1: 100; 3 mL:300 mL) for 5 min, rinsed with DI water using a handgun, soaked in three successive beakers with DI water for 1 min each, and then rinsed again with DI water using the handgun. The substrate was then plasma cleaned by exposing the substrate surface to O₂. A SAMCO PC-300 instrument was used to plasma etch O₂ at 250 watts for 1 min in downstream mode.

The cleaned substrate surface was actively functionalized with a solution comprising N-(3-triethoxysilylpropyl)-4-hydroxybutyramide using a YES-1224P vapor deposition oven system with the following parameters: 0.5 to 1 torr, 60 min, 70 °C, 135 °C vaporizer. The substrate surface was resist coated using a Brewer Science 200X spin coater. SPR^{™} 3612 photoresist was spin coated on the substrate at 2500 rpm for 40 sec. The substrate was pre-baked for 30 min at 90 °C on a Brewer hot plate. The substrate was subjected to photolithography using a Karl Suss MA6 mask aligner instrument. The substrate was exposed for 2.2 sec and developed for 1 min in MSF 26A. Remaining developer was rinsed with the handgun and the substrate soaked in water for 5 min. The substrate was baked for 30 min at 100 °C in the oven, followed by visual inspection for lithography defects using a Nikon L200. A descum process was used to remove residual resist using the SAMCO PC-300 instrument to O₂ plasma etch at 250 watts for 1 min.

The substrate surface was passively functionalized with a 100 µL solution of perfluorooctyltrichlorosilane mixed with 10 µL light mineral oil. The substrate was placed in a chamber, pumped for 10 min, and then the valve was closed to the pump and left to stand for 10 min. The chamber was vented to air. The substrate was resist stripped by performing two soaks for 5 min in 500 mL NMP at 70 °C with ultrasonication at maximum power (9 on Crest system). The substrate was then soaked for 5 min in 500 mL isopropanol at room temperature with ultrasonication at maximum power. The substrate was dipped in 300 mL of 200 proof ethanol and blown dry with N₂. The functionalized surface was activated to serve as a support for polynucleotide synthesis.

### Example 2: Synthesis of a 50-mer sequence on a polynucleotide synthesis device

A two dimensional polynucleotide synthesis device was assembled into a flowcell, which was connected to a flowcell (Applied Biosystems (ABI394 DNA Synthesizer"). The polynucleotide synthesis device was uniformly functionalized with N-(3-TRIETHOXYSILYLPROPYL)-4-HYDROXYBUTYRAMIDE (Gelest) was used to synthesize an exemplary polynucleotide of 50 bp ("50-mer polynucleotide") using polynucleotide synthesis methods described herein.

The sequence of the 50-mer was as described in SEQ ID NO.: 1. 5'AGACAATCAACCATTTGGGGTGGACAGCCTTGACCTCTAGACTTCGGCAT##TTTTTTT TTT3' (SEQ ID NO.: 1), where # denotes Thymidine-succinyl hexamide CED phosphoramidite (CLP-2244 from ChemGenes), which is a cleavable linker enabling the release of polynucleotides from the surface during deprotection.

The synthesis was done using standard DNA synthesis chemistry (coupling, capping, oxidation, and deblocking) according to the protocol in **Table 1** and an ABI synthesizer.

**Table 1**

| | **Table 1** | |
|---|---|---|
| **General DNA Synthesis Process Name** | **Process Step** | **Time (sec)** |
| **WASH** (Acetonitrile Wash Flow) | Acetonitrile System Flush | 4 |
| | Acetonitrile to Flowcell | 23 |
| | N2 System Flush | 4 |
| | Acetonitrile System Flush | 4 |
| **DNA BASE ADDITION** (Phosphoramidite + Activator Flow) | Activator Manifold Flush | 2 |
| | Activator to Flowcell | 6 |
| | Activator + Phosphoramidite to Flowcell | 6 |
| | Activator to Flowcell | 0.5 |
| | Activator + Phosphoramidite to Flowcell | 5 |
| | Activator to Flowcell | 0.5 |
| | Activator + Phosphoramidite to Flowcell | 5 |
| | Activator to Flowcell | 0.5 |
| | Activator + Phosphoramidite to Flowcell | 5 |
| | Incubate for 25sec | 25 |
| **WASH** (Acetonitrile Wash Flow) | Acetonitrile System Flush | 4 |
| | Acetonitrile to Flowcell | 15 |
| | N2 System Flush | 4 |
| | Acetonitrile System Flush | 4 |
| **DNA BASE ADDITION** (Phosphoramidite + Activator Flow) | Activator Manifold Flush | 2 |
| | Activator to Flowcell | 5 |
| | Activator + Phosphoramidite to Flowcell | 18 |
| | Incubate for 25sec | 25 |
| **WASH** (Acetonitrile Wash Flow) | Acetonitrile System Flush | 4 |
| | Acetonitrile to Flowcell | 15 |
| | N2 System Flush | 4 |
| | Acetonitrile System Flush | 4 |
| **CAPPING** (CapA+B, 1:1, Flow) | CapA+B to Flowcell | 15 |
| **WASH** (Acetonitrile Wash Flow) | Acetonitrile System Flush | 4 |
| | Acetonitrile to Flowcell | 15 |
| | Acetonitrile System Flush | 4 |
| **OXIDATION** (Oxidizer Flow) | Oxidizer to Flowcell | 18 |
| **WASH** (Acetonitrile Wash Flow) | Acetonitrile System Flush | 4 |
| | N2 System Flush | 4 |
| | Acetonitrile System Flush | 4 |
| | Acetonitrile to Flowcell | 15 |
| | Acetonitrile System Flush | 4 |
| | Acetonitrile to Flowcell | 15 |
| | N2 System Flush | 4 |
| | Acetonitrile System Flush | 4 |
| | Acetonitrile to Flowcell | 23 |
| | N2 System Flush | 4 |
| | Acetonitrile System Flush | 4 |
| **DEBLOCKING** (Deblock Flow) | Deblock to Flowcell | 36 |
| | Acetonitrile System Flush | 4 |
| **WASH** (Acetonitrile Wash Flow) | N2 System Flush | 4 |
| | Acetonitrile System Flush | 4 |
| | Acetonitrile to Flowcell | 18 |
| | N2 System Flush | 4.13 |
| | Acetonitrile System Flush | 4.13 |
| | Acetonitrile to Flowcell | 15 |

The phosphoramidite/activator combination was delivered similar to the delivery of bulk reagents through the flowcell. No drying steps were performed as the environment stays "wet" with reagent the entire time.

The flow restrictor was removed from the ABI 394 synthesizer to enable faster flow. Without flow restrictor, flow rates for amidites (0.1M in ACN), Activator, (0.25M Benzoylthiotetrazole ("BTT"; 30-3070-xx from GlenResearch) in ACN), and Ox (0.02M I2 in 20% pyridine, 10% water, and 70% THF) were roughly ~100uL/sec, for acetonitrile ("ACN") and capping reagents (1:1 mix of CapA and CapB, wherein CapA is acetic anhydride in THF/Pyridine and CapB is 16% 1-methylimidizole in THF), roughly ~200uL/sec, and for Deblock (3% dichloroacetic acid in toluene), roughly -300uL/sec (compared to ~50uL/sec for all reagents with flow restrictor). The time to completely push out Oxidizer was observed, the timing for chemical flow times was adjusted accordingly and an extra ACN wash was introduced between different chemicals. After polynucleotide synthesis, the chip was deprotected in gaseous ammonia overnight at 75 psi. Five drops of water were applied to the surface to recover polynucleotides. The recovered polynucleotides were then analyzed on a BioAnalyzer small RNA chip (data not shown).

### Example 3: Synthesis of a 100-mer sequence on a polynucleotide synthesis device

The same process as described in Example 2 for the synthesis of the 50-mer sequence was used for the synthesis of a 100-mer polynucleotide ("100-mer polynucleotide"; 5' CGGGATCCTTATCGTCATCGTCGTACAGATCCCGACCCATTTGCTGTCCACCAGTCATG CTAGCCATACCATGATGATGATGATGATGAGAACCCCGCAT##TTTTTTTTTT3', where # denotes Thymidine-succinyl hexamide CED phosphoramidite (CLP-2244 from ChemGenes); SEQ ID NO.: 2) on two different silicon chips, the first one uniformly functionalized with N-(3-TRIETHOXYSILYLPROPYL)-4-HYDROXYBUTYRAMIDE and the second one functionalized with 5/95 mix of 11-acetoxyundecyltriethoxysilane and n-decyltriethoxysilane, and the polynucleotides extracted from the surface were analyzed on a BioAnalyzer instrument (data not shown).

All ten samples from the two chips were further PCR amplified using a forward (5'ATGCGGGGTTCTCATCATC3'; SEQ ID NO.: 3) and a reverse (5'CGGGATCCTTATCGTCATCG3'; SEQ ID NO.: 4) primer in a 50uL PCR mix (25uL NEB Q5 master mix, 2.5uL 10uM Forward primer, 2.5uL 10uM Reverse primer, 1uL polynucleotide extracted from the surface, and water up to 50uL) using the following thermal cycling program:
98 C, 30 sec
98 C, 10 sec; 63C, 10 sec; 72C, 10 sec; repeat 12 cycles
72C, 2 min

The PCR products were also run on a BioAnalyzer (data not shown), demonstrating sharp peaks at the 100-mer position. Next, the PCR amplified samples were cloned, and Sanger sequenced. **Table 2** summarizes the results from the Sanger sequencing for samples taken from spots 1-5 from chip 1 and for samples taken from spots 6-10 from chip 2.

**Table 2**

| **Spot** | **Error rate** | **Cycle efficiency** |
|---|---|---|
| 1 | 1/763 bp | 99.87% |
| 2 | 1/824 bp | 99.88% |
| 3 | 1/780 bp | 99.87% |
| 4 | 1/429 bp | 99.77% |
| 5 | 1/1525 bp | 99.93% |
| 6 | 1/1615 bp | 99.94% |
| 7 | 1/531 bp | 99.81% |
| 8 | 1/1769 bp | 99.94% |
| 9 | 1/854 bp | 99.88% |
| 10 | 1/1451 bp | 99.93% |

Thus, the high quality and uniformity of the synthesized polynucleotides were repeated on two chips with different surface chemistries. Overall, 89%, corresponding to 233 out of 262 of the 100-mers that were sequenced were perfect sequences with no errors.

Finally, **Table 3** summarizes error characteristics for the sequences obtained from the polynucleotides samples from spots 1-10.

**Table 3**

| Sample ID/Spot no. | OSA_0 046/1 | OSA_0 047/2 | OSA_0 048/3 | OSA_0 049/4 | OSA_0 050/5 | OSA_0 051/6 | OSA_0 052/7 | OSA_0 053/8 | OSA_0 054/9 | OSA_00 55/10 |
|---|---|---|---|---|---|---|---|---|---|---|
| Total Sequences | 32 | 32 | 32 | 32 | 32 | 32 | 32 | 32 | 32 | 32 |
| Sequencin g Quality | 25 of 28 | 27 of 27 | 26 of 30 | 21 of 23 | 25 of 26 | 29 of 30 | 27 of 31 | 29 of 31 | 28 of 29 | 25 of 28 |
| Oligo Quality | 23 of 25 | 25 of 27 | 22 of 26 | 18 of 21 | 24 of 25 | 25 of 29 | 22 of 27 | 28 of 29 | 26 of 28 | 20 of 25 |
| ROI Match Count | 2500 | 2698 | 2561 | 2122 | 2499 | 2666 | 2625 | 2899 | 2798 | 2348 |
| ROI Mutation | 2 | 2 | 1 | 3 | 1 | 0 | 2 | 1 | 2 | 1 |
| ROI Multi Base Deletion | 0 | 0 | 0 | 0 | 0 | 0 | 0 | 0 | 0 | 0 |
| ROI Small Insertion | 1 | 0 | 0 | 0 | 0 | 0 | 0 | 0 | 0 | 0 |
| ROI Single Base Deletion | 0 | 0 | 0 | 0 | 0 | 0 | 0 | 0 | 0 | 0 |
| Large Deletion Count | 0 | 0 | 1 | 0 | 0 | 1 | 1 | 0 | 0 | 0 |
| Mutation: G>A | 2 | 2 | 1 | 2 | 1 | 0 | 2 | 1 | 2 | 1 |
| Mutation: T>C | 0 | 0 | 0 | 1 | 0 | 0 | 0 | 0 | 0 | 0 |
| ROI Error Count | 3 | 2 | 2 | 3 | 1 | 1 | 3 | 1 | 2 | 1 |
| ROI Error Rate | Err: ~1 in 834 | Err: ~1 in 1350 | Err: ~1 in 1282 | Err: ~1 in 708 | Err: ~1 in 2500 | Err: ~1 in 2667 | Err: ~1 in 876 | Err: ~1 in 2900 | Err: ~1 in 1400 | Err: ~1 in 2349 |
| ROI Minus Primer Error Rate | MP Err: ~1 in 763 | MP Err: ~1 in 824 | MP Err: ~1 in 780 | MP Err: ~1 in 429 | MP Err: ~1 in 1525 | MP Err: ~1 in 1615 | MP Err: ~1 in 531 | MP Err: ~1 in 1769 | MP Err: ~1 in 854 | MP Err: ~1 in 1451 |

### Example 4: Parallel assembly of 29,040 unique polynucleotides

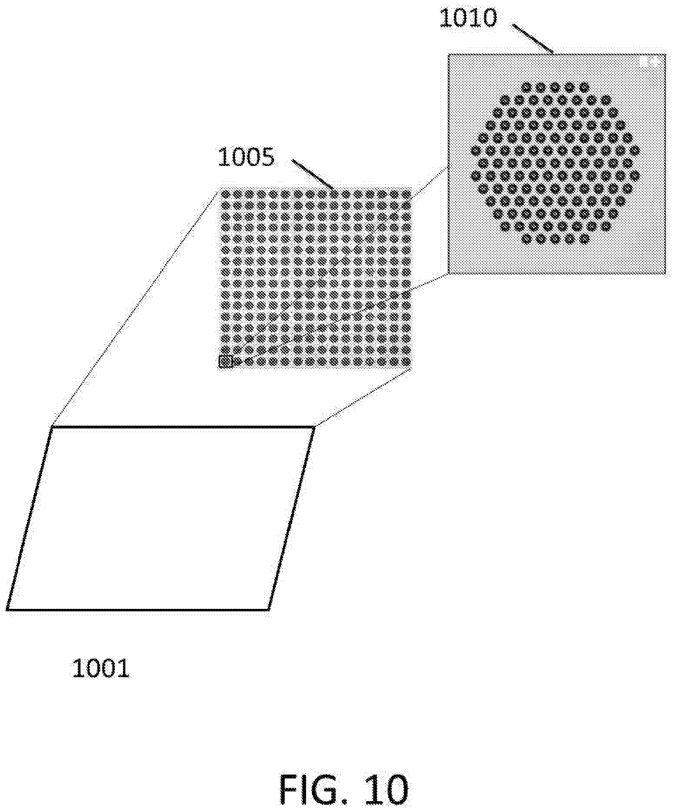

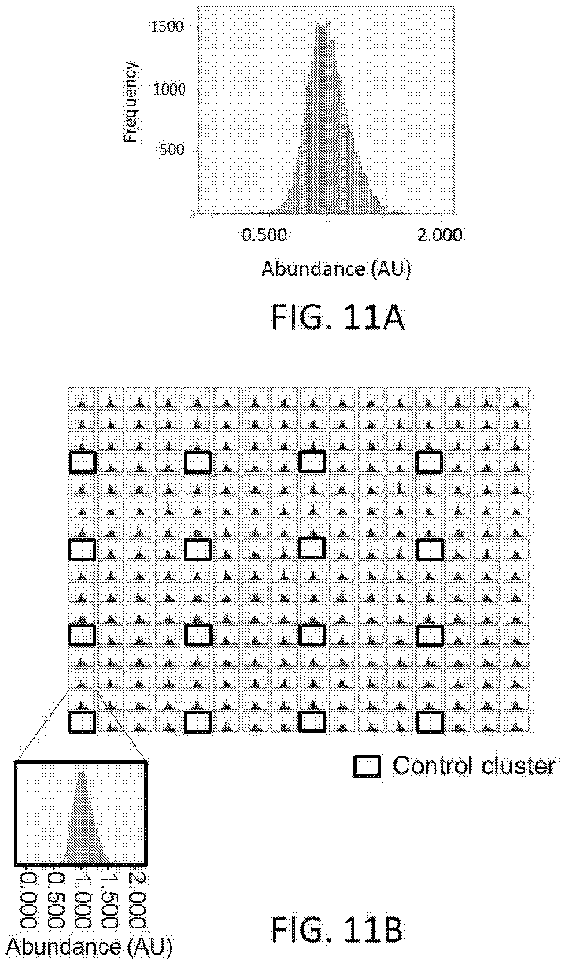

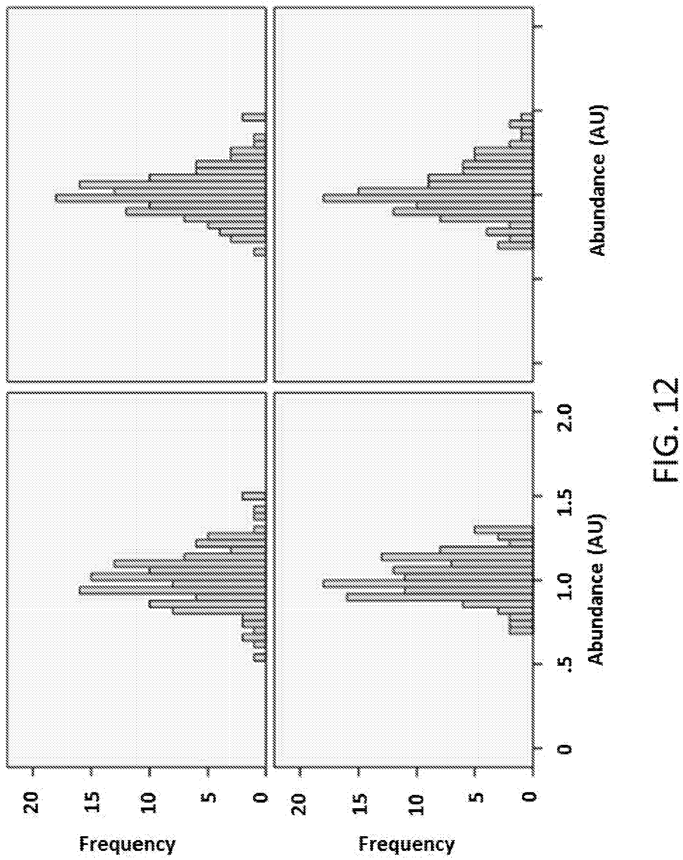

A structure comprising 256 clusters **1005** each comprising 121 loci on a flat silicon plate **1001** was manufactured as shown in **FIG. 10****.** An expanded view of a cluster is shown in **1010** with 121 loci. Loci from 240 of the 256 clusters provided an attachment and support for the synthesis of polynucleotides having distinct sequences. Polynucleotide synthesis was performed by phosphoramidite chemistry using general methods from Example 3. Loci from 16 of the 256 clusters were control clusters. The global distribution of the 29,040 unique polynucleotides synthesized (240 x 121) is shown in **FIG. 11A****.** Polynucleotide libraries were synthesized at high uniformity. 90% of sequences were present at signals within 4x of the mean, allowing for 100% representation. Distribution was measured for each cluster, as shown in **FIG. 11B****.** The distribution of unique polynucleotides synthesized in 4 representative clusters is shown in **FIG. 12****.** On a global level, all polynucleotides in the run were present and 99% of the polynucleotides had abundance that was within 2x of the mean indicating synthesis uniformity. This same observation was consistent on a per-cluster level.

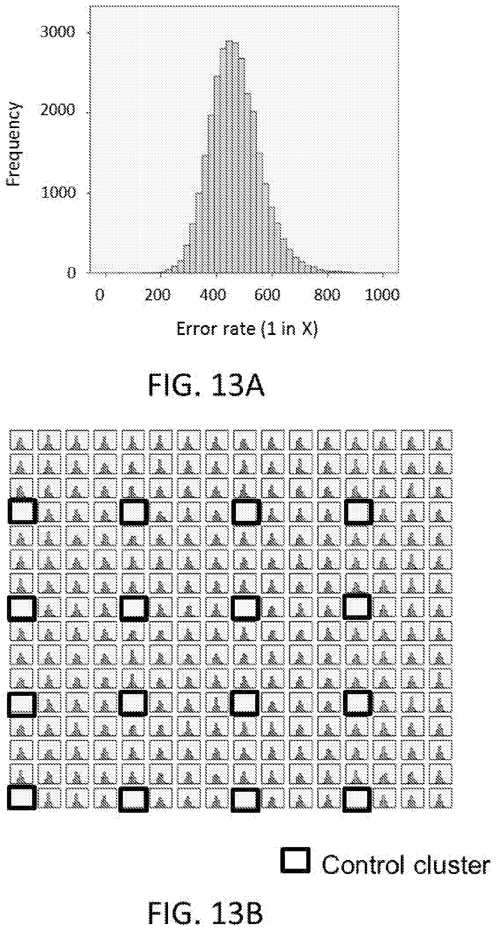

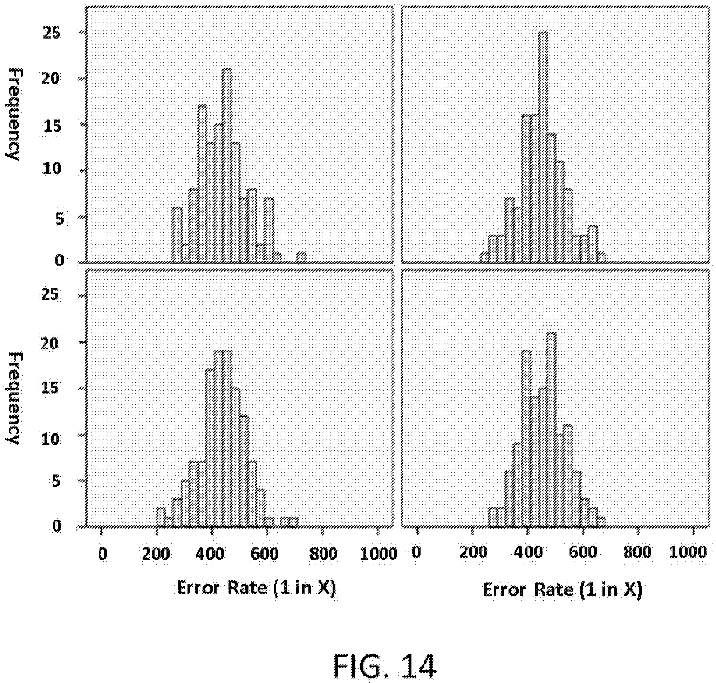

The error rate for each polynucleotide was determined using an Illumina MiSeq gene sequencer. The error rate distribution for the 29,040 unique polynucleotides is shown in **FIG. 13A** and averages around 1 in 500 bases, with some error rates as low as 1 in 800 bases. Distribution was measured for each cluster, as shown in **FIG. 13B****.** The error rate distribution for unique polynucleotides in four representative clusters is shown in **FIG. 14****.** The library of 29,040 unique polynucleotides was synthesized in less than 20 hours.

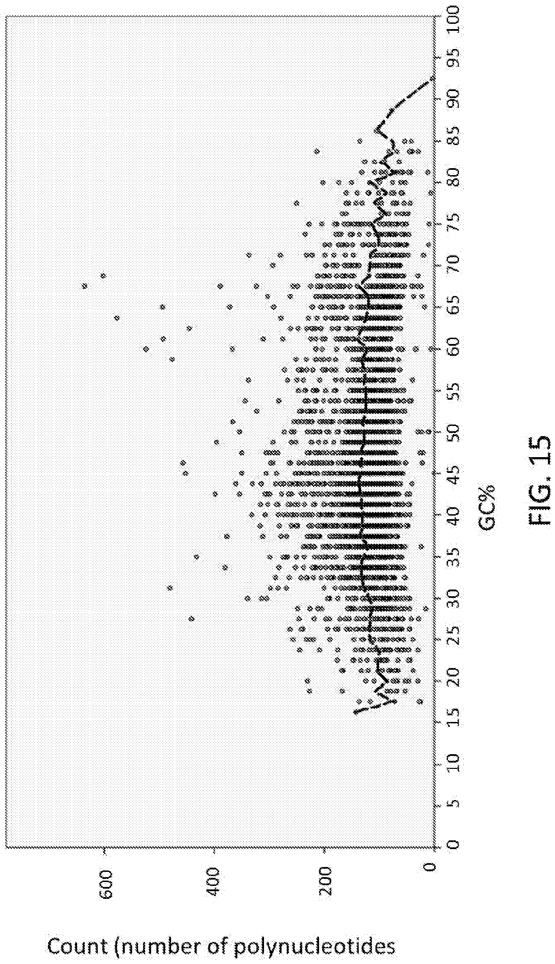

Analysis of GC percentage versus polynucleotide representation across all of the 29,040 unique polynucleotides showed that synthesis was uniform despite GC content, **FIG. 15****.**

### Example 5: PCR amplification of a synthesized polynucleotide library

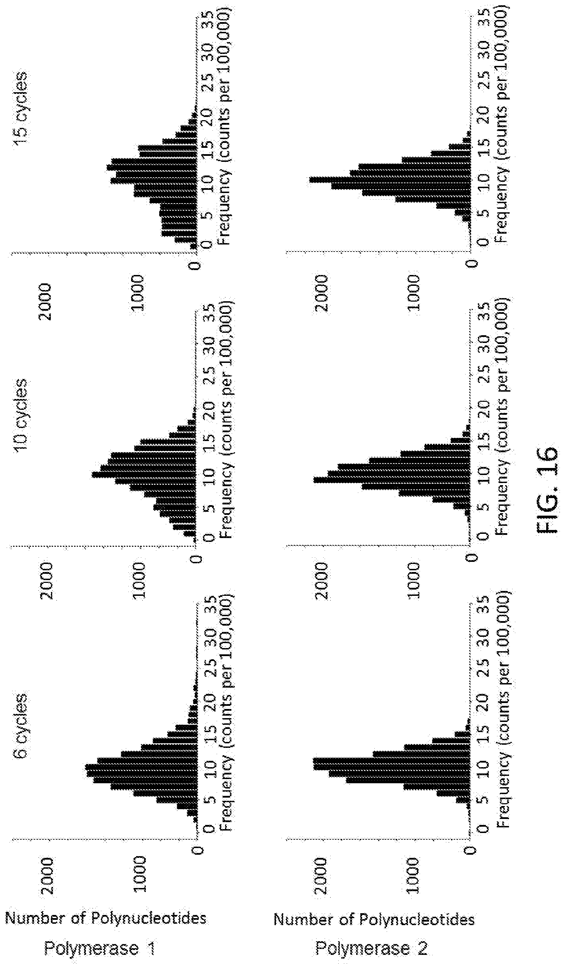

9,996 polynucleotides, each 100 bases in length of randomized sequences with varying GC content, from 20-80% GC were designed and synthesized on a structure with a similar arrangement is described in Example 3. To determine the effect of PCR amplification on GC representation, the polynucleotide population was amplified for either 6 or 20 cycles with a high fidelity DNA polymerase (DNA polymerase 1). Alternatively, the polynucleotide population was amplified using two other high-fidelity PCR enzymes for 6, 8, 10 or 15 cycles, to determine whether polymerase selection had an effect on overall sequence representation post-amplification. Following PCR amplification, samples were prepped for next generation sequencing and sequenced on the Illumina MiSeq platform. 150 bp SE reads were generated to an approximate read depth of 100X. Raw FASTQ files were analyzed. Polynucleotide representation with either polymerase for 6, 10 or 15 cycles is depicted in **FIG. 16****.** Polynucleotide representation uniformity was assessed for the various conditions and is summarized in **Table 4.**

**Table 4**

| | **Cycles** | **% within 1.5x** | **% within 2x** |
|---|---|---|---|
| Polymerase 1 | 6 | 72.1% | 92.6% |
| | 8 | 76.1% | 90.3% |
| | 10 | 70.9% | 86.6% |
| | 15 | 64.1% | 82.7% |
| Polymerase 2 | 6 | 91.9% | 98.9% |
| | 8 | 89.9% | 98.1% |
| | 10 | 90.1% | 98.4% |
| | 15 | 89.2% | 97.9% |

The number of dropouts for each amplified polynucleotide population was quantified as shown in **FIG. 15****,** amplification cycles versus fraction of population below a 10% of mean threshold. Polymerase 1 dropouts grew quickly whereas Polymerase 2 dropouts stayed relatively constant.

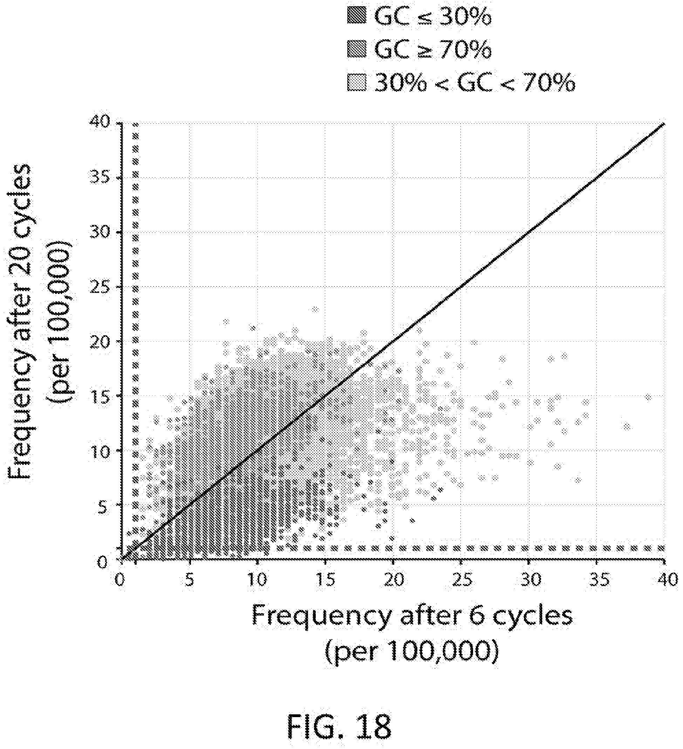

The impact of over amplification on GC distribution was assessed, **FIG. 18****.** Generally, polynucleotides with a GC content 30% to 70% followed the trend line, Y=X, and increased in frequency with more cycles. Polynucleotides with a GC content greater than 70% were, generally, slightly more frequent after 20 cycles, while polynucleotides with a GC content lower than 30% were, generally, slightly more frequent after 6 cycles.

### Example 6. Comparison of polynucleotide representation from whole plate amplification to parallel polynucleotide cluster amplification

Polynucleotides were synthesized on a structure comprising 256 clusters each comprising 121 loci on a flat silicon plate manufactured as shown in **FIG. 10****.** Polynucleotide synthesis was performed by phosphoramidite chemistry using general methods from Example 3. Polynucleotides on the structure were cleaved and combined.

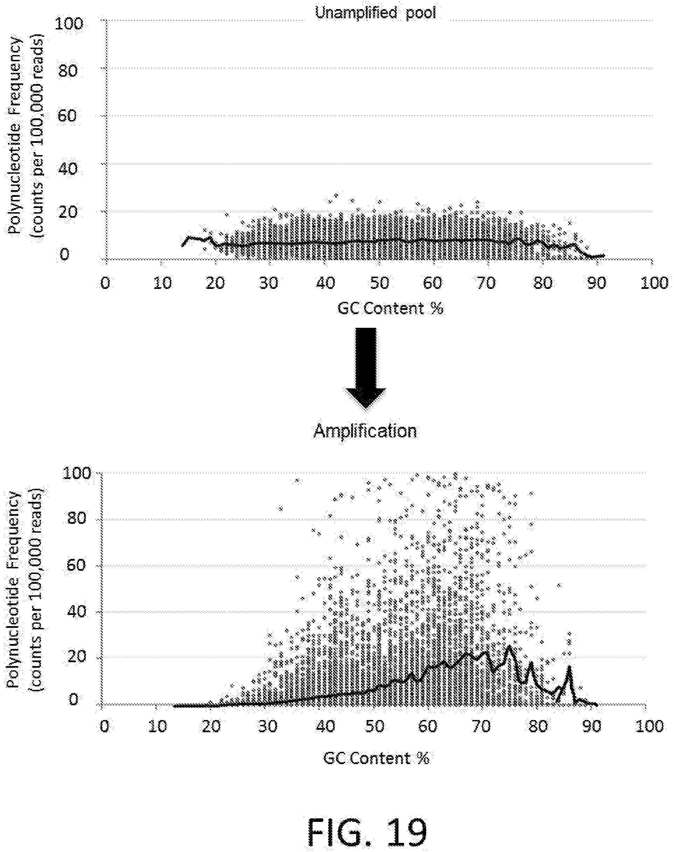

Polynucleotides were combined across the plate and amplified. Following amplification, there was noticeable GC bias and variance from the mean as seen in the line as seen in **FIG 19****.** As a result, more sequencing was required and there were more dropouts.

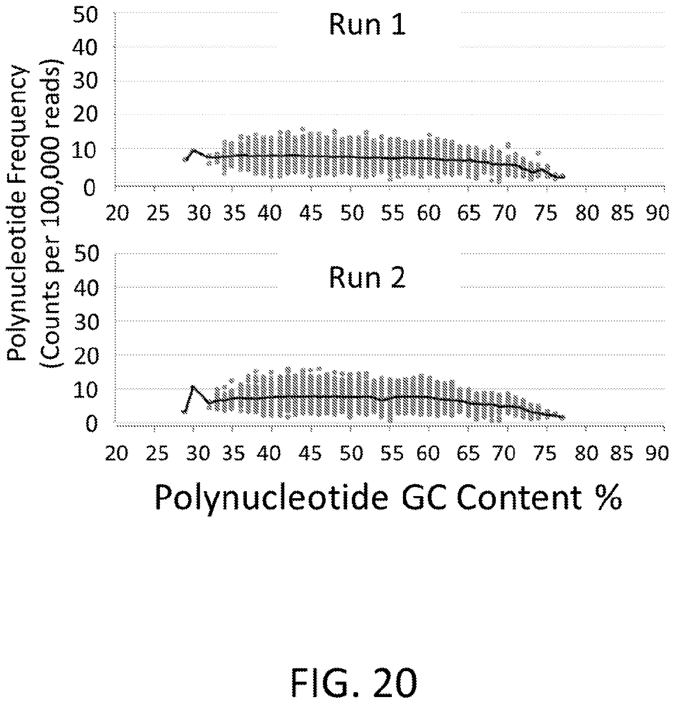

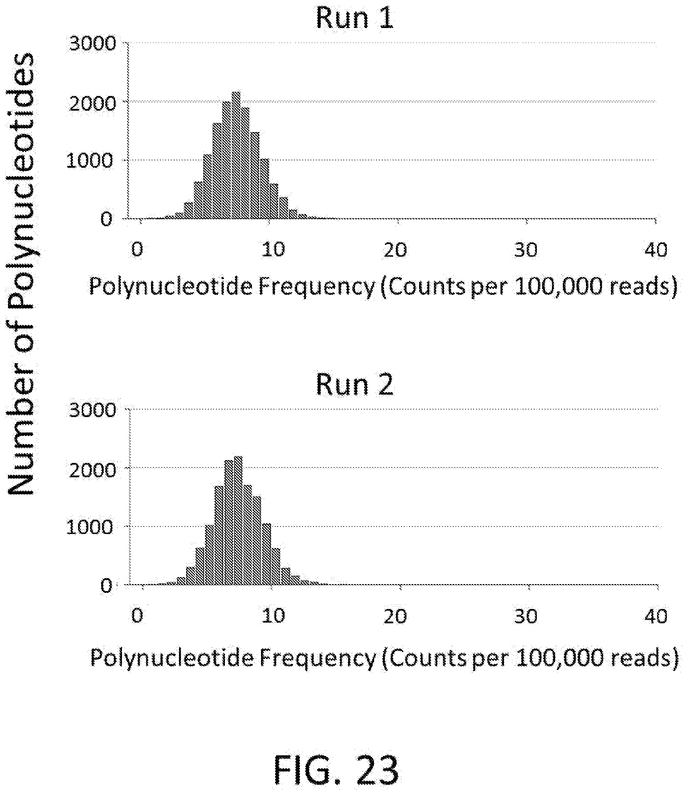

The distribution of polynucleotides from amplification of clusters is seen in **FIG. 20****.** In Run 1 and Run 2, the frequency distribution from the mean (line) was about 8, and the variance from the mean was about 1.7X. The GC percentage was in the range of 17% and 94%. **FIG. 23** and **FIG. 20** illustrate that there is reproducibility, and the polynucleotide population show a dramatic reduction in GC-bias **(****FIG. 20****).** In addition, there were zero dropouts and 30% less sequencing was required.

### Example 7. Polynucleotide libraries synthesized with different GC content

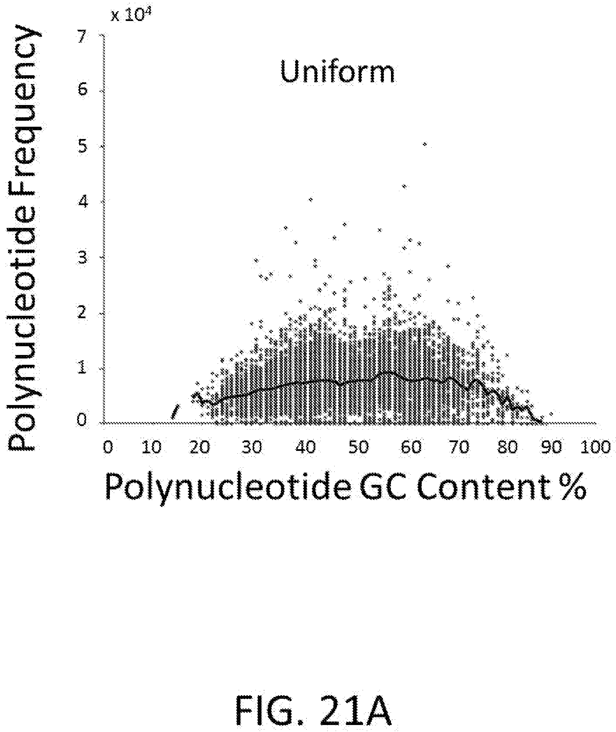

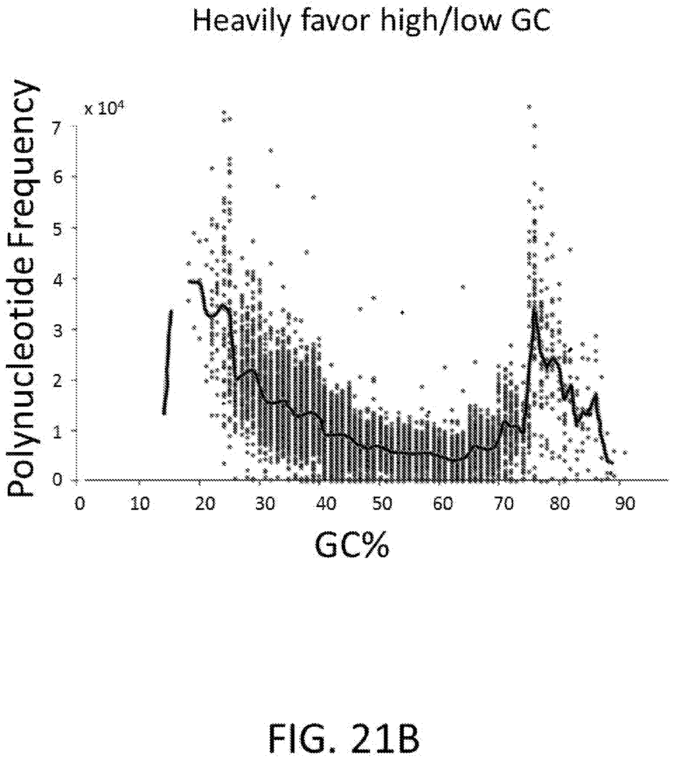

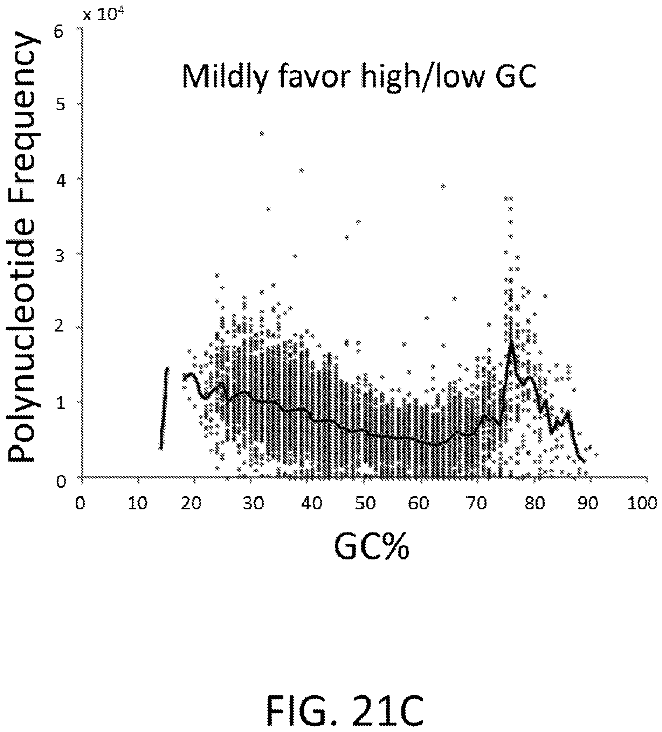

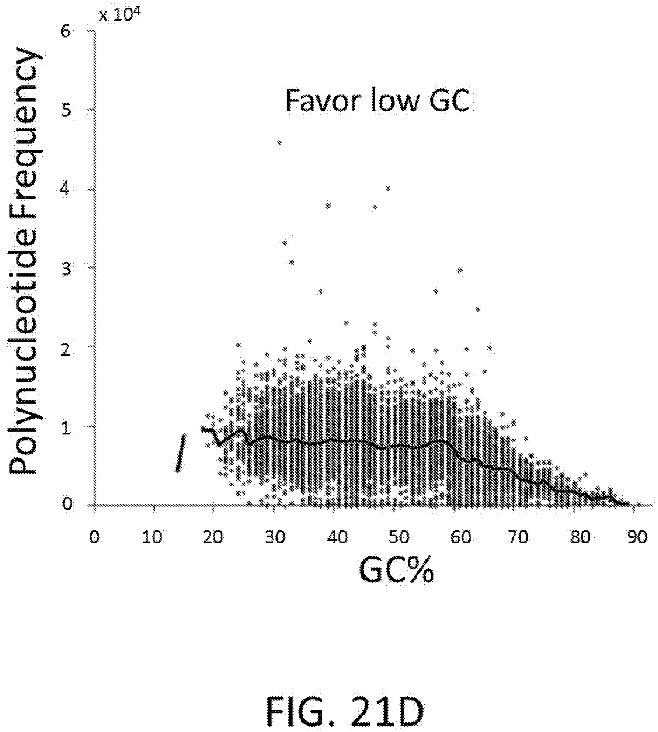

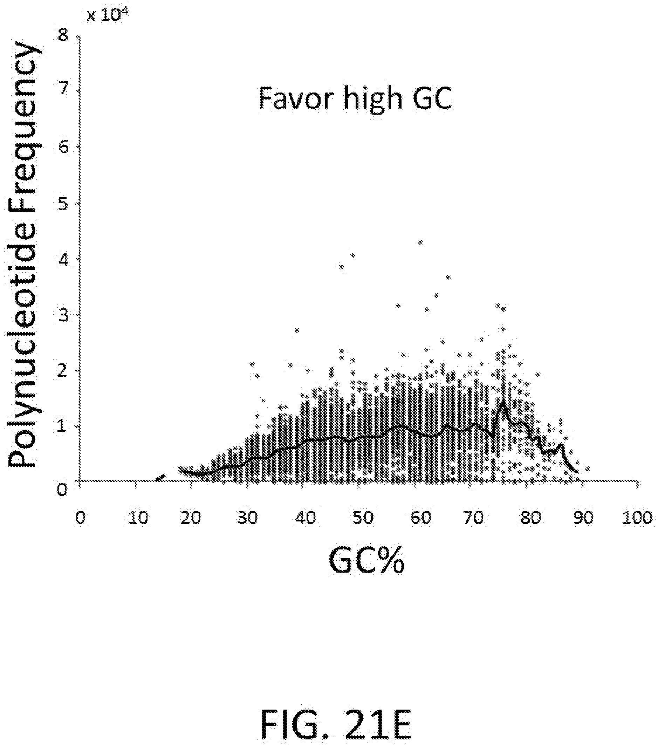

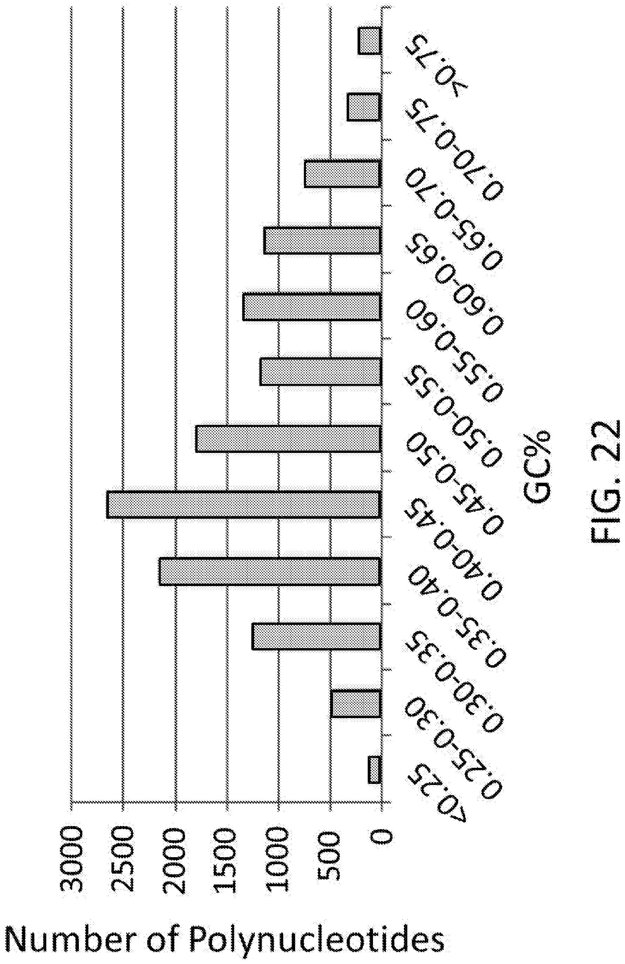

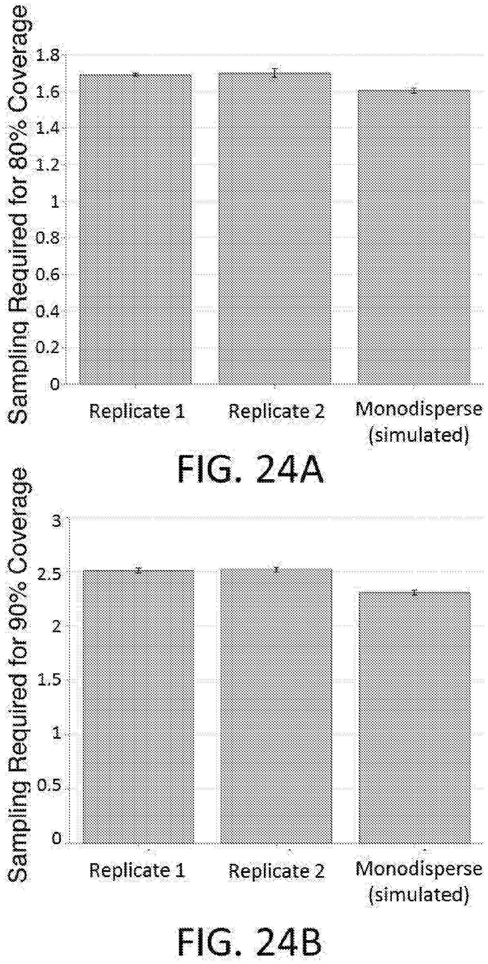

A library of 13,000 polynucleotide sequences containing GC content from about 15% to about 85% was preselected for synthesis **(****FIG. 22****).** A first polynucleotide library was synthesized on a structure, and synthesis was performed by phosphoramidite chemistry using general methods from Example 3. Polynucleotides on the structure were cleaved and combined followed by amplification to generate a PCR-biased library of polynucleotides. Polynucleotide sequences in the library were binned according to GC content, and the stoichiometry of each bin was adjusted to account for the observed GC bias generated by PCR amplification. For example, polynucleotides containing higher or lower GC content have higher initial concentrations that lead to uniform stoichiometric representation after amplification. This effectively reduces or eliminates PCR GC bias from the amplification step. The second library of polynucleotides was synthesized on a structure, and synthesis was performed by phosphoramidite chemistry using general methods from Example 3. Polynucleotides on the structure were cleaved and combined followed by amplification to generate a highly uniform library of polynucleotides **(****FIG. 23****)** with uniform GC representation after amplification **(****FIG. 21A****).** One advantage of a GC balanced library is that it requires less sampling for a desired sampling coverage. For example, sampling rates for 80% and 90% coverage of the library approached the theoretical minimum for a monodispersed library **(****FIG. 24A** and **FIG. 24B****).** Polynucleotide libraries were also synthesized that favored varying degrees of both high and low GC content **(****FIGS. 21B** and **21C****,** respectively). A polynucleotide library was also synthesized to favor low GC content **(****FIG. 21D****)** or high GC content **(****FIG. 21E****).**

### Example 8. GC-balanced polynucleotide libraries synthesized with 80- and 120-mer polynucleotide lengths

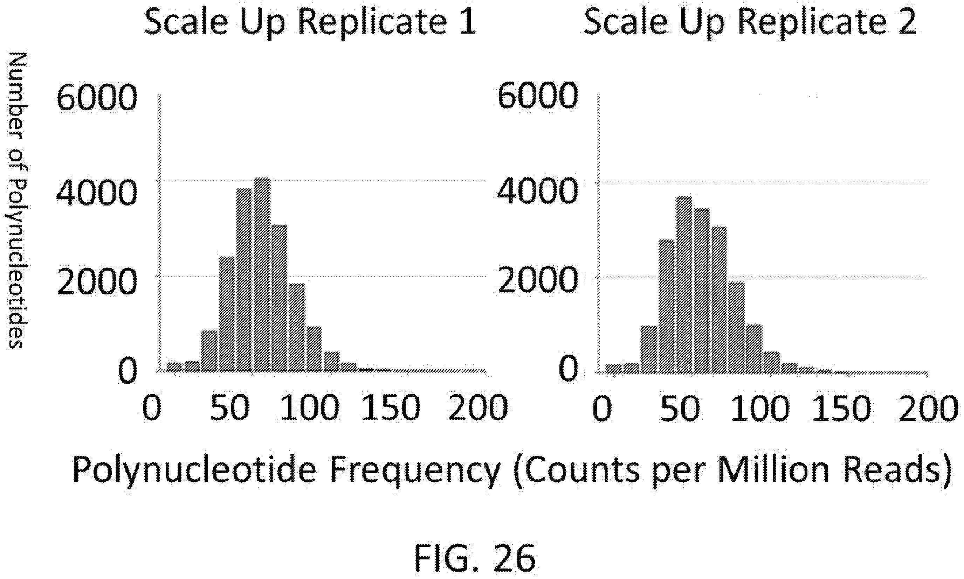

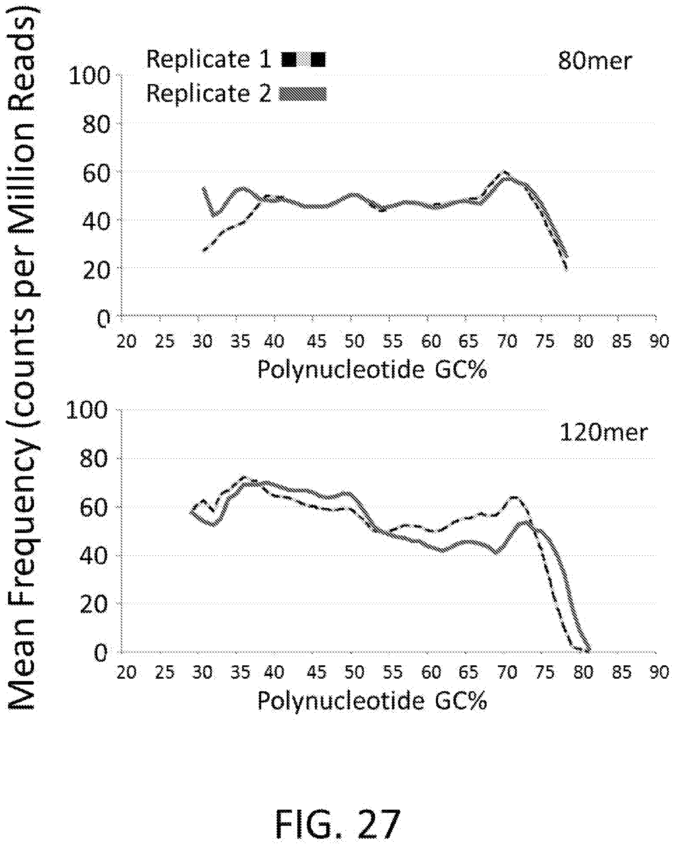

A library containing approximately 20,000 unique polynucleotides, each 80 nucleic acids in length was designed and GC-balanced using the general methods of Example 7, and synthesized on a structure; the synthesis was performed by phosphoramidite chemistry using general methods from Example 3. A similar library containing polynucleotides, each 120 nucleic acids in length was also synthesized **(****FIG. 26****).** Both libraries showed a highly uniform distribution, with >99% of the unique sequences identified. The libraries also displayed uniformity across variance in GC content, with high agreement across replicates **(****FIG. 27****),** with the low number of polynucleotides at the tails subject to noise.

### Example 9. GC content assessment after repeated amplification of a polynucleotide library

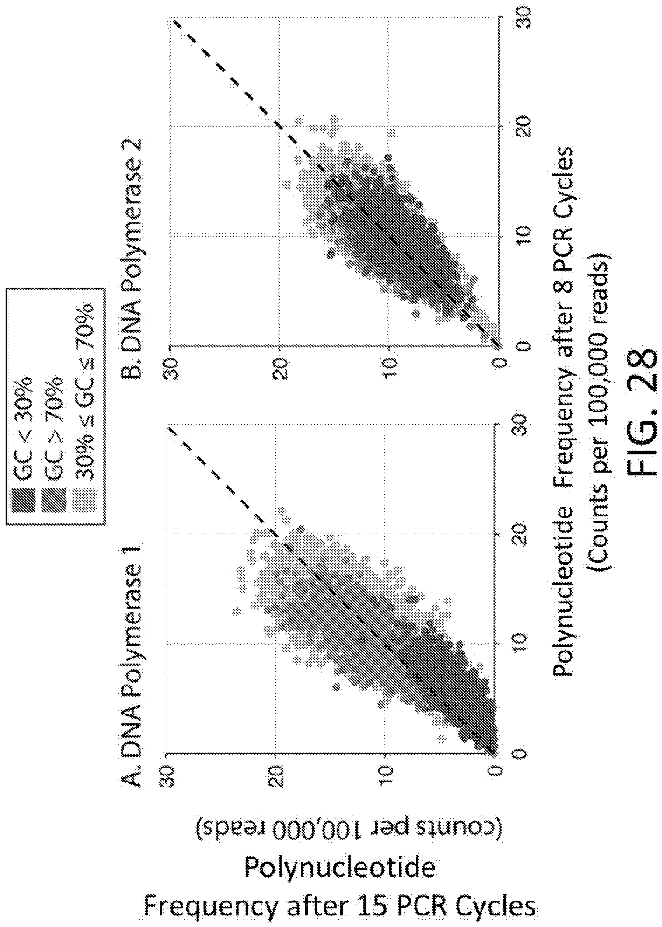

A polynucleotide library consisting of 9,996 unique polynucleotides containing GC content from 20-80%, each 100 bases long was synthesized on a structure by phosphoramidite chemistry using the general methods from Example 3. The library was amplified for either 8 or 15 PCR cycles with two different high-fidelity DNA polymerases and the frequency of polynucleotides in the population were compared between these two conditions **(****FIG. 28****).** The identity line (black dashed line) indicates polynucleotides with the same frequency in a population following either 8 or 15 PCR cycles. Sequences above the identity line are over-represented in the population following 15 cycles, and sequences below the line are under-represented in the population following 15 cycles, relative to 8 cycles of amplification. In this case, polymerase 1 exhibits a GC bias with increasing PCR cycles. High-GC sequences (GC over 70%, medium grey) were observed to be over-represented, and low-GC sequences (GC under 30%, shown in darkest grey) were under-represented following 15 cycles of amplification, compared to 8 cycles. In addition, the large magnitude of variation in enrichment within similar GC percentages suggests that factors other than GC content, such as hairpin formation or homopolymeric stretches, can influence amplification bias. Polymerase 2 did not exhibit the same sequence representation bias.

### Example 10. Dropouts and representation assessment after repeated amplification of a polynucleotide library

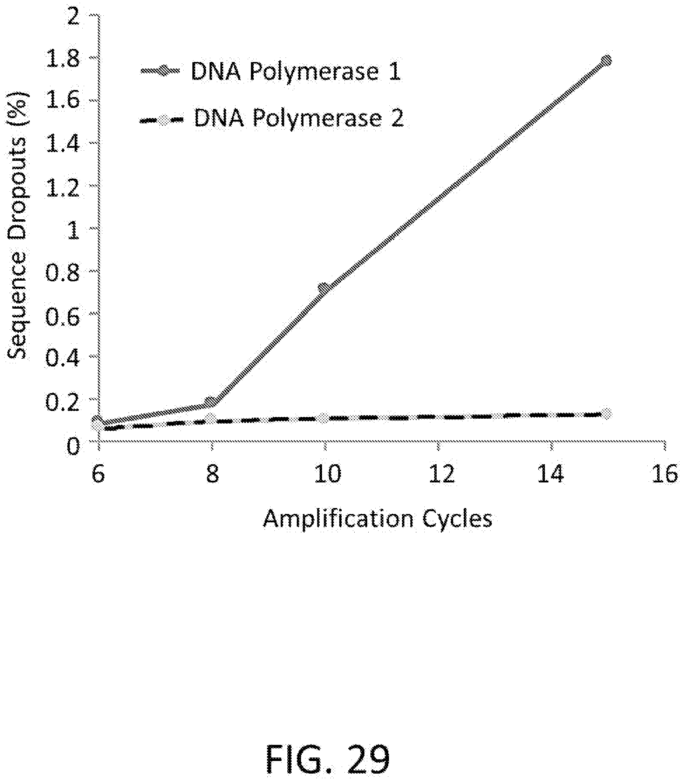

Bias introduced by amplification with different DNA polymerase enzymes was investigated. The polynucleotide library of Example 9 was amplified with either DNA polymerase 1 **(****FIG. 29****,** dark line) or DNA polymerase 2 **(****FIG. 29****,** light line) for 6, 8, 10, or 15 cycles. Increasing PCR cycles correlated with increased polynucleotide sequence dropout frequency, where dropout frequency is defined as sequences with an abundance less than 10% of the mean. The extent of this effect was dependent on the DNA polymerase used for the amplification. A greater proportion of sequences dropped out following amplification with DNA polymerase 1 (approximately 20 times more dropouts at 15 cycles) compared to amplification with DNA polymerase 2. Different polymerases may be optimal for amplifying different library sequences depending on GC content, length, and sequence complexity.

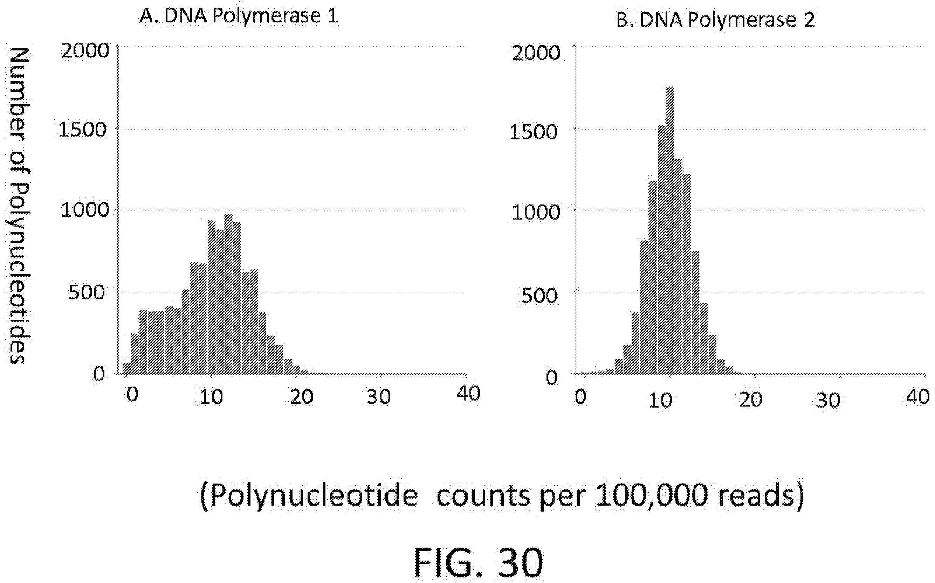

The libraries amplified for 15 PCR cycles with each DNA polymerase were investigated in more detail to assess the representation of the polynucleotide sequences **(****FIG. 30****).** Amplification of the polynucleotide library with DNA polymerase 1 resulted in 20 times more sequence dropouts following 15 PCR cycles **(****FIG. 29****).** The distribution of polynucleotides amplified with DNA polymerase 1 was greater than the distribution of polynucleotides amplified with DNA polymerase 2. The polynucleotides distribution of the library amplified with DNA polymerase 1 had 64% of sequences present within 1.5-fold of the mean. When the same library was amplified with DNA polymerase 2, >89% of the sequences were present within 1.5-fold of the mean, indicating that DNA polymerase 2 amplified the library with much lower bias than DNA polymerase 1. The bias introduced in the library amplified with DNA polymerase 1 increases the screening effort needed to cover a polynucleotide library.

### Example 11. Use of a controlled stoichiometry polynucleotide library for exome targeting with Next Generation Sequencing (NGS)

A first polynucleotide cDNA targeting library (probe library), comprising up to 370,000 or more non-identical polynucleotides which overlap with one or more gene exons is designed and synthesized on a structure by phosphoramidite chemistry using the general methods from Example 3. The polynucleotides are ligated to a molecular tag such as biotin using PCR (or directly during solid-phase synthesis) to form a probe for subsequent capture of the target exons of interest. The probes are hybridized to sequences in a library of genomic nucleic acids, and separated from non-binding sequences. Unbound probes are washed away, leaving the target library enriched in cDNA sequences. The enriched library is then sequenced using NGS, and reads for each expected gene are measured as a function of the cDNA probe(s) used to target the gene.

In some instances, a target sequence's frequency of reads is affected by target sequence abundance, probe binding, secondary structure, or other factors which decrease representation after sequencing of the target sequence despite enrichment. Polynucleotide library stoichiometric control is performed by modifying the stoichiometry of the first polynucleotide cDNA targeting library to obtain a second polynucleotide cDNA targeting library, with increased stoichiometry for polynucleotide probe sequences that lead to fewer reads. This second cDNA targeting library is designed and synthesized on a structure by phosphoramidite chemistry using the general methods from Example 3, and used to enrich sequence exons of the target genomic DNA library as described previously.

### Example 12. Multiple iterations of stoichiometric control with an exome probe library

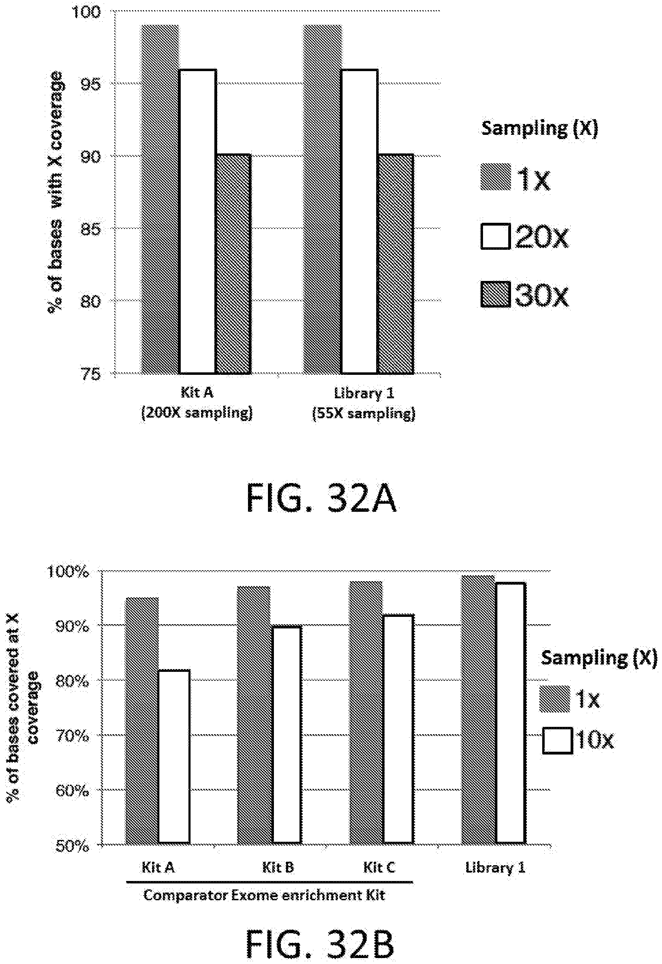

An exome probe library was synthesized and tested using the general methods of Example 11. Multiple iterations of stoichiometric modification were performed resulting in a controlled stoichiometry probe library, Library 1. Compared to several comparator exome enrichment kits, this resulted in significantly fewer sequencing reads to obtain the desired coverage of the targets. For accurate sequencing, a 30x read depth of at least 90% of the target exome bases is desirable, and over-sequencing (theoretical read depth, more than 30x read depth) is often needed to compensate for uniformity issues. The controlled stoichiometry exome probe library was able to achieve 30x read depth of 90% of the target bases with 55x theoretical read depth **(****FIG. 32A****),** which was significantly less sequencing coverage than required by another comparator exome enrichment kit, and faster sequence throughput (samples per run, **Table 5)**. When normalized to 4.5Gb of sequencing, the controlled stoichiometry probe library provided 10x read depth of >95% of all target bases, and which was significantly higher than all other comparator exome probe kits compared **(****FIG. 32B****).**

**Table 5**

| **Average Sequencing Coverage Required** | **Samples per run** |
|---|---|
| Comparator exome enrichment kit A | 4 |
| Controlled stoichiometry exome probes (Library 1) | 17 |

### Example 13: Production of Hybridization Panels

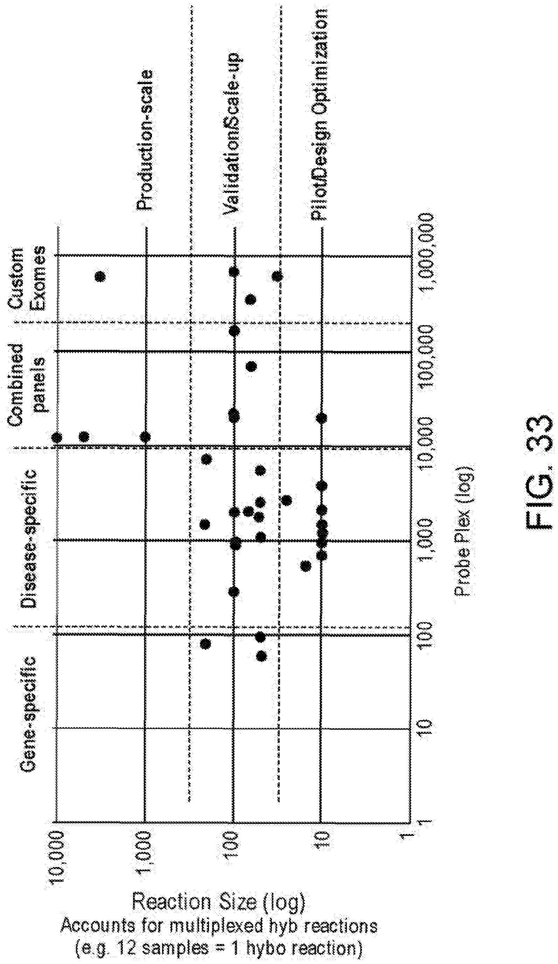

Polynucleotide targeting libraries were prepared using the general methods of Example 11 which target specific genes, diseases, combinations of panels, or custom exomes. Reaction sizes spanned 10³ in scale, and probe panel sizes ranged from about 80 to about 900,000 probes **(****FIG.** 33).

### Example 14: Production of a 70,000 Probe Panel

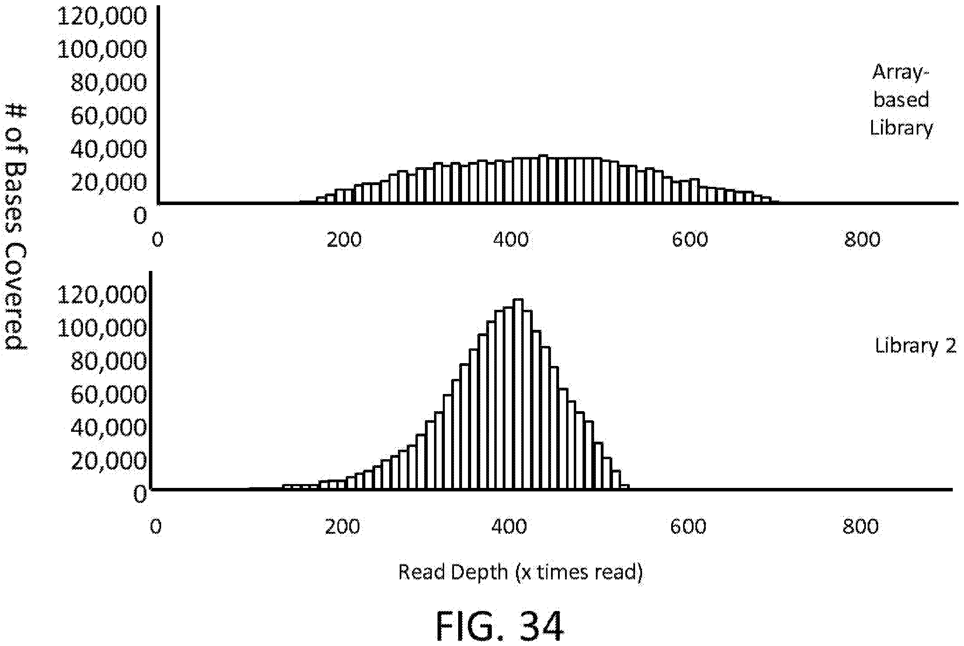

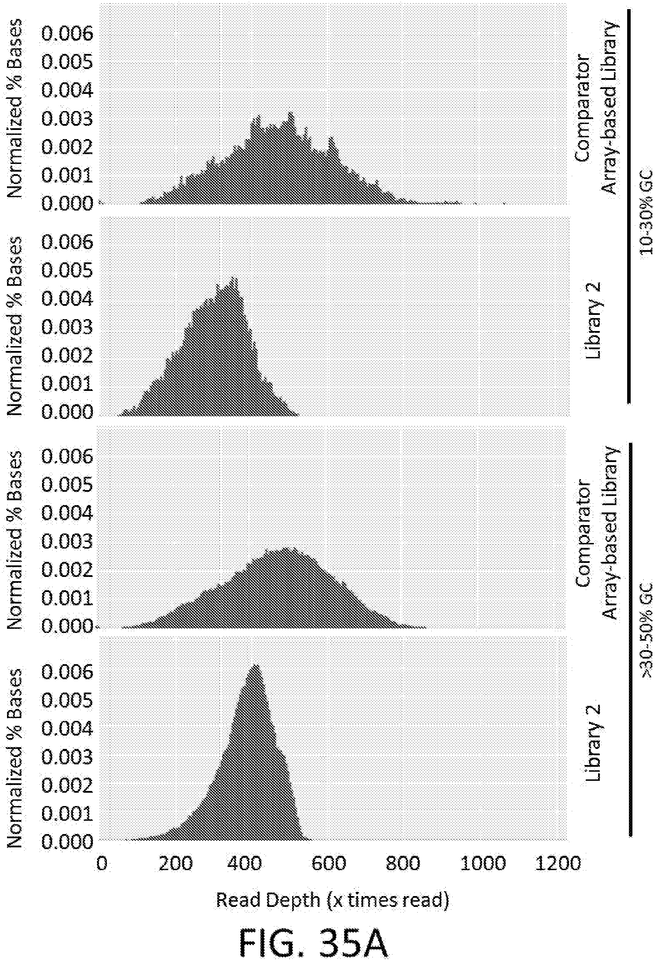

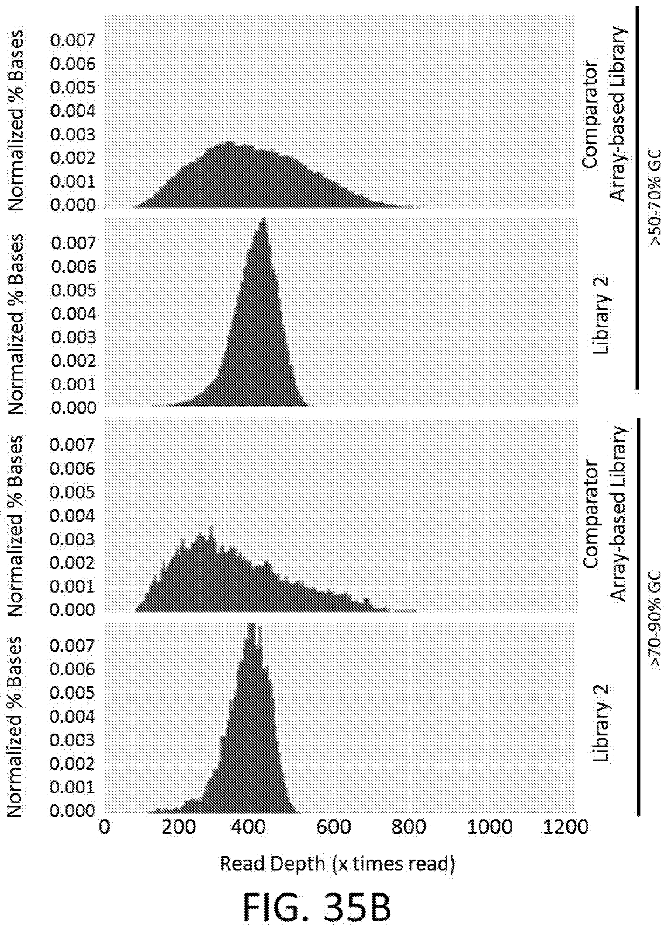

A polynucleotide targeting library (probe library), comprising 70,000 non-identical polynucleotides was designed and synthesized on a structure by phosphoramidite chemistry using the general methods from Example 3, and GC-controlled using the general methods of Example 11 to generate Library 2. The read distribution after sequencing is shown in **FIG. 34****,** and the GC-binned target coverage is shown in **FIG. 35A** and **FIG. 35B****.**

### Example 15: Production of a 2,544 Probe Panel

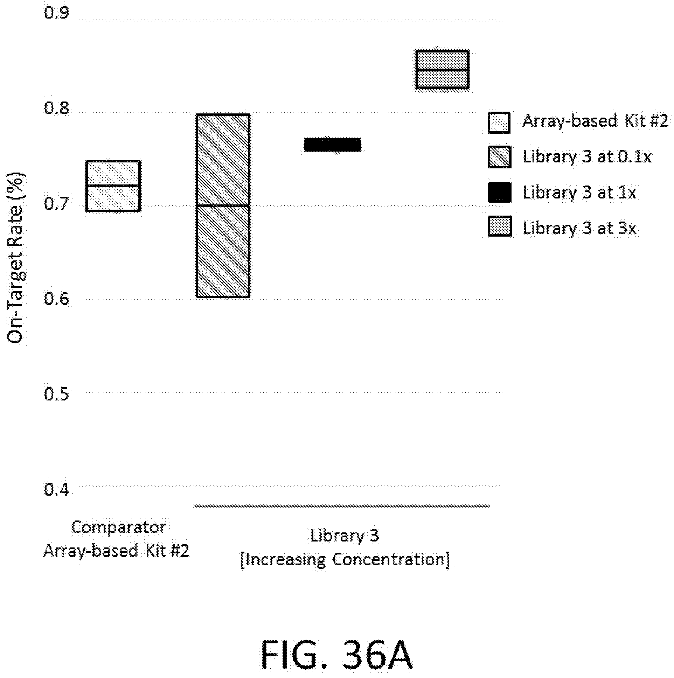

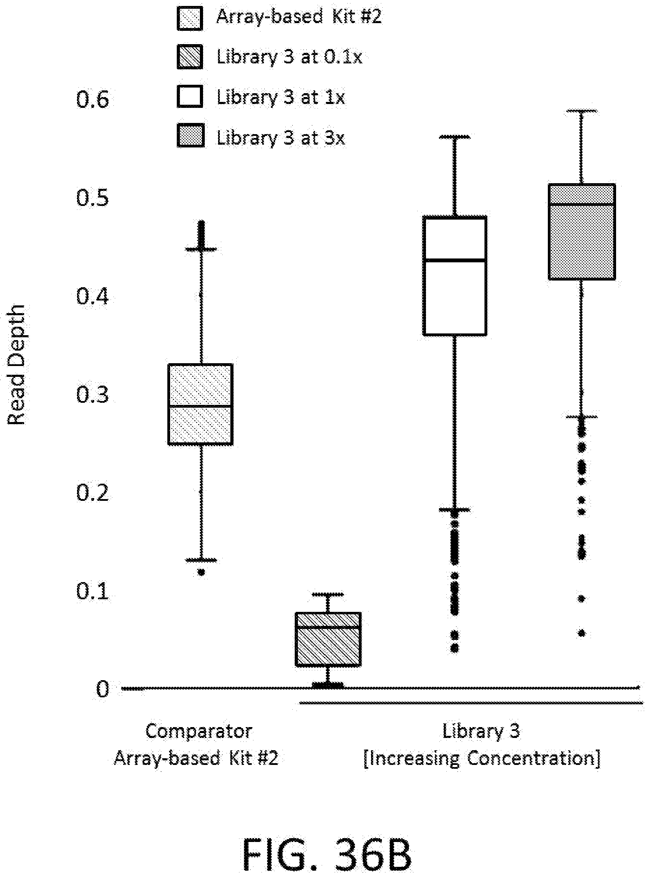

A polynucleotide targeting library (probe library), comprising 2,544 non-identical polynucleotides was designed and synthesized on a structure by phosphoramidite chemistry using the general methods from Example 3, and the stoichiometry controlled using the general methods of Example 11 to generate Library 3. The on-target rate is shown in **FIG. 36A****,** and the coverage rate is shown in **FIG. 36B****.** Target enrichment with Library 3 resulted in both a higher on-target rate and coverage rate than a comparator array-based kit #2.

### Example 16: Sample Preparation and Enrichment With a Polynucleotide Targeting Library

Genomic DNA (gDNA) is obtained from a sample, and fragmented enzymatically in a fragmentation buffer, end-repaired, and 3' adenylated. Adapters are ligated to both ends of the genomic DNA fragments to produce a library of adapter-tagged gDNA strands, and the adapter-tagged DNA library is amplified with a high-fidelity polymerase. The gDNA library is then denatured into single strands at 96°C, in the presence of adapter blockers. A polynucleotide targeting library (probe library) is denatured in a hybridization solution at 96°C, and combined with the denatured, tagged gDNA library in hybridization solution for 16 hours at 70°C. Binding buffer is then added to the hybridized tagged gDNA-probes, and magnetic beads comprising streptavidin are used to capture biotinylated probes. The beads are separated from the solution using a magnet, and the beads are washed three times with buffer to remove unbound adapters, gDNA, and adapter blockers before an elution buffer is added to release the enriched, tagged gDNA fragments from the beads. The enriched library of tagged gDNA fragments is amplified with a high-fidelity polymerase to get yields sufficient for cluster generation, and then the library is sequenced using an NGS instrument.

### Example 17: General Sample Preparation and Enrichment With a Polynucleotide Targeting Library

A plurality of polynucleotides is obtained from a sample, and fragmented, optionally end-repaired, and adenylated. Adapters are ligated to both ends of the polynucleotide fragments to produce a library of adapter-tagged polynucleotide strands, and the adapter-tagged polynucleotide library is amplified. The adapter-tagged polynucleotide library is then denatured at high temperature, preferably 96°C, in the presence of adapter blockers. A polynucleotide targeting library (probe library) is denatured in a hybridization solution at high temperature, preferably about 90 to 99°C, and combined with the denatured, tagged polynucleotide library in hybridization solution for about 10 to 24 hours at about 45 to 80°C. Binding buffer is then added to the hybridized tagged polynucleotide probes, and a solid support comprising a capture moiety are used to selectively bind the hybridized adapter-tagged polynucleotide-probes. The solid support is washed one or more times with buffer, preferably about 2 and 5 times to remove unbound polynucleotides before an elution buffer is added to release the enriched, adapter-tagged polynucleotide fragments from the solid support. The enriched library of adapter-tagged polynucleotide fragments is amplified and then the library is sequenced.

### Example 18: General Enrichment before Tagging With a Polynucleotide Targeting Library

A plurality of polynucleotides is obtained from a sample, fragmented, and optionally end-repaired. The fragmented polynucleotide sample is then denatured at high temperature, preferably 96°C. A polynucleotide targeting library (probe library) is denatured in a hybridization solution at high temperature, preferably about 90 to 99°C, and combined with the denatured, polynucleotide library in hybridization solution for about 10 to 24 hours at about 45 to 80°C. Binding buffer is then added to the hybridized polynucleotide probes, and a solid support comprising a capture moiety are used to selectively bind the hybridized fragmented polynucleotide-probes. The solid support is washed one or more times with buffer, preferably about 2 to 5 times to remove unbound polynucleotides before an elution buffer is added to release the enriched, polynucleotide fragments from the solid support. The enriched polynucleotides are adenylated, adapters are ligated to both ends of the polynucleotides to produce an enriched library of adapter-tagged polynucleotide strands, and the adapter-tagged polynucleotide library is amplified. The enriched library of adapter-tagged polynucleotide fragments is then sequenced.

### Example 19: General Sample Preparation and Filtering With a Polynucleotide Targeting Library

A plurality of polynucleotides is obtained from a sample, and fragmented, optionally end-repaired, and adenylated. Adapters are ligated to both ends of the polynucleotide fragments to produce a library of adapter-tagged polynucleotide strands, and the adapter-tagged polynucleotide library is amplified. The adapter-tagged polynucleotide library is then denatured at high temperature, preferably 96°C, in the presence of adapter blockers. A polynucleotide filtering library (probe library) designed to remove undesired, non-target sequences is denatured in a hybridization solution at high temperature, preferably about 90 to 99°C, and combined with the denatured, tagged polynucleotide library in hybridization solution for about 10 to 24 hours at about 45 to 80°C. Binding buffer is then added to the hybridized tagged polynucleotide probes, and a solid support comprising a capture moiety are used to selectively bind the hybridized adapter-tagged polynucleotide-probes. The solid support is washed one or more times with buffer, preferably about 1 to 5 times to elute target adapter-tagged polynucleotide fragments. The enriched library of target adapter-tagged polynucleotide fragments is amplified and then the library is sequenced.

### Example 20: Preparation of a 160-mer probe library

A library comprising at least 1,000 probes was synthesized on a structure, and synthesis is performed by phosphoramidite chemistry using general methods from Example 3. Each probe is double stranded, and each strand of the probe comprises a 120 nucleotide target binding sequence complementary to the target. Each probe further comprises a 20 nucleotide forward priming site and a 20 nucleotide reverse priming site. Each strand of the probes is labeled at the 5' position with two biotin molecules.

### Example 21: Preparation of a 210-mer probe library comprising a non-target binding sequence

A library comprising at least 1,000 probes was synthesized on a structure, and synthesis is performed by phosphoramidite chemistry using general methods from Example 3. Each probe is double stranded, and each strand of the probe comprises a 120 nucleotide target binding sequence complementary to the target. Each probe further comprises a 20 nucleotide forward primer binding site, a 20 nucleotide reverse primer binding site, a 25 nucleotide 5' non-target binding sequence comprising polyadenine, and a 25 nucleotide 3' non-target binding sequence comprising polyadenine. Each strand of the probes is labeled at the 5' position with two biotin molecules.

### Example 22: 210-mer probes targeting exon 1 of human HLA

A library comprising at least 1,000 probes was synthesized on a structure, and synthesis is performed by phosphoramidite chemistry using general methods from Example 3. Each probe is double stranded, and each strand of the probe comprises a 120 nucleotide target binding sequence complementary to a region of exon 1 of human HLA. Each probe further comprises a 20 nucleotide forward primer binding site, a 20 nucleotide reverse primer binding site, a 25 nucleotide 5' non-target binding sequence comprising polyadenine, and a 25 nucleotide 3' non-target binding sequence comprising polyadenine. Each strand of the probes is labeled at the 5' position with two biotin molecules.

### Example 22: Design method for a non-overlapping probe library

At least 100 target sequences are provided and sorted into discrete categories based on length, compared to the desired length of a complementary probe target binding sequence. For example, categories include but are not limited to (a) targets shorter than the insert length, (b) targets shorter or equal to the insert length + X, and (c) targets longer than the insert length + X, wherein X is a desired gap length that is not targeted by a probe. Target sequence in category (a) are targeted with target binding sequences that are either centered or aligned on the left or right side of the target sequence, depending on the complexity of the non-target region (repeating, high/low GC, palindromic sequence, etc.) that the insert will also be complementary to. Target sequences in category (b) are targeted in the same manner as category (a), wherein X is a desired gap length that the target binding sequence does not target. For targets in category (c), the total length of the target is divided by the length of the target binding sequence, and rounded up to the nearest integer value, which represents the number of target binding sequences needed to completely target all of the target sequence to generate an insert set for the target sequence. Optionally, the number of target binding sequences may be reduced wherein after reduction, gaps between the target binding sequences are less than a desired gap length Y. This overall process is then repeated for each of the target sequences, forming an insert library. The target binding sequences in the library are then modified by adding one or more non-target sequences comprising one or more priming sequences, the library is synthesized on a structure, synthesis is performed by phosphoramidite chemistry using general methods from Example 3, and probes are labeled with a molecular tag(s).

### Example 23: Design method for an overlapping probe library

At least 100 target sequences are provided and sorted into discrete categories based on length, compared to the desired length of a complementary probe target binding sequence. For example, categories include but are not limited to (a) targets shorter than the insert length, (b) targets shorter or equal to the insert length + X, and (c) targets longer than the insert length + X, wherein X is a desired gap length that is not targeted by a probe. Target sequence in category (a) are targeted with target binding sequences that are either centered or aligned on the left or right side of the target sequence, depending on the complexity of the non-target region (repeating, high/low GC, palindromic sequence, etc.) that the insert will also be complementary to. Target sequences in category (b) are targeted in the same manner as category (a), wherein X is a desired gap length that the target binding sequence does not target. For targets in category (c), the total length of the target is divided by the length of the target binding sequence, and rounded up to the nearest integer value, which represents the number of target binding sequences needed to completely target all of the target sequence. Complementary target binding sequences are then spaced across the target sequence (optionally evenly), allowing for overlap to generate an insert set for the target sequence. This overall process is then repeated for each of the target sequences, forming an insert library. The target binding sequences in the library are then modified by adding one or more non-target sequences comprising one or more priming sequences, the library is synthesized on a structure, synthesis is performed by phosphoramidite chemistry using general methods from Example 3, and probes are labeled with a molecular tag(s).

### Example 24: Design method for a mixed probe library

A probe library is synthesized following the general methods of Example 22 and 23 with modification. An set comprising non-overlapping inserts, overlapping inserts, or mixed (overlapping and non-overlapping) inserts is generated for each target sequence.

### Example 25: Polynucleotide probes for exon targeting

Polynucleotide probes may target exons in a genome, and a gene may comprise a plurality of exons. For example, the human leukocyte antigen (HLA) gene comprises seven exons, three of which are listed in **Table 6.**

**Table 6**

| Seq ID | Description | Exon Length (bp) | Genomic Sequence Comprising an Exon (underlined) |
|---|---|---|---|
| 5 | human HLA gene exon 1 | 63 | CCCACCGGGACTCAGATTCTCCCCAGACGCCGAGGAT GGTGCTCATGGCGCCCCGAACCCTCCTCCTGCTGCTC TCAGGGGCCCTGGCCCTGACCCAGACCTGGGCGCGT GAGTGCAGGGTCTGCAGGGAAATGGGCGCGTGAGTG CAGGGTCTGC |
| 6 | human HLA gene exon 2 | 270 | GCTCCCAGGTTCCCACTCCATGAGGTATTTCTACACC ACCATGTCCCGGCCCGGCGCCGGGGAGCCCCGCTTCA TCTCCGTCGGCTACGTGGACGATACGCAGTTCGTGCG GTTCGACAGCGACGACGCGAGTCCGAGAGAGGAGCC GCGGGCGCCGTGGATGGAGCGGGAGGGGCCAAAGTA TTGGGACCGGAACACACAGATCTGCAAGGCCCAGGC ACAGACTGAACGAGAGAACCTGCGGATCGCGCTCCG |
| | | | CTACTACAACCAGAGCGAGGGCGGTGAGTTGACCCC GG |
| 7 | human HLA gene exon 5 | 117 | TAGCAGGGTCAGGGTTCCCTCACCTTCCCCCCTTTTCC CAGCCATCTTCCCAGCCCACCGTCCCCATCGTGGGCA TCGTTGCTGGCTTGGTTCTACTTGTAGCTGTGGTCACT GGAGCTGTGGTCGCTGCTGTAATGTGGAGGAAGAAG AGCTCAGGTAAGGAAGGGGT |

For a given exon, various combinations of target binding sequences and non-target binding sequences may be used to design probes of various configurations and lengths. Non-limiting probe designs targeting HLA exon 1 are shown as examples in **Table 7,** and the sequence of only one strand of the probe is shown. The size of the non-target binding sequence(s), target binding sequence, and overall probe lengths are listed in **Table 8**.

**Table 7**

| Seq ID | Target | 5' Non-target binding sequence | Target binding sequence | 3' Non-target binding sequence |
|---|---|---|---|---|
| 8 | human HLA exon 1 | GTTACCCA AGAACGCA GCTGATTC TCCCCAGA CGCCGAGG ATGGTGCT C | ATGGCGCCCCGAACCCTCCTCCTGC TGCTCTCAGGGGCCCTGGCCCTGAC CCAGACCTGGGCG | CGTGAGTGCAG GGTCTGCAGGG AAATGGTAGTG TCGGAGGTCGT TCCT |
| 9 | human HLA exon 2 | GTTACCCA AGAACGCA GCTG | GGTTCCCACTCCATGAGGTATTTCT ACACCACCATGTCCCGGCCCGGCG CCGGGGAGCCCCGCTTCATCTCCGT CGGCTACGTGGACGATACGCAGTT CGTGCGGTTCGACAGCGACGAC | TAGTGTCGGAG GTCGTTCCT |
| 10 | human HLA exon 2 | GTTACCCA AGAACGCA GCTG | TGGATGGAGCGGGAGGGGCCAAAG TATTGGGACCGGAACACACAGATC TGCAAGGCCCAGGCACAGACTGAA CGAGAGAACCTGCGGATCGCGCTC CGCTACTACAACCAGAGCGAGGGC | TAGTGTCGGAG GTCGTTCCT |
| 11 | human HLA exon 2 | GTTACCCA AGAACGCA GCTG | GGCTACGTGGACGATACGCAGTTC GTGCGGTTCGACAGCGACGACGCG AGTCCGAGAGAGGAGCCGCGGGCG CCGTGGATGGAGCGGGAGGGGCCA AAGTATTGGGACCGGAACACACAG | TAGTGTCGGAG GTCGTTCCT |
| 12 | human HLA exon 1 | GTTACCCA AGAACGCA GCTG | ATGGCGCCCCGAACCCTCCTCCTGC TGCTCTCAGGGGCCCTGGCCCTGAC CCAGACCTGGGCG | TAGTGTCGGAG GTCGTTCCT |
| 13 | human HLA exon 2 | AAAAAAAA AAAAAAAA AAAAAAAA AGTTACCC AAGAACGC AGCTG | GGTTCCCACTCCATGAGGTATTTCT ACACCACCATGTCCCGGCCCGGCG CCGGGGAGCCCCGCTTCATCTCCGT CGGCTACGTGGACGATACGCAGTT CGTGCGGTTCGACAGCGACGAC | TAGTGTCGGAG GTCGTTCCTAAA AAAAAAAAAAAA AAAAAAAAAAAA |
| 14 | human HLA exon 2 | GTTACCCA AGAACGCA GCTGAAAA AAAAAAAA AAAAAAAA AAAAA | GGTTCCCACTCCATGAGGTATTTCT ACACCACCATGTCCCGGCCCGGCG CCGGGGAGCCCCGCTTCATCTCCGT CGGCTACGTGGACGATACGCAGTT CGTGCGGTTCGACAGCGACGAC | AAAAAAAAAAA AAAAAAAAAAA AAATAGTGTCG GAGGTCGTTCCT |
| 15 | human HLA exon 5 | GTTACCCA AGAACGCA GCTG | AGCCATCTTCCCAGCCCACCGTCCC CATCGTGGGCATCGTTGCTGGCTTG GTTCTACTTGTA | TAGTGTCGGAG GTCGTTCCT |
| 16 | human HLA exon 5 | GTTACCCA AGAACGCA GCTG | GCTGTGGTCACTGGAGCTGTGGTCG CTGCTGTAATGTGGAGGAAGAAGA GCTCAG | TAGTGTCGGAG GTCGTTCCT |

The priming sequence of the non-target binding sequence(s) is underlined.

**Table 8**

| Seq ID | Target | 5' Non-target binding sequence length (bp) | Target binding sequence length (bp) | 3' Non-target binding sequence length (bp) | Total probe length length (bp) |
|---|---|---|---|---|---|
| 8 | human HLA exon 1 | 49 | 63 | 48 | 160 |
| 9 | human HLA exon 2 | 20 | 120 | 20 | 160 |
| 10 | human HLA exon 2 | 20 | 120 | 20 | 160 |
| 11 | human HLA exon 2 | 20 | 120 | 20 | 160 |
| 12 | human HLA exon 1 | 20 | 63 | 20 | 103 |
| 13 | human HLA exon 2 | 45 | 120 | 45 | 210 |
| 14 | human HLA exon 2 | 45 | 120 | 45 | 210 |
| 15 | human HLA exon 5 | 20 | 62 | 20 | 102 |
| 16 | human HLA exon 5 | 20 | 55 | 20 | 95 |

Various arrangements of a plurality of probes may be used to cover a given exon, for example human HLA exon 2. Probes comprising SEQ IDs: 9 or 10 comprise a set and target human HLA exon 2, but together leave a gap in the target exon and do not comprise overlapping sequences (see **FIG. 4F****).** Probes comprising SEQ ID: 11 target HLA exon 2, and comprise an target binding sequence that overlaps with SEQ IDs: 9 and 10 (see **FIG. 4G****).** Probes each comprising SEQ IDs 15 or 16 comprise a set, target human HLA exon 5, and do not target any overlapping regions of the target or non-target regions. Probes corresponding to SEQ IDs 8-16 are synthesized on a structure, and synthesis is performed by phosphoramidite chemistry using general methods from Example 3, and optionally labeled with at least one molecular tag, such as biotin.

### Example 26: Genomic DNA capture with a polynucleotide probe library

A polynucleotide targeting library comprising at least 500,000 non-identical polynucleotides targeting the human exome was designed and synthesized on a structure by phosphoramidite chemistry using the general methods from Example 3, and the stoichiometry controlled using the general methods of Example 11 to generate Library 4. The polynucleotides were then labeled with biotin, and then dissolved to form an exome probe library solution. A dried indexed library pool was obtained from a genomic DNA (gDNA) sample using the general methods of Example 16.

The exome probe library solution, a hybridization solution, a blocker mix A, and a blocker mix B were mixed by pulse vortexing for 2 seconds. The hybridization solution was heated at 65°C for 10 minutes, or until all precipitate was dissolved, and then brought to room temperature on the benchtop for 5 additional minutes. 20 µL of hybridization solution and 4 µL of the exome probe library solution were added to a thin-walled PCR 0.2 mL strip-tube and mixed gently by pipetting. The combined hybridization solution/exome probe solution was heated to 95°C for 2 minutes in a thermal cycler with a 105°C lid and immediately cooled on ice for at least 10 minutes. The solution was then allowed to cool to room temperature on the benchtop for 5 minutes. While the hybridization solution/exome probe library solution was cooling, water was added to 9 µl for each genomic DNA sample, and 5 µL of blocker mix A, and 2 µL of blocker mix B were added to the dried indexed library pool in the thin-walled PCR 0.2 mL strip-tube. The solution was then mixed by gentle pipetting. The pooled library/blocker tube was heated at 95°C for 5 minutes in a thermal cycler with a 105°C lid, then brought to room temperature on the benchtop for no more than 5 minutes before proceeding onto the next step. The hybridization mix/probe solution was mixed by pipetting and added to the entire 24 µL of the pooled library/blocker tube. The entire capture reaction well was mixed by gentle pipetting, to avoid generating bubbles. The sample tube was pulse-spun to make sure the tube was sealed tightly. The capture/hybridization reaction was heated at 70 °C for 16 hours in a PCR thermocycler, with a lid temperature of 85 °C.

Binding buffer, wash Buffer 1 and wash Buffer 2 were heated at 48°C until all precipitate was dissolved into solution. 700 µL of wash buffer 2 was aliquoted per capture and preheated to 48°C. Streptavidin binding beads and DNA purification beads were equilibrated at room temperature for at least 30 minutes. A polymerase, such as KAPA HiFi HotStart ReadyMix and amplification primers were thawed on ice. Once the reagents were thawed, they were mixed by pulse vortexing for 2 seconds. 500 µL of 80 percent ethanol per capture reaction was prepared. Streptavidin binding beads were pre-equilibrated at room temperature and vortexed until homogenized. 100 µL of streptavidin binding beads were added to a clean 1.5 mL microcentrifuge tube per capture reaction. 200 µL of binding buffer was added to each tube and each tube was mixed by pipetting until homogenized. The tube was placed on magnetic stand. Streptavidin binding beads were pelleted within 1 minute. The tube was removed and the clear supernatant was discarded, making sure not to disturb the bead pellet. The tube was removed from the magnetic stand., and the washes were repeated two additional times. After the third wash, the tube was removed and the clear supernatant was discarded. A final 200 µL of binding buffer was added, and beads were resuspended by vortexing until homogeneous.

After completing the hybridization reaction, the thermal cycler lid was opened and the full volume of capture reaction was quickly transferred (36-40 µL) into the washed streptavidin binding beads. The mixture was mixed for 30 minutes at room temperature on a shaker, rocker, or rotator at a speed sufficient to keep capture reaction/streptavidin binding bead solution homogenized. The capture reaction/streptavidin binding bead solution was removed from mixer and pulse-spun to ensure all solution was at the bottom of the tube. The sample was placed on a magnetic stand, and streptavidin binding beads pelleted, leaving a clear supernatant within 1 minute. The clear supernatant was removed and discarded. The tube was removed from the magnetic stand and 200 µL of wash buffer was added at room temperature, followed by mixing by pipetting until homogenized. The tube was pulse-spun to ensure all solution was at the bottom of the tube. A thermal cycler was programmed with the following conditions **(Table 9)**.

The temperature of the heated lid was set to 105°C.

**Table 9**

| **Step** | **Temperature** | **Time** | **Cycle Number** |
|---|---|---|---|
| 1 | 98°C | 45 seconds | 1 |
| 2 | 98°C | 15 seconds | 9 |
| | 60°C | 30 seconds | |
| | 72°C | 30 seconds | |
| 3 | 72°C | 1 minute | 1 |
| 4 | 4°C | HOLD | |

Amplification primers (2.5 µL) and a polymerase, such as KAPA HiFi HotStart ReadyMix (25 µL) were added to a tube containing the water/streptavidin binding bead slurry, and the tube mixed by pipetting. The tube was then split into two reactions. The tube was pulse-spun and transferred to the thermal cycler and the cycling program in **Table 9** was started. When thermal cycler program was complete, samples were removed from the block and immediately subjected to purification. DNA purification beads pre-equilibrated at room temperature were vortexed until homogenized. 90 µL (1.8x) homogenized DNA purification beads were added to the tube, and mixed well by vortexing. The tube was incubated for 5 minutes at room temperature, and placed on a magnetic stand. DNA purification beads pelleted, leaving a clear supernatant within 1 minute. The clear supernatant was discarded, and the tube was left on the magnetic stand. The DNA purification bead pellet was washed with 200 µL of freshly prepared 80 percent ethanol, incubated for 1 minute, then removed and the ethanol discarded. The wash was repeated once, for a total of two washes, while keeping the tube on the magnetic stand. All remaining ethanol was removed and discarded with a 10 µL pipette, making sure to not disturb the DNA purification bead pellet. The DNA purification bead pellet was air-dried on a magnetic stand for 5-10 minutes or until the pellet was dry. The tube was removed from the magnetic stand and 32 µL of water was added, mixed by pipetting until homogenized, and incubated at room temperature for 2 minutes. The tube was placed on a magnetic stand for 3 minutes or until beads were fully pelleted. 30 µL of clear supernatant was recovered and transferred to a clean thin-walled PCR 0.2 mL strip-tube, making sure not to disturb DNA purification bead pellet. Average fragment length was between about 375 bp to about 425 bp using a range setting of 150 bp to 1000 bp on an analysis instrument. Ideally, the final concentration values is at least about 15 ng/µL. Each capture was quantified and validated using Next Generation Sequencing (NGS).

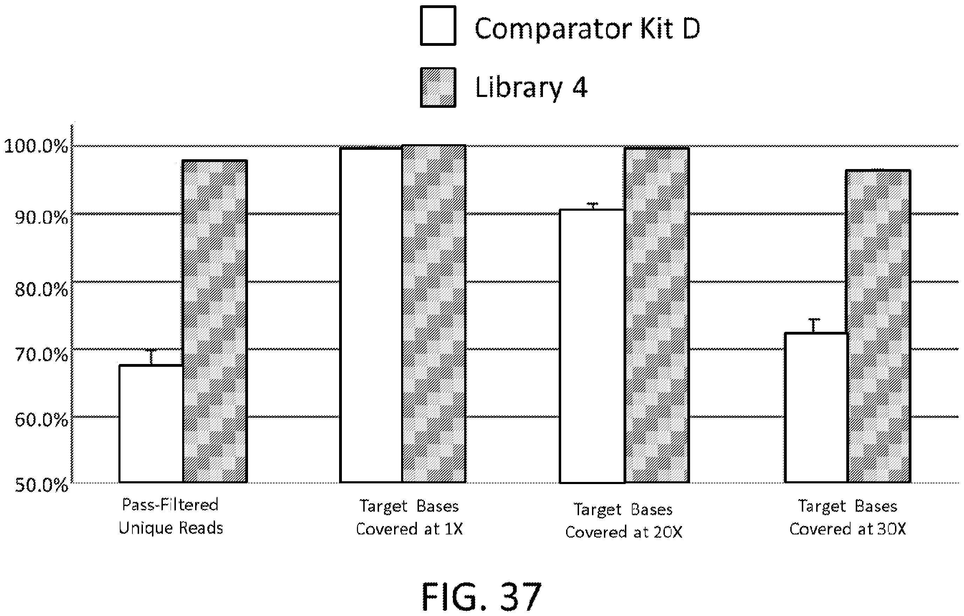

A summary of NGS metrics is shown in **Table 10, Table 11,** and **FIG. 37** as compared to a comparator exome capture kit (Comparator Kit D). Library 4 has probes (baits) that correspond to a higher percentage of exon targets than Comparator Kit D. This results in less sequencing to obtain comparable quality and coverage of target sequences using Library 4.

**Table 10**

| **NGS Metric** | **Comparator Kit D** | **Library 4** |
|---|---|---|
| Target Territory | 38.8 Mb | 33.2 Mb |
| Bait Territory | 50.8 Mb | 36.7 Mb |
| Bait Design Efficiency | 76.5% | 90.3% |
| Capture Plex | 8-plex | 8-plex |
| PF Reads | 57.7M | 49.3M |
| Normalized Coverage | 150X | 150X |
| HS Library Size | 30.3 M | 404.0 M |
| Percent Duplication | 32.5% | 2.5% |
| Fold Enrichment | 43.2 | 48.6 |
| Fold 80 Base Penalty | 1.84 | 1.40 |

**Table 11**

| **NGS Metric** | **Comparator Kit D** | **Library 4** |
|---|---|---|
| Percent Pass Filtered Unique Reads (PCT_PF_UQ_READS) | 67.6% | 97.5% |
| Percent Target Bases at 1X | 99.8% | 99.8% |
| Percent Target Bases at 20X | 90.3% | 99.3% |
| Percent Target Bases at 30X | 72.4% | 96.2% |

A comparison of overlapping target regions for both Kit D and Library 4 (total reads normalized to 96X coverage) is shown in **Table 12** and **FIG. 37**. Library 4 was processed as 8 samples per hybridization, and Kit D was processed at 2 samples per hybridization. Additionally, for both libraries, single nucleotide polymorphism and in-frame deletion calls from overlapping regions were compared against high-confidence regions identified from "Genome in a Bottle" NA12878 reference data **(Table 13)**. Library 4 performed similarly or better (higher indel precision) that Kit D in identifying SNPs and indels.

**Table 12**

| **NGS Metric** | **Comparator Kit D** | **Library 4** |
|---|---|---|
| Percent Pass Filtered Reads (PCT_PF_UQ_READS) | 94.60% | 97.7% |
| Percent Selected Bases | 79% | 80% |
| Percent Target Bases at 1X | 100% | 100% |
| Percent Target Bases at 20X | 90% | 96% |
| Percent Target Bases at 30X | 71% | 77% |
| Fold Enrichment | 44.9 | 49.9 |
| Fold 80 Base Penalty | 1.76 | 1.4 |
| HS Library Size | 122 M | 267 M |

**Table 13**

| **Variants** | **Comparator Kit D** | | **Library 4** | |
|---|---|---|---|---|
| | Precision | Sensitivity | Precision | Sensitivity |
| Single Nucleotide Polymorphisms (SNPs) | 98.59% | 99.23% | 99.05% | 99.27% |
| In-Frame Deletions (Indels) | 76.42% | 94.12% | 87.76% | 94.85% |
| Total | 98.14% | 99.15% | 98.85% | 99.20% |

Precision represents the ratio of true positive calls to total (true and false) positive calls. Sensitivity represents the ratio of true positive calls to total true values (true positive and false negative).

It is intended that the following claims define the scope of the invention.

## Claims

1. A polynucleotide library with controlled stoichiometry, the polynucleotide library comprising at least 5000 polynucleotides, wherein the controlled stoichiometry comprises at least 30 percent of the least 5000 polynucleotides have a GC percentage from 10 percent to 30 percent or 70 percent to 90 percent, such that, following hybridization with genomic fragments and sequencing of the hybridized genomic fragments, the polynucleotide library provides for at least 30 fold read depth of at least 90 percent of the bases of the genomic fragments under conditions for up to a 55 fold theoretical read depth for the bases of the genomic fragments, wherein the theoretical read depth comprises a distribution wherein every base of the genomic fragments has the same read depth.

2. The polynucleotide library of claim 1, wherein the polynucleotide library provides for at least 30 fold read depth of at least 95 percent of the bases of the genomic fragments under conditions for up to a 55 fold theoretical read depth for the bases of the genomic fragments.

3. The polynucleotide library of claim 1, wherein the polynucleotide library provides for at least 30 fold read depth of at least 98 percent of the bases of the genomic fragments under conditions for up to a 55 fold theoretical read depth for the bases of the genomic fragments.

4. The polynucleotide library of claim 1, wherein the polynucleotide library provides for at least 90 percent unique reads for the bases of the genomic fragments.

5. The polynucleotide library of claim 1, wherein each of the genomic fragments are about 100 bases to about 500 bases in length.

6. The polynucleotide library of claim 1, wherein greater than about 80 percent of the at least 5000 polynucleotides are represented in an amount within at least about 1.5 times the mean representation for the polynucleotide library.

7. The polynucleotide library of claim 1, wherein the at least 5000 polynucleotides encode for at least 1000 genes.

8. The polynucleotide library of claim 1, wherein the polynucleotide library comprises at least 700,000 polynucleotides.

9. The polynucleotide library of claim 8, wherein the at least 700,000 polynucleotides comprise at least one set of polynucleotides collectively comprising a single exon sequence.

10. The polynucleotide library of claim 1, wherein the ratio of the read depth of the at least 90 percent of the bases to the theoretical read depth is at least 0.5.

11. The polynucleotide library of claim 1, wherein the fragments are sequenced by a method comprising sequencing by synthesis.

12. The polynucleotide library of claim 1, wherein the fragments are sequenced on an Illumina sequencing instrument.

## Patentansprüche

1. Polynukleotidbibliothek mit gesteuerter Stöchiometrie, wobei die Polynukleotidbibliothek mindestens 5000 Polynukleotide umfasst, wobei die gesteuerte Stöchiometrie umfasst, dass mindestens 30 Prozent der mindestens 5000 Polynukleotide einen GC-Prozentsatz von 10 Prozent bis 30 Prozent oder 70 Prozent bis 90 Prozent aufweisen, sodass die Polynukleotidbibliothek nach Hybridisierung mit Genomfragmenten und Sequenzieren der hybridisierten Genomfragmente eine mindestens 30-fache Lesetiefe von mindestens 90 Prozent der Basen der Genomfragmente unter Bedingungen für eine bis zu 55-fache theoretische Lesetiefe für die Basen der Genomfragmente bereitstellt, wobei die theoretische Lesetiefe eine Verteilung umfasst, wobei jede Base der Genomfragmente die gleiche Lesetiefe aufweist.

2. Polynukleotidbibliothek nach Anspruch 1, wobei die Polynukleotidbibliothek eine mindestens 30-fache Lesetiefe von mindestens 95 Prozent der Basen der Genomfragmente unter Bedingungen für eine bis zu 55-fache theoretische Lesetiefe für die Basen der Genomfragmente bereitstellt.

3. Polynukleotidbibliothek nach Anspruch 1, wobei die Polynukleotidbibliothek eine mindestens 30-fache Lesetiefe von mindestens 98 Prozent der Basen der Genomfragmente unter Bedingungen für eine bis zu 55-fache theoretische Lesetiefe für die Basen der Genomfragmente bereitstellt.

4. Polynukleotidbibliothek nach Anspruch 1, wobei die Polynukleotidbibliothek mindestens 90 Prozent eindeutige Lesevorgänge für die Basen der Genomfragmente bereitstellt.

5. Polynukleotidbibliothek nach Anspruch 1, wobei jedes der Genomfragmente etwa 100 Basen bis etwa 500 Basen lang ist.

6. Polynukleotidbibliothek nach Anspruch 1, wobei mehr als etwa 80 Prozent der mindestens 5000 Polynukleotide in einer Menge innerhalb von mindestens etwa dem 1,5-Fachen der mittleren Darstellung für die Polynukleotidbibliothek dargestellt sind.

7. Polynukleotidbibliothek nach Anspruch 1, wobei die mindestens 5000 Polynukleotide für mindestens 1000 Gene codieren.

8. Polynukleotidbibliothek nach Anspruch 1, wobei die Polynukleotidbibliothek mindestens 700.000 Polynukleotide umfasst.

9. Polynukleotidbibliothek nach Anspruch 8, wobei die mindestens 700.000 Polynukleotide mindestens einen Satz von Polynukleotiden umfassen, die zusammen eine einzige Exonsequenz umfassen.

10. Polynukleotidbibliothek nach Anspruch 1, wobei das Verhältnis der Lesetiefe der mindestens 90 Prozent der Basen zu der theoretischen Lesetiefe mindestens 0,5 beträgt.

11. Polynukleotidbibliothek nach Anspruch 1, wobei die Fragmente durch ein Verfahren sequenziert werden, das Sequenzieren durch Synthese umfasst.

12. Polynukleotidbibliothek nach Anspruch 1, wobei die Fragmente auf einem Sequenzierungsinstrument von Illumina sequenziert werden.

## Revendications

1. Banque de polynucléotides à stoechiométrie contrôlée, la banque de polynucléotides comprenant au moins 5000 polynucléotides, dans laquelle la stoechiométrie contrôlée comprend le fait qu'au moins 30 pour cent des au moins 5000 polynucléotides présentent un pourcentage de GC de 10 pour cent à 30 pour cent ou de 70 pour cent à 90 pour cent, de sorte que, après hybridation avec des fragments génomiques et séquençage des fragments génomiques hybridés, la banque de polynucléotides permet une profondeur de lecture d'au moins 30 fois pour au moins 90 pour cent des bases des fragments génomiques dans des conditions permettant une profondeur de lecture théorique jusqu'à 55 fois pour les bases des fragments génomiques, dans laquelle la profondeur de lecture théorique comprend une distribution dans laquelle chaque base des fragments génomiques présente la même profondeur de lecture.

2. Banque de polynucléotides selon la revendication 1, dans laquelle la banque de polynucléotides permet une profondeur de lecture d'au moins 30 fois pour au moins 95 pour cent des bases des fragments génomiques dans des conditions permettant une profondeur de lecture théorique jusqu'à 55 fois pour les bases des fragments génomiques.

3. Banque de polynucléotides selon la revendication 1, dans laquelle la banque de polynucléotides permet une profondeur de lecture d'au moins 30 fois pour au moins 98 pour cent des bases des fragments génomiques dans des conditions permettant une profondeur de lecture théorique jusqu'à 55 fois pour les bases des fragments génomiques.

4. Banque de polynucléotides selon la revendication 1, dans laquelle la banque de polynucléotides permet d'obtenir au moins 90 pour cent de lectures uniques pour les bases des fragments génomiques.

5. Banque de polynucléotides selon la revendication 1, dans laquelle chacun des fragments génomiques présente une longueur d'environ 100 bases à environ 500 bases.

6. Banque de polynucléotides selon la revendication 1, dans laquelle plus de 80 pour cent environ des au moins 5000 polynucléotides sont représentés dans une proportion au moins égale à environ 1,5 fois la représentation moyenne de la banque de polynucléotides.

7. Banque de polynucléotides selon la revendication 1, dans laquelle les au moins 5000 polynucléotides codent pour au moins 1000 gènes.

8. Banque de polynucléotides selon la revendication 1, dans laquelle la banque de polynucléotides comprend au moins 700000 polynucléotides.

9. Banque de polynucléotides selon la revendication 8, dans laquelle les au moins 700000 polynucléotides comprennent au moins un ensemble de polynucléotides comprenant collectivement une seule séquence d'exon.

10. Banque de polynucléotides selon la revendication 1, dans laquelle le rapport entre la profondeur de lecture d'au moins 90 pour cent des bases et la profondeur de lecture théorique est d'au moins 0,5.

11. Banque de polynucléotides selon la revendication 1, dans laquelle les fragments sont séquencés par un procédé comprenant le séquençage par synthèse.

12. Banque de polynucléotides selon la revendication 1, dans laquelle les fragments sont séquencés à l'aide d'un instrument de séquençage Illumina.
